(19) Europäisches Patentamt
European Patent Office
Office européen des brevets

(11) EP 2 454 253 B1

(12) # EUROPÄISCHE PATENTSCHRIFT

(45) Veröffentlichungstag und Bekanntmachung des Hinweises auf die Patenterteilung:
**21.01.2015 Patentblatt 2015/04**

(21) Anmeldenummer: **10730073.3**

(22) Anmeldetag: **17.06.2010**

(51) Int Cl.:
***C07D 401/14*** (2006.01) ***C07D 405/14*** (2006.01)
***C07D 409/14*** (2006.01) ***C07D 413/14*** (2006.01)
***C07D 417/14*** (2006.01) ***C07D 471/04*** (2006.01)
***C07D 473/00*** (2006.01) ***C07D 487/04*** (2006.01)
***C07D 491/056*** (2006.01) ***C07D 498/04*** (2006.01)
***A61K 31/4439*** (2006.01) ***A61K 31/52*** (2006.01)
***A61P 35/00*** (2006.01) ***A61P 29/00*** (2006.01)
***A61P 27/14*** (2006.01)

(86) Internationale Anmeldenummer:
**PCT/EP2010/003659**

(87) Internationale Veröffentlichungsnummer:
**WO 2011/006567 (20.01.2011 Gazette 2011/03)**

(54) **AMINOPYRIDINDERIVATE ZUR BEHANDLUNG VON TUMOREN UND ENTZÜNDUNGSKRANKHEITEN**

AMINOPYRIDINE DERIVATIVES FOR TREATING TUMORS AND INFLAMMATORY DISEASES

DÉRIVÉS AMINOPYRIDINE POUR LE TRAITEMENT DE TUMEURS ET DE MALADIES INFLAMMATOIRES

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **15.07.2009 DE 102009033208**

(43) Veröffentlichungstag der Anmeldung:
**23.05.2012 Patentblatt 2012/21**

(73) Patentinhaber: **Merck Patent GmbH**
**64293 Darmstadt (DE)**

(72) Erfinder:
- **CALDERINI, Michel**
**64291 Darmstadt (DE)**
- **WUCHERER-PLIETKER, Margarita**
**64291 Darmstadt (DE)**
- **GRAEDLER Ulrich**
**69469 Weinheim (DE)**
- **ESDAR, Christina**
**55128 Mainz (DE)**

(56) Entgegenhaltungen:
**WO-A2-2004/069160 WO-A2-2007/111904**

- **F.-J. GAMO ET AL.: "Thousands of chemical starting points for antimalarial lead identification" NATURE, Bd. 465, 20. Mai 2010 (2010-05-20), Seiten 305-310, XP002600760**
- **DATABASE ChEMBL-NTD [Online] EBI; 20. Mai 2010 (2010-05-20), XP002605207 Database accession no. 527649**
- **DATABASE ChEMBL-NTD [Online] EBI; 20. Mai 2010 (2010-05-20), XP002605208 Database accession no. 530945**
- **DATABASE ChEMBL-NTD [Online] EBI; 20. Mai 2010 (2010-05-20), XP002605209 Database accession no. 524805**
- **DATABASE ChEMBL-NTD [Online] EBI; 20. Mai 2010 (2010-05-20), XP002605210 Database accession no. 542271**
- **DATABASE ChEMBL-NTD [Online] EBI; 20. Mai 2010 (2010-05-20), XP002605211 Database accession no. 525200**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Offenbart werden Verbindungen der Formel I

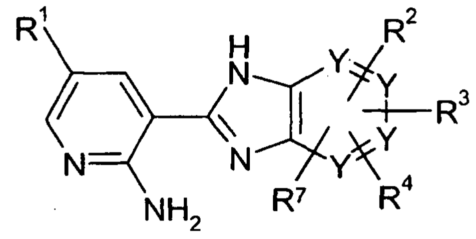

I

worin

$R^1$ einen ein- oder zweikernigen gesättigten, ungesättigten oder aromatischen Heterocyclus mit 1 bis 4 N-, O- und/oder S-Atomen, der unsubstituiert oder ein- oder zweifach durch Hal, A, $OR^5$, $SR^5$, $NO_2$, CN, $NR^5COA$, $NR^5SO_2A$, $SO_2N(R^5)_2$, $S(O)_mA$, $[C(R^5)_2]_nCOOR^5$, $[C(R^5)_2]_nCON(R^5)_2$, $[C(R^5)_2]_nCONR^5[C(R^5)_2]_nAr$, $C(R^5)_2]_nCONR^5[C(R^5)_2]_nHet$, $[C(R^5)_2]_nCO\text{-}Het$, $[C(R^5)_2]_nN(R^5)_2$, $[C(R^5)_2]_nAr$, $[C(R^5)_2]_nHet$, $O[C(R^5)_2]_nN(R^5)_2$, $O[C(R^5)_2]_nHet$, $NR^5COOA$, $NR^5CON(R^5)_2$, $NR^5COO[C(R^5)_2]_nN(R^5)_2$, $NR^5COO[C(R^5)_2]_nHet^1$, $NR^5CONR^5[C(R^5)_2]_nN(R^5)_2$, $NR^5CONR^5[C(R^5)_z]_nHet^1$, $OCONR^5[C(R^5)_2]_nN(R^5)_2$, $OCONR^5[C(R^5)_2]_nHet^1$, $[C(R^5)_2]_nOR^5$, $[C(R^5)_2]_nCONR^5[C(R^5)_2]_nN(R^5)_2$, CHO, COA, =S, =NH, =NA und/oder =O (Carbonylsauerstoff) substituiert sein kann,

Y unabhängig voneinander N oder C, wobei maximal zwei Y N bedeuten können,

$R^2$, $R^3$, $R^4$, $R^7$ jeweils unabhängig voneinander H, Hal, A, OA, $SR^5$, $NO_2$, CN, $NR^5COA$, $NR^5SO_2A$, $SO_2N(R^5)_2$, $S(O)_mA$, $S(O)_mHet$, CO-Ar, CO-Het, $[C(R^5)_{2]n}Ar$, $[C(R^5)_2]_nHet$, $C(R^5)(OR^5)Ar$, $C(R^5)(OR^5)Het$, $[C(R^5)_2]_nOR^5$, $[C(R^5)_2]_nOAr$, $[C(R^5)_2]_nOHet$, $[C(R^5)_2]_nN(R^5)_2$, $[C(R^5)_2]_nCON(R^5)_2$, $[C(R^5)_2]_nCOOR^5$, $[C(R^S)_2]_nCONR^5[C(R^5)_2]_nAr$, $[C(R^5)_2]_nCONR^5[C(R^5)_2]_nHet$, $[C(R^5)_2]_nCOO[C(R^5)_2]_nAr$, $[C(R^5)_2]_nCOO[C(R^5)_2]_nHet$, $[C(R^5)_2]_nNR^5CO[C(R^5)_2]_nAr$ oder $[C(R^5)_2]_nNR^5CO[C(R^5)_2]_nHet$, mit der Maßgabe, daß

a) wenn ein Y N bedeutet, $R^7$ fehlt;

b) wenn zwei Y N bedeuten, $R^4$ und $R^7$ fehlen;

zwei benachbarte Reste,
ausgewählt aus der Gruppe $R^2$, $R^3$, $R^4$, $R^7$
zusammen auch $OCH_2O$, $OCH_2CH_2O$, NHCONH, $OCF_2O$, CH=N-NH oder NH-N=CH,

$R^5$ A oder H,

A unverzweigtes oder verzweigtes Alkyl mit 1-10 C-Atomen, worin 1-7 H-Atome durch F ersetzt sein können und/oder worin eine oder zwei $CH_2$-Gruppen durch O, S, $NR^5$ und/oder durch CH=CH-Gruppen ersetzt sein können, oder cyclisches Alkyl mit 3-7 C-Atomen,

Ar unsubstituiertes oder ein-, zwei- oder dreifach durch Hal, A, $OR^5$, $N(R^5)_2$, $SR^5$, $NO_2$, CN, $COOR^5$, $CON(R^5)_2$, $NR^5COA$, $NR^5SO_2A$, $SO_2N(R^5)_2$, $S(O)_mA$, CO-$Het^1$, $Het^1$, $O[C(R^5)_2]_n\text{-}N(R^5)_2$, $O[C(R^5)_2]_nHet^1$, NHCOOA, NH-$CON(R^5)_2$, $NHCOO[C(R^5)_2]_nN(R^5)_2$, $NHCOO[C(R^5)_2]_nHet^1$, $NHCONH[C(R^5)_2]_nN(R^5)_2$, $NHCONH[C(R^5)_2]_nHet^1$, $OCONH[C(R^5)_2]_nN(R^5)_2$, $OCONH[C(R^5)_2]_nHet^1$ und/oder COA substituiertes Phenyl, Naphthyl oder Biphenyl,

Het einen ein- oder zweikernigen gesättigten, ungesättigten oder aromatischen Heterocyclus mit 1 bis 4 N-, O- und/oder S-Atomen, der unsubstituiert oder ein- bis dreifach durch Hal, A, $OR^5$, $N(R^5)_2$, $SR^5$, $NO_2$, CN, $COOR^5$, $CON(R^5)_2$, $NR^5COA$, $NR^5SO_2A$, $SO_2N(R^5)_2$, $S(O)_mA$, CO-$Het^1$, $Het^1$, $[C(R^5)_2]_nN(R^5)_2$, $[C(R^5)_2]_nHet^1$, $O[C(R^5)_2]_nN(R^5)_2$, $O[C(R^5)_2]_nHet^1$, NHCOOA, $NHCON(R^5)_2$, $NHCOO[C(R^5)_2]_nN(R^5)_2$, $NHCOO[C(R^5)_2]_nHet^1$, $NHCONH[C(R^5)_2]_nN(R^5)_2$, $NHCONH[C(R^5)_2]_nHet^1$, $OCONH[C(R^5)_2]_nN(R^5)_2$, $OCONH[C(R^5)_2]_nHet^1$, CO-$Het^1$, CHO, COA, =S, =NH, =NA und/oder =O (Carbonylsauerstoff) substituiert sein kann,

Het[1] einen einkernigen gesättigten Heterocyclus mit 1 bis 2 N und/oder O-Atomen, der ein- oder zweifach durch A, OA, OH, Hal und/oder =O (Carbonylsauerstoff) substituiert sein kann,

Hal F, Cl, Br oder I,

m 0, 1 oder 2,

n 0, 1, 2, 3 oder 4,

bedeuten,
sowie ihre pharmazeutisch verwendbaren Salze, Tautomere und Stereoisomere, einschließlich deren Mischungen in allen Verhältnissen.

Die Erfindung betrifft die in Anspruch 1 genannten Verbindungen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze und/oder Solvate bei guter Verträglichkeit sehr wertvolle pharmakologische Eigenschaften besitzen.

Insbesondere zeigen sie eine inhibierende Wirkung der Zellproliferation/ Zellvitalität als Antagonisten oder Agonisten. Die erfindungsgemäßen Verbindungen können daher zur Bekämpfung und/oder Behandlung von Tumoren, Tumorwachstum und/oder Tumormetastasen verwendet werden.

Die antiproliferative Wirkung kann in einem Proliferationsassay/ Vitalitätsassay getestet werden.

Andere Amino-heteroarylverbindungen sind als Kinase-Inhibitoren zur Krebsbekämpfung in WO 2006/021886, WO 2007/111904 und WO 2004/069160 beschrieben.

Andere heterocyclische Verbindungen kennt man aus WO 2009/053737, WO 2009/126003 und US 2009/0197862.

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom).
Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.
Die Verbindungen sind ferner nützlich bei der Behandlung der durch HIV-1 (Human Immunodeficiency Virus Typ 1) induzierten Immunschwäche.

Als krebsartige hyperproliferative Erkrankungen sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie anzusehen. Insbesondere krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen

Es kann gezeigt werden, dass die erfindungsgemäßen Verbindungen antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen

Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Die Verbindungen der Formel I, wirken auch als Regulatoren, Modulatoren oder Inhibitoren von Proteinkinasen, insbesondere des Typs Serin/Threonin-Kinase, zu denen unter anderem die Phosphoinositid-abhängige Kinase 1 (PDK 1) gehören. Eine gewisse Wirkung zeigen die erfindungsgemäßen Verbindungen bei der Inhibierung der Serin/Threonin-Kinase PDK1.

PDK1 phosphoryliert und aktiviert eine Untergruppe der AGC Proteinkinasen-Familie, umfassend PKB, SGK, S6K und PKC Isoformen. Diese Kinasen sind an dem PI3K Signalübertragungsweg beteiligt und kontrollieren grundlegende zelluläre Funktionen wie Überleben, Wachstum und Differenzierung. PDK1 ist somit ein bedeutender Regulator diverser metabolischer, proliferativer und Lebenserhaltungs-Effekte.

Durch Proteinkinasen hervorgerufene Erkrankungen sind durch eine anomale Aktivität oder Hyperaktivität solcher Proteinkinasen gekennzeichnet. Anomale Aktivität betrifft entweder: (1) die Expression in Zellen, die gewöhnlich diese Proteinkinasen nicht exprimieren; (2) erhöhte Kinasen-Expression, die zu unerwünschter Zellproliferation, wie Krebs, führt; (3) erhöhte Kinasen-Aktivität, die zu unerwünschter Zellproliferation, wie Krebs, und/oder zu Hyperaktivität der entsprechenden Proteinkinasen führt. Hyperaktivität bezieht sich entweder auf eine Amplifikation des Gens, das eine bestimmte Proteinkinase codiert, oder die Erzeugung eines Aktivitäts-Spiegels, der mit einer Zellproliferationserkrankung korreliert werden kann (d.h. mit steigendem Kinase-Spiegel steigt die Schwere eines oder mehrerer Symptome der Zellproliferationserkrankung) die biologische Verfügbarkeit einer Proteinkinase kann auch durch das Vorhandensein oder Fehlen eines Satzes von Bindungsproteinen dieser Kinase beeinflusst werden.

Die wichtigsten Krebsarten, die unter Verwendung einer erfindungsgemäßen Verbindung behandelt werden können, umfassen Kolorektalkrebs, kleinzelligen Lungenkrebs, nicht-kleinzelligen Lungenkrebs, das multiple Myelom sowie das Nierenzellkarzinom und das Endometriumkarzinom, bersonders auch Krebsarten, in denen PTEN mutiert ist, u. a. Brustkrebs, Prostata-Krebs und Glioblastom.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemo -therapien und -bestrahlungen wiederherzustellen.

Die Verbindungen der Formel I, wirken auch als Regulatoren, Modulatoren oder Inhibitoren bei der Expression und/oder Funktion von Proteinen, die bei Entzündungskrankheiten, Regulierung der Immunantwort oder Zellproliferation eine Rolle spielen.

Die vorliegende Erfindung betrifft Verbindungen, die Interleukin-1- (IL-1-)-Rezeptor-assoziierte Kinase (IRAK) modulieren und bei der Vorbeugung oder Behandlung von entzündlichen, zellproliferativen und mit dem Immunsystem zusammenhängenden Zuständen und Erkrankungen von Nutzen sind. Die Erfindung betrifft auch pharmazeutische Zusammensetzungen, die diese Verbindungen enthalten, sowie die Verwendung der betreffenden Verbindungen und Zusammensetzungen zur Vorbeugung oder Behandlung von durch IRAK vermittelten Zuständen oder Erkrankungen.

Die Bedeutung von IL-1 bei Entzündungen wurde durch die Fähigkeit des hochspezifischen IL-1-Rezeptor-Antagonistproteins (IL-1 Ra oder IRAP) zur Linderung von entzündlichen Zuständen nachgewiesen (siehe für einen Überblick, z. B. Dinarello (1997) Cytokine Growth Factor Rev. 8:253-265).

Eine Behandlung von Zellen mit IL-1 induziert die Bildung eines Komplexes bestehend aus den beiden IL-1-Rezeptorketten, IL-1R1 und IL-1RAcP, und das resultierende Heterodimer rekrutiert ein Adaptermolekül, das als MyD88 bezeichnet wird (Wesche et al. (1999) J. Biol. Chem. 274:19403-19410). MyD88 bindet an ein Protein, das mit IRAK (IL-1-Rezeptor-assoziierte Kinase) bezeichnet wird (siehe, O'Neill et al. (1998) J. Leukoc. Biol. 63(6):650-657, Auron (1998) Cytokine Growth Factor Rev. 9(3-4):221-237 und O'Neill (2000) Biochem. Soc. Trans. 28(5)557-563, für Übersichtsartikel). IRAK wird anschließend phosphoryliert und aus dem Rezeptorkomplex freigesetzt, um mit einem Tumornekrosefaktor-Rezeptor-assoziierten Faktor, TRAF6, zu wechselwirken, der das Signal in downstream-Effektormoleküle transduziert (Cao et al. (1996) Nature 383:443-446). TRAF6 kann die NIK/IKK-Kinase-Kaskade auslösen, um den Transkriptionsfaktor NF-κB zu aktivieren. NF-κB reguliert eine Anzahl an Genen, die ihrerseits die Immun- und Entzündungsantworten regulieren.

Es wurden vier IRAKs identifiziert: IRAK-1 (Cao, et al. (1996) Science 271:1128-1131), IRAK-2 (Muzio, et al. (1997) Science 278:1612-1615), das für Monozyten spezifische IRAK-M, auch bekannt als IRAK-3 (Wesche, et al. (1999) J. Biol. Chem. 274:19403-10) und IRAK-4 (PCT Publikationsnr. WO 01/051641). Es wurde nachgewiesen, dass IRAK-Proteine eine Rolle bei der Signaltransduktion spielen, außer bei denen, die von den IL-1-Rezeptoren stammen, einschließlich von Signalen, die durch die Aktivierung von IL-18-Rezeptoren (Kanakaraj et al. (1999) J. Exp. Med. 189(7):1129-1138) und LPS-Rezeptoren ausgelöst werden (Yang et al. (1999) J. Immunol. 163:639-643; Wesche et al. (1999) J. Biol. Chem. 274:19403-19410). Überexpression von IRAK-2 und IRAK-M ist nachweislich in der Lage, die Antwort auf IL-1 und LPS in einer Zelllinie mit IRAK-Mangel zu rekonstituieren.

Wie hierin verwendet, bezieht sich der Begriff "IRAK" auf ein Interleukin--1-(IL-1-)-Rezeptor-assoziiertes Kinaseprotein oder eine Variante davon, die in der Lage ist, eine zelluläre Reaktion auf IL-1 in vitro oder in vivo zu vermitteln. Bei IRAK kann es sich um Kinase aktive oder inaktive Proteine handeln. Beispiele für Kinase-aktive IRAKs umfassen IRAK-1 und IRAK-4. Beispiele für Kinase-inaktive IRAKs umfassen IRAK-2 und IRAK-3 (auch bekannt als IRAK-M). Kinase-aktive IRAKs können in der Lage sein, andere Proteine zu transphosphorylieren oder sich selbst zu phosphorylieren. In bevorzugten Ausführungsformen ist IRAK IRAK-1 und/oder IRAK-4.

IRAK-Varianten umfassen Proteine, die im Wesentlichen homolog zu nativem IRAK sind, d. h. Proteine mit einer oder mehreren natürlich oder nicht natürlich vorkommenden Deletionen, Insertionen oder Substitutionen von Aminosäuren (z. B. IRAK-Derivate, Homologe oder Fragmente). Die Aminosäuresequenz einer IRAK-Variante ist vorzugsweise zu mindestens 80 % identisch mit dem nativen IRAK, mehr bevorzugt zu mindestens etwa 90 % identisch und noch mehr bevorzugt zu mindestens etwa 95 % identisch.

Die Begriffe "Signaltransduktion", "Signalisieren" und verwandte Begriffe beziehen sich auf einen Prozess, bei dem ein extrazelluläres Signal (z. B. die Konzentration eines Cytokins, Hormons, Neurotransmitters, Wachstumsfaktors) über eine Kaskade von intrazellulären Protein-Protein-Wechselwirkungen zum Zellkern übermittelt werden und eine oder mehrere Zellantworten erzeugen (z. B. Gentranskription, Proteinsekretion, Mitose, Apoptose). Die Wechselwirkung eines extrazellulären Signalmoleküls (z. B. eines Cytokins, eines Hormons, eines Neurotransmitters, eines Wachstumsfaktors) mit einem oder mehreren Transmembranproteinrezeptoren auf der Zelloberfläche kann einen oder mehrere Signaltransduktionswege aktivieren. Die Protein-Protein-Wechselwirkungen in einem Signaltransduktionsweg können multivalent sein und umfassen kovalente und/oder nicht kovalente Proteinmodifizierungen. Ein intrazelluläres Signalmolekül, d. h. ein Signaltransduktionsprotein oder ein Signaltransducer, kann an einem oder mehreren Signaltransduktionswegen beteiligt sein. Wie hierin beschrieben, umfassen Protein-Protein-Wechselwirkungen direkte und indirekte Wechselwirkungen.

Wie hierin verwendet, bezieht sich der Ausdruck "IRAK-responsiver Zustand oder Störung" sowie verwandte Ausdrücke und Begriffe auf einen Zustand oder eine Störung, die günstig auf eine Modulation der IRAK-Aktivität reagiert. Günstige Reaktionen auf eine IRAK-Modulation umfassen die Linderung oder Aufhebung der Erkrankung und/oder ihrer Begleitsymptome, Inhibierung einer Erkrankung, d. h. Stopp oder Vermindung der Entwicklung der Erkrankung oder ihrer klinischen Symptome und Regression der Erkrankung oder ihrer klinischen Symptome. Ein IRAK-responsiver Zustand oder Erkrankung können vollständig oder teilweise auf eine IRAK-Modulation reagieren. Ein IRAK-responsiver Zustand oder Störung kann mit einer unpassenden, z. B. geringeren oder größeren IRAK-Aktivität als normal einhergehen. Eine unpassende funktionelle IRAK-Aktivität kann als Folge einer IRAK-Expression in Zellen, die normalerweise kein IRAK exprimieren, einer verminderten IRAK-Expression (was, z. B., zu Lipid- und metabolischen Störungen und Erkrankungen führt) oder einer verminderten IRAK-Expression auftreten. Ein IRAK-responsiver Zustand oder Erkrankung kann jeglichen IRAK vermittelten Zustand oder Erkrankung wie unten definiert umfassen.

Wie hierin verwendet, bezieht sich der Ausdruck "IRAK vermittelter Zustand oder Störung" sowie verwandte Ausdrücke und Begriffe auf einen Zustand oder eine Störung, der/die durch eine unpassende, z. B. geringere oder größere IRAK-Aktivität als normal gekennzeichnet ist. Eine unpassende funktionelle IRAK-Aktivität kann als Folge einer IRAK-Expression in Zellen, die normalerweise kein IRAK exprimieren, einer verminderten IRAK-Expression oder eines Grads an intrazellulärer Aktivierung (was, z. B., zu entzündlichen und Autoimmunstörungen und -erkrankungen führt) oder einer verminderten IRAK-Expression auftreten. Ein IRAK vermittelter Zustand oder Störung kann vollständig oder teilweise durch eine unpassende funktionelle IRAK-Aktivität vermittelt werden. Ein IRAK vermittelter Zustand oder Störung ist jedoch einer/eine, bei dem/der eine Modulation von IRAK eine Wirkung auf den zugrunde liegenden Zustand oder die zugrunde liegende Störung zur Folge hat (z. B. führt ein IRAK-Inhibitor zu einer Verbesserung des Wohlbefindens von Patienten, zumindest bei einigen von ihnen).

Der Begriff "moduliert" bezieht sich auf die Fähigkeit einer Verbindung, die Funktion und/oder Expression von IRAK zu erhöhen oder zu vermindern, wobei eine IRAK-Funktion eine Kinaseaktivität und/oder Proteinbindung umfassen

kann. Eine Modulation kann in vitro oder in vivo auftreten. Modulation, wie hierin beschrieben, umfasst die Inhibierung oder Aktivierung einer IRAK-Funktion und/oder die Herabregulierung oder Hochregulierung der IRAK-Expression, entweder direkt oder indirekt. Ein Modulator aktiviert vorzugsweise eine IRAK-Funktion und/oder reguliert die IRAK-Expression hoch. Mehr bevorzugt aktiviert oder inhibiert ein Modulator eine IRAK-Funktion und/oder reguliert die IRAK-Expression hoch oder herab. Am meisten bevorzugt inhibiert ein Modulator eine IRAK-Funktion und/oder reguliert die IRAK-Expression herab. Die Fähigkeit einer Verbindung, die IRAK-Funktion zu inhibieren, kann in enzymatischen Assays oder zellbasierten Assays nachgewiesen werden (z. B. Inhibierung der IL-1-stimulierten NF-κB-Aktivierung).

Erkrankungen oder Zustände, einschließlich chronischer Erkrankungen, von Menschen oder anderen Spezies können mit Inhibitoren der IRAK-Funktion behandelt oder ihnen kann vorgebeugt werden. Diese Erkrankungen oder Zustände umfassen (1) entzündliche oder allergische Erkrankungen wie z. B. systemische Anaphylaxie und Überempfindlichkeitsreaktionen, Allergien auf Wirkstoffe, Allergien bei Insektenstichen und Nahrungsmittelallergien, (2) entzündliche Darmerkrankungen wie z. B. Morbus Crohn, Colitis ulcerosa, Ileitis und Enteritis, (3) Vaginitis, (4) Psoriasis und entzündliche Dermatosen wie z. B. Dermatitis, Ekzeme, atopische Dermatitis, allergische Kontaktdermatitis und Urtikaria, (5) Vasculitis, (6) Spondyloarthropathien, (7) Sklerodermie, (8) Asthma und respiratorische allergische Erkrankungen wie allergisches Asthma, allergische Rhinitis, allergische Konjunktivitis, Hypersensitivitätslungenerkrankungen und dergleichen, sowie (9) Autoimmunerkrankungen, wie z. B. Arthritis (einschließlich rheumatoider und psoriatischer), systemischer Lupus erythematodes, Diabetes Typ I, Glomerulonephritis und dergleichen, (10) Transplantatabstoßung (einschließlich Allotransplantatabstoßung und Graft-versus-Host-Erkrankungen), (11) weitere Erkrankungen, bei denen unerwünschte entzündliche Erkrankungen inhibiert werden sollen, z. B. Atherosklerose, Myositis, neurologische Störungen wie Schlaganfall, ischämische Reperfusionsverletzungen, offene Schädelhirntraumata und gedeckte Schädelhirntraumata, neurodegenerative Erkrankungen (z. B., Morbus Parkinson), Multiple Sklerose, Morbus Alzheimer, Enzephalitis, Meningitis, Osteoporose, Gicht, Hepatitis, Nephritis, Gallenblasenerkrankungen, Sepsis, Sarkoidose, Konjunktivitis, Otitis, chronisch obstruktive Lungenerkrankung, Sinusitis und Behcet-Syndrom; (12) zellproliferative oder neoplastische Erkrankungen wie z. B. Brust-, Haut-, Prostata-, Gebärmutterhals-, Gebärmutter-, Eierstock, Hoden-, Blasen-, Lungen-, Leber-, Kehlkopf-, Mundhöhlen-, Darmkrebs oder Krebs des Gastrointestinaltrakts (z. B. Speiseröhre, Magen, Bauchspeicheldrüse), Hirn-, Schilddrüsen-, Blutkrebs oder Krebs der lymphatischen Systeme und Erkrankungen, bei denen Angiogenese und Neovaskularisierung eine Rolle spielen, (13) Metabolismusstörungen, die empfindlich auf eine Inhibierung der TNF- oder IL-1-Signalgebung reagieren, wie z. B. Fettsucht, Diabetes Typ II, metabolisches Syndrom, Insulinresistenz, Hyperglykämie, Hyperurikämie, Hyperinsulinämie, Kachexie, Hypercholesterolämie, Hyperlipidämie, Dyslipidämie, gemischte Dyslipidämie und Hypertriglyceridämie, Essstörungen wie z. B. Anorexia Nervosa und Bulimie, (14) infektiöse Erkrankungen, z. B. Bakteriämie und septischer Schock; (15) kardiovaskuläre Störungen wie z. B. akutes Herzversagen, Hypotonie, Hypertonie, Angina Pectoris, Myokardinfarkt, Kardiomyopathie, kongestive Herzinsuffizienz, Atherosklerose, Erkrankungen der Koronararterien, Restenose und vaskuläre Stenose sowie (16) Immunerkrankungen und - zustände.

In einer Ausführungsform sind die vorliegenden Verfahren auf die Behandlung oder Vorbeugung von Erkrankungen oder Zuständen gerichtet, die aus rheumatoider Arthritis, septischer Schock, entzündlichen Darmerkrankungen, Knochenmasseverlust, Krebs, Hautsensibilisierungsstörungen, Diabetes, Fettsucht, ischämischer Hirnschlag, ischämischer Reperfusionsverletzung, Schädelhirntrauma, Asthma, allergischen Erkrankungen, multipler Sklerose und Transplantatabstoßung ausgewählt werden.

Andere heterocyclische Verbindungen sind als IRAK-Inhibitoren zur Bekämpfung von Entzündungskrankheiten beschrieben in US 7,132,438 B2 oder US 7,199,119 B2.

Unter Verbindungen der Formel I versteht man auch die Hydrate und Solvate dieser Verbindungen. Hierin offenbart sind auch die optisch aktiven Formen (Stereoisomeren), Salze, die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:

verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Hierin offenbart ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Hierin offenbart sind die Verbindungen der Formel I und ihre Salze sowie ein Verfahren zur Herstellung von Verbindungen der Formel I sowie ihrer pharmazeutisch verwendbaren Salze, Tautomeren und Stereoisomeren, dadurch gekennzeichnet, daß man

a) eine Verbindung der Formel II

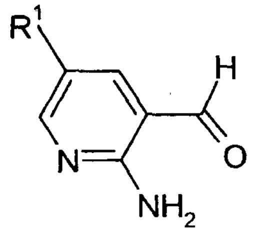

II

worin $R^1$ die in Anspruch 1 angegebene Bedeutung hat,
mit einer Verbindung der Formel III

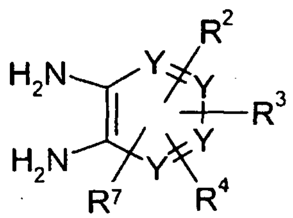

III

worin Y, $R^2$, $R^3$, $R^4$, $R^7$ die in Anspruch 1 angegebenen Bedeutungen haben,
umsetzt,
oder
b) eine Verbindung der Formel IV

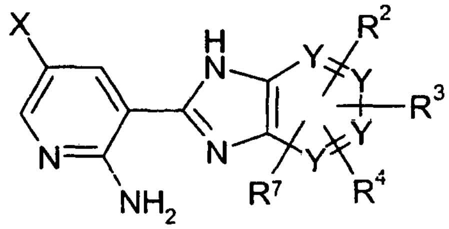

IV

worin Y, $R^2$, $R^3$, $R^4$, $R^7$ die in Anspruch 1 angegebenen Bedeutungen haben und X Br oder I bedeutet,
mit einer Verbindung der Formel V

$R^1$-L V

worin $R^1$ die in Anspruch 1 angegebene Bedeutung hat und L einen Boronsäure- oder Boronsäureesterrest bedeutet,

umsetzt,
und/oder eine Base oder Säure der Formel I in eines ihrer Salze umwandelt.

Vor- und nachstehend haben die Reste $R^1$, $R^2$, $R^3$, $R^4$, $R^7$ und Y die bei der Formel I angegebenen Bedeutungen, sofern nicht ausdrücklich etwas anderes angegeben ist. Für alle Reste, die mehrfach auftreten, gilt, daß deren Bedeutungen unabhängig voneinander sind.

Abkürzungen

| | |
|---|---|
| Ac | Acetyl |
| BOC | tert.-Butoxycarbonyl |
| CBZ oder Z | Benzyloxycarbonyl |
| DCCI | Dicyclohexylcarbodiimid |
| DMF | Dimethylformamid |
| EDCI | N-Ethyl-N,N'-(dimethylaminopropyl)-carbodiimid |
| Et | Ethyl |
| Fmoc | 9-Fluorenylmethoxycarbonyl |
| HOBt | 1-Hydroxybenzotriazol |
| Me | Methyl |
| Mtr | 4-Methoxy-2,3,6-trimethylphenyl-sulfonyl |
| HONSu | N-Hydroxysuccinimid |
| OBut | tert.-Butylester |
| Oct | Octanoyl |
| OMe | Methylester |
| OEt | Ethylester |
| POA | Phenoxyacetyl |
| PPA | Polyphosphorsäure |
| TFA | Trifluoressigsäure |
| Trt | Trityl (Triphenylmethyl). |

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methyl-propyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
In A können auch eine oder zwei $CH_2$-Gruppen durch N, O- oder S-Atome und/oder durch -CH=CH-Gruppen ersetzt sein. So bedeutet A z.B. auch 2-Methoxy-ethyl oder 2-Hydroxyethyl.
A bedeutet weiterhin cyclisches Alkyl, vorzugsweise Cyclopropyl, Cyclobutyl, Cyclopentyl, Cyclohexyl oder Cycloheptyl.

A bedeutet weiterhin vorzugsweise unverzweigtes oder verzweigtes Alkyl mit 1-10 C-Atomen, worin eine $CH_2$-Gruppe durch ein O-Atom ersetzt sein kann und/oder auch 1-5 H-Atome durch F ersetzt sein können.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Hydroxyphenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Methylaminosulfonylphenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m- oder p-Acetylphenyl, o-, m- oder p-Cyanphenyl, o-, m- oder p-Acetaminophenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4-bromphenyl oder 2,5-Dimethyl-4-chlorphenyl.

Ar bedeutet vorzugsweise unsubstituiertes oder ein-, zwei- oder dreifach durch Hal und/oder $NR^5COA$ substituiertes Phenyl.

Het bedeutet, ungeachtetet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3-oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7-oder 8-Chinolyl, 1-, 3-, 4-, 5-, 6-, 7- oder 8-Isochinolyl, 3-, 4-, 5-, 6-, 7- oder 8-Cinnolinyl,

2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.

Unsubstituiertes Het kann also z. B. auch bedeuten 2,3-Dihydro-2-, -3-, -4-oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2-oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5-oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, - 6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)-phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet weiterhin vorzugsweise einen ein- oder zweikernigen gesättigten, ungesättigten oder aromatischen Heterocyclus mit 1 bis 4 N-, O- und/oder S-Atomen, der unsubstituiert oder ein- bis dreifach durch A, $COOR^5$ und/oder =O (Carbonylsauerstoff) substituiert sein kann.

Het bedeutet besonders bevorzugt unsubstituiertes oder ein- oder zweifach durch durch A, $COOR^5$ und/oder =O (Carbonylsauerstoff) substituiertes Piperidinyl, 4,5-Dihydro-pyridazinyl, Pyridyl, Morpholinyl, Piperazinyl, Pyrrolidinyl, Tetrahydrofuranyl, [1,3]Oxazinanyl, Thienyl, Pyrazolyl, Thiazolyl, Benzofuranyl, Isoxazolyl, Benzothienyl, 3,4-Dihydro-2H-pyrido[3,2-b][1,4]oxazinyl, Pyrrolyl, Pyrimidinyl, Furanyl, Imidazolyl, Oxazolyl, Triazolyl, Tetrazolyl, Thiadiazol, Pyridazinyl, Pyrazinyl, Indolyl, Isoindolyl, Benzimidazolyl, Indazolyl, Chinolyl, 1,3-Benzodioxolyl, Tetrahydro-imidazolyl, Tetrahydro-pyrazolyl, Oxazolidinyl oder Isoxazolidinyl.

$Het^1$ bedeutet vorzugsweise einen einkernigen gesättigten Heterocyclus mit 1 bis 2 N und/oder O-Atomen, der ein- oder zweifach durch A und/oder =O substituiert sein kann.
$Het^1$ bedeutet ganz besonders bevorzugt unsubstituiertes oder ein- oder zweifach durch A und/oder =O substituiertes Pyrrolidinyl, Tetrahydro-imidazolyl, Tetrahydro-pyrazolyl, Piperidinyl, Morpholinyl, Piperazinyl, Oxazolidinyl oder Isoxazolidinyl.

Ungeachtet weiterer Substitutionen bedeutet $R^1$ einen ein- oder zweikernigen gesättigten, ungesättigten oder aromatischen Heterocyclus mit 1 bis 4 N-, O- und/oder S-Atomen, worin der Heterocyclus die Bedeutungen hat, wie oben für Het angegeben.
$R^1$ bedeutet vorzugsweise einen ein- oder zweikernigen gesättigten, ungesättigten oder aromatischen Heterocyclus mit 1 bis 4 N-, O- und/oder S-Atomen, der unsubstituiert oder ein- oder zweifach durch Hal, A, $OR^5$, CN, $[C(R^5)_2]_nCOOR^5$, $[C(R^5)_2]_nCON(R^5)_2$, $[C(R^5)_2]_n$,$CONR^5[C(R^5)_2]_nAr$, $[C(R^5)_2]_nCONR^5[C(R^5)_2]_nHet$, $[C(R^5)_2]_nCO$-Het, $[C(R^5)_2]_nN(R^5)_2$, $[C(R^5)_2]_nAr$, $[C(R^5)_2]_nHet$, $[C(R^5)_2]_nOR^5$ und/oder $[C(R^5)_2]_nCONR^5[C(R^5)_2]_nN(R^5)_2$ substituiert sein kann.
$R^1$ bedeutet besonders bevorzugt unsubstituiertes oder ein- oder zweifach durch A, $CH_2COHet$, $(CH_2)_nOH$, $(CH_2)_nHet$, $CONH(CH_2)_nHet$, $(CH_2)_nNH_2$, OA, Hal, $(CH_2)_nNHA$, $(CH_2)_nNA_2$, $CH_2Ar$, $CH_2CONA_2$, $(CH_2)_nOA$, $(CH_2)_nCOOH$, $(CH_2)_nCOOA$, $CONH(CH_2)_2NA_2$, $CONH_2$, CONHA, $CONA_2$, $CONH(CH_2)_2OA$ und/oder CN substituiertes Pyrazolyl, Thiazolyl, Thienyl, Pyridyl, Benzofuranyl, Isoxazolyl, Benzothienyl, 3,4-Dihydro-2H-pyrido[3,2-b][1,4]oxazinyl, Pyrrolyl, Pyrimidinyl, Furanyl, Imidazolyl, Oxazolyl, Triazolyl, Tetrazolyl, Thiadiazol, Pyridazinyl, Pyrazinyl, Indolyl, Isoindolyl, Benzimidazolyl, Indazolyl, Chinolyl oder 1,3-Benzodioxolyl.

$R^2$, $R^3$, $R^4$, $R^7$, bedeuten vorzugsweise, jeweils unabhängig voneinander H, Hal, A, OA, $SO_2N(R^5)_2$, $S(O)_mHet$, CO-Ar, $[C(R^5)_2]_nAr$, $[C(R^5)_2]_nHet$, $C(R^5)(OR^5)Ar$, $[C(R^5)_2]_nOR^5$, $[C(R^5)_2]_nOAr$, $[C(R^5)_2]_nN(R^5)_2$, $[C(R^5)_2]_nCON(R^5)_2$, $[C(R^5)_2]_nCOOR^5$, $[C(R^5)_2]_nCONR^5[C(R^5)_2]_nAr$, $[C(R^5)_2]_nCONR^5[C(R^5)_2]_nHet$ oder $[C(R^5)_2]_nNR^5CO[C(R^5)_2]_nAr$,
mit der Maßgabe, daß

a) wenn ein Y N bedeutet, $R^7$ fehlt;
b) wenn zwei Y N bedeuten, $R^4$ und $R^7$ fehlen;
zwei benachbarte Reste,
ausgewählt aus der Gruppe $R^2$, $R^3$, $R^4$, $R^7$ zusammen auch $OCH_2O$, $OCH_2CH_2O$, NHCONH, $OCF_2O$, CH=N-NH oder NH-N=CH.

$R^2$, $R^3$, $R^4$, $R^7$, bedeuten besonders bevorzugt, jeweils unabhängig voneinander H, $(CH_2)_nOH$, $(CH_2)_nOA$, $(CH_2)_nHet$, Hal, A, $SO_2NH_2$, $SO_2NHA$, $SO_2NA_2$, $(CH_2)_nAr$, CH(OH)Ar, $(CH_2)_nNHCO(CH_2)_nAr$, COAr, $(CH_2)_nCONH(CH_2)_nAr$, $(CH_2)_nCONH(CH_2)_nHet$, $(CH_2)_nCOOH$, $(CH_2)_nCOOA$, $(CH_2)_nCONH_2$, $(CH_2)_nCONHA$, $(CH_2)_nCONA_2$, $SO_2Het$, $(CH_2)_nNH_2$, $(CH_2)_nNHA$, $(CH_2)_nNA_2$, $(CH_2)_nOAr$ oder $CONH(CH_2)_2OA$,
mit der Maßgabe, daß

a) wenn ein Y N bedeutet, $R^7$ fehlt;
b) wenn zwei Y N bedeuten, $R^4$ und $R^7$ fehlen;

zwei benachbarte Reste,
ausgewählt aus der Gruppe $R^2$, $R^3$, $R^4$, $R^7$
zusammen auch $OCH_2O$, $OCH_2CH_2O$, NHCONH, $OCF_2O$, CH=N-NH oder NH-N=CH,

Hal bedeutet vorzugsweise F, Cl oder Br, aber auch I, besonders bevorzugt F oder Cl.

Für die gesamte Offenbarung gilt, daß sämtliche Reste, die mehrfach auftreten, gleich oder verschieden sein können, d.h. unabhängig voneinander sind. Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Offenbarung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ij ausgedrückt werden, die der Formel I entsprechen und worin die nicht näher bezeichneten Reste die bei der Formel I angegebene Bedeutung haben, worin jedoch

in Ia $R^1$ einen ein- oder zweikernigen gesättigten, ungesättigten oder aromatischen Heterocyclus mit 1 bis 4 N-, O- und/oder S-Atomen, der unsubstituiert oder ein- oder zweifach durch Hal, A, $OR^5$, CN, $[C(R^5)_2]_nCOOR^5$, $[C(R^5)_2]_nCON(R^5)_2$, $[C(R^5)_2]_nCONR^5[C(R^5)_2]_nAr$, $[C(R^5)_2]_nCONR^5[C(R^5)_2]_nHet$, $[C(R^5)_2]_nCO$-Het, $[C(R^5)_2]_nN(R^5)_2$, $[C(R^5)_2]_nAr$ $[C(R^5)_2]_nHet$, $[C(R^5)_2]_nOR^5$ und/oder $[C(R^5)_2]_nCONR^5[C(R^5)_2]_nN(R^5)_2$ substituiert sein kann
bedeutet;

in Ib $R^1$ unsubstituiertes oder ein- oder zweifach durch A, $CH_2COHet$, $(CH_2)_nOH$, $(CH_2)_nHet$, $CONH(CH_2)_nHet$, $(CH_2)_nNH_2$, OA, Hal, $(CH_2)_nNHA$, $(CH_2)_nNA_2$, $CH_2Ar$, $CH_2CONA_2$, $(CH_2)_nOA$, $(CH_2)_nCOOH$, $(CH_2)_nCOOA$, $CONH(CH_2)_2NA_2$, $CONH_2$, CONHA, $CONA_2$, $CONH(CH_2)_2OA$ und/oder CN substituiertes Pyrazolyl, Thiazolyl, Thienyl, Pyridyl, Benzofuranyl, Isoxazolyl, Benzothienyl, 3,4-Dihydro-2H-pyrido[3,2-b][1,4]oxazinyl, Pyrrolyl, Pyrimidinyl, Furanyl, Imidazolyl, Oxazolyl, Triazolyl, Tetrazolyl, Thiadiazol, Pyridazinyl, Pyrazinyl, Indolyl, Isoindolyl, Benzimidazolyl, Indazolyl, Chinolyl oder 1,3-Benzodioxolyl
bedeutet;

in $I_C$ $R^2$, $R^3$, $R^4$, $R^7$ jeweils unabhängig voneinander H, Hal, A, OA, $SO_2N(R^5)_2$, $S(O)_mHet$, CO-Ar, $[C(R^5)_2]_nAr$, $[C(R^5)_2]_nHet$, $C(R^5)(OR^5)Ar$, $[C(R^5)_2]_nOR^5$, $[C(R^5)_2]_nOAr$, $[C(R^5)_2]_nN(R^5)_2$, $[C(R^5)_2]_nCON(R^5)_2$, $[C(R^5)_2]_nCOOR^5$, $[C(R^5)_2]_nCONR^5[C(R^5)_2]_nAr$, $[C(R^5)_2]_nCONR^5[C(R^5)_2]_nHet$ oder $[C(R^5)_2]_nNR^5CO[C(R^5)_2]_nAr$
mit der Maßgabe, daß

a) wenn ein Y N bedeutet, $R^7$ fehlt;
b) wenn zwei Y N bedeuten, $R^4$ und $R^7$ fehlen;

zwei benachbarte Reste,
ausgewählt aus der Gruppe $R^2$, $R^3$, $R^4$, $R^7$
zusammen auch $OCH_2O$, $OCH_2CH_2O$, NHCONH, $OCF_2O$, CH=N-NH oder NH-N=CH
bedeuten;

in Id $R^2$, $R^3$, $R^4$, $R^7$ jeweils unabhängig voneinander H, $(CH_2)_nOH$, $(CH_2)_nOA$, Het, Hal, A, $SO_2NH_2$, $SO_2NHA$, $SO_2NA_2$, $(CH_2)_nAr$, $(CH_2)_nHet$, CH(OH)Ar, $(CH_2)_nNHCO(CH_2)_nAr$, COAr, $(CH_2)_nCONH(CH_2)_nAr$, $(CH_2)_nCONH(CH_2)_nHet$, $(CH_2)_nCOOH$, $(CH_2)_nCOOA$, $(CH_2)_nCONH_2$, $(CH_2)_nCONHA$, $(CH_2)_nCONA_2$, $SO_2Het$, $(CH_2)_nNH_2$, $(CH_2)_nNHA$, $(CH_2)_nNA_2$, $(CH_2)_nOAr$ oder $CONH(CH_2)_2OA$
mit der Maßgabe, daß

a) wenn ein Y N bedeutet, $R^7$ fehlt;
b) wenn zwei Y N bedeuten, $R^4$ und $R^7$ fehlen;

zwei benachbarte Reste,
ausgewählt aus der Gruppe $R^2$, $R^3$, $R^4$, $R^7$
zusammen auch $OCH_2O$, $OCH_2CH_2O$, NHCONH, $OCF_2O$, CH=N-NH oder NH-N=CH

bedeuten;

in Ie A — unverzweigtes oder verzweigtes Alkyl mit 1-10 C-Atomen, worin 1-5 H-Atome durch F ersetzt sein können und/oder worin eine $CH_2$-Gruppe durch O ersetzt sein kann bedeutet;

in If Ar — unsubstituiertes oder ein-, zwei- oder dreifach durch Hal und/oder $NR^5COA$ substituiertes Phenyl bedeutet;

in Ig Het — einen ein- oder zweikernigen gesättigten, ungesättigten oder aromatischen Heterocyclus mit 1 bis 4 N-, O- und/oder S-Atomen, der unsubstituiert oder ein- bis dreifach durch A, $COOR^5$ und/oder =O (Carbonylsauerstoff) substituiert sein kann bedeutet;

in Ih Het — unsubstituiertes oder ein- oder zweifach durch durch A,$COOR^5$ und/oder =O (Carbonylsauerstoff) substituiertes Piperidinyl, 4,5-Dihydro-pyridazinyl, Pyridyl, Morpholinyl, Piperazinyl, Pyrrolidinyl, Tetrahydrofuranyl, [1,3]Oxazinanyl, Thienyl, Pyrazolyl, Thiazolyl, Benzofuranyl, Isoxazolyl, Benzothienyl, 3,4-Dihydro-2H-unsubstituiertes oder ein- oder zweifach durch durch A,$COOR^5$ pyrido[3,2-b][1,4]oxazinyl, Pyrrolyl, Pyrimidinyl, Furanyl, Imidazolyl, Oxazolyl, Triazolyl, Tetrazolyl, Thiadiazol, Pyridazinyl, Pyrazinyl, Indolyl, Isoindolyl, Benzimidazolyl, Indazolyl, Chinolyl, 1,3-Benzodioxolyl, Tetrahydro-imidazolyl, Tetrahydro-pyrazolyl, Oxazolidinyl oder Isoxazolidinyl bedeutet;

in Ii $Het^1$ — unsubstituiertes oder ein- oder zweifach durch A und/oder =O substituiertes Pyrrolidinyl, Tetrahydro-imidazolyl, Tetrahydro-pyrazolyl, Piperidinyl, Morpholinyl, Piperazinyl, Oxazolidinyl oder Isoxazolidinyl, bedeutet;

in Ij $R^1$ — einen ein- oder zweikernigen gesättigten, ungesättigten oder aromatischen Heterocyclus mit 1 bis 4 N-, O- und/oder S-Atomen, der unsubstituiert oder ein- oder zweifach durch Hal, A, $OR^5$, CN, $[C(R^5)_2]_NCOOR^5$, $[C(R^5)_2]_nCON(R^5)_2$, $[C(R^5)_2]_nCONR^5[C(R^5)_2]_nAr$, $[C(R^5)_2]_nCONR^5[C(R^5)_2]_nHet$, $[C(R^5)_2]_nCO\text{-}Het$, $[C(R^5)_2]_nN(R^5)_2$, $[C(R^5)_2]_nAr$, $[C(R^5)_2]_nHet$, $[C(R^5)_2]_nOR^5$ und/oder $[C(R^5)_2]_nCONR^5[C(R^5)_2]_nN(R^5)_2$ substituiert sein kann,

Y — unabhängig voneinander N oder C, wobei maximal zwei Y N bedeuten können,

$R^2$, $R^3$, $R^4$, $R^7$ — jeweils unabhängig voneinander H, Hal, A, OA, $SO_2N(R^5)_2$, $S(O)_mHet$, CO-Ar, $[C(R^5)_2]_nAr$, $[C(R^5)_2]_nHet$, $C(R^5)(OR^5)Ar$, $[C(R^5)_2]_nOR^5$, $[C(R^5)_2]_nOAr$, $[C(R^5)_2]_nN(R^5)_2$, $[C(R^5)_2]_nCON(R^5)_2$, $[C(R^5)_2]_nCOOR^5$, $[C(R^5)_2]_nCONR^5[C(R^5)_2]_nAr$, $[C(R^5)_2]_nCONR^5[C(R^5)_2]_nHet$ oder $[C(R^5)_2]_nNR^5CO[C(R^5)_2]_nAr$
mit der Maßgabe, daß

a) wenn ein Y N bedeutet, $R^7$ fehlt;
b) wenn zwei Y N bedeuten, $R^4$ und $R^7$ fehlen;

zwei benachbarte Reste,
ausgewählt aus der Gruppe $R^2$, $R^3$, $R^4$, $R^7$zusammen auch $OCH_2O$, $OCH_2CH_2O$, NHCONH, $OCF_2O$, CH=N-NH oder NH-N=CH,

$R^5$ — A oder H,

A — unverzweigtes oder verzweigtes Alkyl mit 1-10 C-Atomen, worin 1-5 H-Atome durch F ersetzt sein können und/oder worin eine $CH_2$-Gruppe durch O ersetzt sein kann,

Ar — unsubstituiertes oder ein-, zwei- oder dreifach durch Hal und/oder $NR^5COA$ substituiertes Phenyl,

Het — einen ein- oder zweikernigen gesättigten, ungesättigten oder aromatischen Heterocyclus mit 1 bis 4 N-, O- und/oder S-Atomen, der unsubstituiert oder ein- bis dreifach durch A, $COOR^5$ und/oder

=O (Carbonylsauerstoff) substituiert sein kann,

| | |
|---|---|
| Hal | F, Cl, Br oder I, |
| m | 0, 1 oder 2, |
| n | 0, 1, 2, 3 oder 4, bedeuten; |

sowie ihre pharmazeutisch verwendbaren Salze, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man eine Verbindung der Formel II mit einer Verbindung der Formel III umsetzt.

Die Ausgangsverbindungen der Formeln II und III sind in der Regel bekannt. Sind sie neu, so können sie aber nach an sich bekannten Methoden hergestellt werden.

Die Umsetzung erfolgt in einem inerten Lösungsmittel und erfolgt in der Regel in Gegenwart von $NaHSO_3/Na_2S_2O_5$.

Die Reaktionszeit liegt je nach den angewendeten Bedingungen zwischen einigen Minuten und 14 Tagen, die Reaktionstemperatur zwischen etwa -15° und 150°, normalerweise zwischen 10° und 100°, besonders bevorzugt zwischen 15° und 80°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmono-methyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Besonders bevorzugt ist DMF.

Verbindungen der Formel I können weiterhin vorzugsweise erhalten werden, indem man eine Verbindung der Formel IV mit einer Verbindung der Formel V umsetzt.

Die Umsetzung erfolgt unter Bedingungen wie sie dem Fachmann für eine Suzuki-Reaktion bekannt sind.

Die Ausgangsverbindungen der Formeln IV und V sind in der Regel bekannt. Sind sie neu, so können sie aber nach an sich bekannten Methoden hergestellt werden.

In den Verbindungen der Formel V bedeutet L vorzugsweise

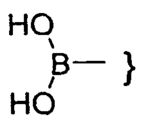 oder 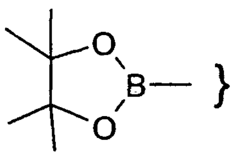 .

Die Umsetzung erfolgt unter Standardbedingungen einer Suzuki-Kopplung. Die Reaktionszeit liegt je nach den angewendeten Bedingungen zwischen einigen Minuten und 14 Tagen, die Reaktionstemperatur zwischen etwa -30° und 140°, normalerweise zwischen 20° und 150°, insbesondere zwischen etwa 60° und etwa 130°.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Besonders bevorzugt ist DMF.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

<u>Pharmazeutische Salze und andere Formen</u>

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methyl-glutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie ($C_1$-$C_4$) Alkylhalogeniden, z.B. Methyl-, Ethyl-, Isopropyl- und tert.-Butylchlorid, -bromid und -iodid; Di($C_1$-$C_4$)Alkylsulfaten, z.B. Dimethyl-, Diethyl- und Diamylsulfat; ($C_{10}$-$C_{18}$)Alkylhalogeniden, z.B. Decyl-, Dodecyl-, Lauryl-, Myristyl- und Stearylchlorid, -bromid und -iodid; sowie Aryl-($C_1$-$C_4$)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien

Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Offenbarung sind ferner Arzneimittel, enthaltend mindestens eine Verbindung der Formel I und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol Glyzerin, Wasser u.ä. kombinierten. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie

Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxyrnethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Tautomeren und Stereoisomeren davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Tautomeren und Stereoisomeren davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder

suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandeln den Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, oben erwähnten Krankheitszustände geeignet sind.

Gegenstand der Offenbarung sind ferner Arzneimittel enthaltend mindestens eine Verbindung der Formel I und/oder ihre pharmazeutisch verwendbaren Salze und Stereoisomere, einschließlich deren Mischungen in allen Verhältnissen, und mindestens einen weiteren Arzneimittelwirkstoff.

Gegenstand der Offenbarung ist auch ein Set (Kit), bestehend aus getrennten Packungen von

(a) einer wirksamen Menge an einer Verbindung der Formel I und/oder ihrer pharmazeutisch verwendbaren Salze und Stereoisomere, einschließlich deren Mischungen in allen Verhältnissen,
und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze und Stereoisomere, einschließlich deren Mischungen in allen Verhältnissen, und einer wirksamen Menge eines weiteren Arzneimittelwirkstoffs gelöst oder in lyophilisierter Form vorliegt.

## VERWENDUNG

Die vorliegenden Verbindungen eignen sich als pharmazeutische Wirkstoffe für Säugetiere, insbesondere für den Menschen, bei der Behandlung und Bekämpfung von Krebserkrankungen.

Die vorliegende Offenbarung umfasst die Verwendung der Verbindungen der Formel I und/oder ihre physiologisch unbedenklichen Salze, Tautomeren und Stereoisomeren zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom Darmkrebs. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.

Ebenfalls umfasst ist die Verwendung der Verbindungen der Formel I und/oder ihre physiologisch unbedenklichen Salze, Tautomeren und Stereoisomeren zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Gegenstand der Offenbarung ist weiterhin die Verwendung der offenbarten Verbindungen zur Behandlung von Knochen-Pathologien, wobei die Knochenpathologie aus der Gruppe Osteosarkom, Osteoarthritis und Rachitis stammt.

Die Verbindungen der Formel I können auch gemeinsam mit anderen gut bekannten Therapeutika, die aufgrund ihrer jeweiligen Eignung für das behandelte Leiden ausgewählt werden, verabreicht werden.
Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie.

"Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzophenon-2 ,4-dinitrophenylhyd razon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5$\alpha$-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, $\alpha$-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin,

Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1 H,12H-benzo[de]-pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo-(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

**Wirkungsnachweis von pharmakologischen Inhibitoren auf die Proliferation/Vitalität von Tumorzellen *in vitro***

**1.0 Hintergrund**

In der vorliegenden Versuchsbeschreibung wird die Hemmung der Tumorzellproliferation/ Tumorzellvitalität durch Wirkstoffe beschrieben.
Die Zellen werden in geeigneter Zelldichte in Mikrotiterplatten (96-well Format) ausgesät und die Testsubstanzen werden in Form einer Konzentrationreihe zugegeben. Nach vier weiteren Tagen der Kultivierung in serumhaltigem Medium kann die Tumorzellproliferation/ Tumorzellvitalität mittels eines Alamarblue-Testsystem bestimmt werden.

**2.0 Versuchsdurchführung**

**2.1 Zellkultur**

Beispielsweise käuflich erhältliche Colon-Carcinom-Zelllinien, Zelllinien des Eierstocks, Zelllinien der Prostata oder Zelllinien der Brust etc.
Die Zellen werden in Medium kultiviert. In Abständen von mehreren Tagen werden die Zellen mit Hilfe von Trypsin-Lösung von den Kulturschalen abgelöst und in geeigneten Verdünnung in frischem Medium ausgesät. Die Zellen werden bei 37° Celsius und 10% $CO_2$ kultiviert.

**2.2. Aussaat der Zellen**

Eine definierte Zellzahl (z.B. 2000 Zellen) werden pro Kultur/ well in einem Volumen von 180μl Kulturmedium in Mikrotiterplatten (96 well Zellkulturplatten) mit einer Mehrkanalpipette ausgesät. Die Zellen werden anschließend in einem CO2-Brutschrank (37°C und 10% CO2) kultiviert.

### 2.3. Zugabe der Testsubstanzen

Die Testsubstanzen werden beispielsweise in DMSO gelöst und anschließend in entsprechender Konzentration (gegebenenfalls einer Verdünnungsreihe) im Zellkulturmedium eingesetzt. Die Verdünnungs-stufen können je nach Effizienz der Wirkstoffe und gewünschter Spreizung der Konzentrationen angepasst werden. Die Testsubstanzen werden in entsprechenden Konzentrationen mit Zellkulturmedium versetzt. Die Zugabe der Testsubstanzen zu den Zellen kann am selben Tag wie die Aussat der Zellen erfolgen. Dazu wird aus der Vorverdünnungsplatte jeweils 20μl Substanzlösung in die Kulturen/wells gegeben. Die Zellen werden für weitere 4 Tage bei 37°Celsius und 10% $CO_2$ kultiviert.

### 2.4. Messung der Farbreaktion

Pro well werden jeweils 20μl AlamarBlue Reagenz gegeben und die Microtiterplatten werden beispielsweise für weitere sieben Stunden in einem CO2-Brutschrank (bei 37°C und 10% CO2) inkubiert. Die Platten werden an einem Reader mit einem Fluoreszenzfilter bei einer Wellenlänge von 540nm gemessen. Die Platten können direkt vor der Messung leicht geschüttelt werden.

### 3. Auswertung

Der Extinktionswert der Mediumkontrolle (keine Verwendung von Zellen und Testsubstanzen) wird von allen anderen Extinktionswerten subtrahiert. Die Kontrollen (Zellen ohne Testsubstanz) werden gleich 100 Prozent gesetzt und alle anderen Extinktionswerte hierzu in Beziehung gesetzt (beispielsweise in % der Kontrolle) ausgedrückt:

Rechnung:

$$\frac{100 * (\text{Wert mit Zellen und Testsubstanz - Wert der Mediumkontrolle})}{(\text{Wert mit Zellen - Wert der Mediumkontrolle})}$$

Die Bestimmung von $IC_{50}$ Werten (50%ige Hemmung) erfolgt mit Hilfe von Statistikprogrammen wie z.B. RS1. $IC_{50}$-Daten erfindungsgemäßer Verbindungen sind in Tabelle 1 angegeben.

### 4.0 Test zur Inhibierung von PDK1

Die Versuchsansätze werden in einem Flashplate-System mit 384 wells/Mikrotitrierplatte durchgeführt. Pro well werden jeweils die PDK1-Probe $His_6$-PDK1(□1-50)( 3.4 nM), das PDK1-Substrat Biotin-bA-bA-KTFCGTPEYLAPEVRREP-RILSEEEQEMFRDFDYIADWC (400 nM), 4 μM ATP (mit 0.2μCi $^{33}$P-ATP/well) und die Testsubstanz in 50μl gebräuchlicher Versuchslösung für 60 Min bei 30°C inkubiert. Die Testsubstanzen werden in entsprechenden Konzentrationen (gegebenenfalls in einer Verdünnungsreihe) eingesetzt. Die Kontrolle wird ohne Testsubstanz durchgeführt. Die Reaktion wird mit gängigen Methoden gestoppt und gewaschen. Die Aktivität der Kinase wird über die eingebaute Radioaktivität im Topcount gemessen. Zur Bestimmung der unspezifischen Kinasereaktion (Leerwert) werden die Versuchsansätze in Gegenwart von 100 nM Staurosporin durchgeführt.

### 5.0 Auswertung

Die Radioaktivität (Zerfälle pro Minute) des Leerwerts (keine Verwendung von Testsubstanz in Gegenwart von Staurosporin) wird von allen anderen Radioaktivitätswerten substrahiert. Die Kontrollen (Kinaseaktivität ohne Testsubstanz) werden gleich 100 Prozent gesetzt und alle anderen Radioaktivitätswerte (nach Abzug des Leerwerts) hierzu in Beziehung gesetzt (beispielsweise in % der Kontrolle) ausgedrückt.

Rechnung:

$$\frac{100* (\text{Wert der Kinaseaktivität mit Testsubstanz - Leerwert})}{(\text{ Wert der Kontrolle - Leerwert})}$$

= % der Kontrolle

Die Bestimmung von $IC_{50}$ Werten (50%ige Hemmung) erfolgt mit Hilfe von Statistikprogrammen wie z.B. RS1.

$IC_{50}$-Daten erfindungsgemäßer Verbindungen sind in Tabelle 1 angegeben.

| Material | Best. Nr. | Hersteller |
|---|---|---|
| Mikrotiterplatten für die Zellkultur (Nunclon Surface 96well Plate) | 167008 | Nunc |
| DMEM | P04-03550 | Pan Biotech |
| PBS (10x) Dulbecco | 14200-067 | Gibco |
| 96well Platten (Polypropylen) | 267334 | Nunc |
| AlamarBlue™ | BUF012B | Serotec |
| FCS | 1302 | Pan Biotech GmbH |
| Trypsin/EDTA Solution 10x | L 2153 | Biochrom AG |
| 75cm$^2$ Kulturflaschen | 353136 | BD Falcon |
| A2780 | 93112519 | ECACC |
| Colo205 | CCL222 | ATCC |
| MCF7 | HTB22 | ATCC |
| PC3 | CRL-1435 | ATCC |
| 384well Flash Platten | SMP410A001PK | Perkin Elmer |

**1.Test zur Inhibierung von IRAK4**

Die Versuchsansätze werden in einem FlashPlate-System mit 384 wells/Mikrotiterplatte durchgeführt. Pro well werden jeweils die IRAK4-Probe $His_6$-IRAK4 (7.3 nM), das biotinylierte Substratpeptid STK Substrat.1-Biotin (Cisbio Bioassays, France) (300 nM), 10 μM ATP (mit 0.25 μCi $^{33}$P-ATP/well) und die Testsubstanz in 50μl gebräuchlicher Versuchslösung für 180 Min bei 22°C inkubiert. Die Testsubstanzen werden in entsprechenden Konzentrationen (gegebenenfalls in einer Verdünnungsreihe) eingesetzt. Die Kontrolle wird ohne Testsubstanz durchgeführt. Die Reaktion wird mit gängigen Methoden gestoppt und gewaschen. Die Aktivität der Kinase wird über die eingebaute Radioaktivität im Topcount gemessen. Zur Bestimmung der unspezifischen Kinasereaktion (Leerwert) werden die Versuchsansätze in Gegenwart von 1 μM Staurosporin durchgeführt.

**2.Auswertung**

Die Radioaktivität (Zerfälle pro Minute) des Leerwerts (keine Verwendung von Testsubstanz, in Gegenwart von 1 μM Staurosporin) wird von allen anderen Radioaktivitätswerten substrahiert. Die Kontrollen (Kinaseaktivität ohne Testsubstanz) werden gleich 100 Prozent gesetzt und alle anderen Radioaktivitätswerte (nach Abzug des Leerwerts) hierzu in Beziehung gesetzt (beispielsweise in % der Kontrolle) ausgedrückt.
Rechnung:

$$\frac{100^{*}\,(\text{Wert der Kinaseaktivität mit Testsubstanz - Leerwert})}{(\text{ Wert der Kontrolle - Leerwert})}$$

$$= \%\ \text{der Kontrolle}$$

Die Bestimmung von $IC_{50}$ Werten (50%ige Hemmung) erfolgt mit Hilfe von Statistikprogrammen wie z.B. RS1.

| Material | Hersteller | Bestellnummer |
|---|---|---|
| Hepes | Merck | 1.10110 |
| $MnCl_2$ | Merck | 1.05934 |
| EDTA, 0.5 M | Sigma | E-7889 |
| EGTA | Merck | 1.08435 |
| DMSO | Merck | 1.02952 |
| [33P]-ATP | Perkin Elmer | NEG302H |
| Spec. radioact. 3000 Ci/mmol | | |
| 1 mCi/0.1ml | | |
| Staurosporin | LC Labs | S-9300 |

(fortgesetzt)

| Material | Hersteller | Bestellnummer |
|---|---|---|
| BSA,30% | Sigma | A9205 |
| $MgCl_2$ | Merck | 1.05833 |
| ATP | Calbiochem | 1191 |
| IRAK-4 | Merck | Merck, Lab. Dr.Jaekel |
| STK 1-biotin | Cisbio | 61ST1BLE/C |
| Tween-60 | Merck | 8.22186 |
| NaCl | Merck | 1.06404 |
| FlashPlate 384 HTS Streptavidin coated | Perkin Elmer | SMP410A |

**1.Test zur Inhibierung von IRAK1**

Die Versuchsansätze werden in einem FlashPlate-System mit 384 wells/Mikrotiterplatte durchgeführt. Zur Untersuchung der Inhibition der Autophosphorylierungsaktivität von IRAK1 werden pro well jeweils die IRAK1-Probe $His_6$-IRAK1 (5.1 nM), 1 μM ATP (mit 0.25 μCi $^{33}$P-ATP/well) und die Testsubstanz in 50μl gebräuchlicher Versuchslösung für 180 Min bei 22°C inkubiert. Die Testsubstanzen werden in entsprechenden Konzentrationen (gegebenenfalls in einer Verdünnungsreihe) eingesetzt. Die Kontrolle wird ohne Testsubstanz durchgeführt. Die Reaktion wird mit gängigen Methoden gestoppt und gewaschen. Die Aktivität der Kinase wird über die eingebaute Radioaktivität im Topcount gemessen. Zur Bestimmung der unspezifischen Kinasereaktion (Leerwert) werden die Versuchsansätze in Gegenwart von 1 μM Staurosporin durchgeführt.

**2.Auswertung**

Die Radioaktivität (Zerfälle pro Minute) des Leerwerts (keine Verwendung von Testsubstanz, in Gegenwart von 1 μM Staurosporin) wird von allen anderen Radioaktivitätswerten substrahiert. Die Kontrollen (Kinaseaktivität ohne Testsubstanz) werden gleich 100 Prozent gesetzt und alle anderen Radioaktivitätswerte (nach Abzug des Leerwerts) hierzu in Beziehung gesetzt (beispielsweise in % der Kontrolle) ausgedrückt.
Rechnung:

$$\frac{100^{*}\,(\text{Wert der Kinaseaktivität mit Testsubstanz - Leerwert})}{(\text{Wert der Kontrolle - Leerwert})} = \%\ \text{der Kontrolle}$$

Die Bestimmung von $IC_{50}$ Werten (50%ige Hemmung) erfolgt mit Hilfe von Statistikprogrammen wie z.B. RS1.

| Material | Hersteller | Bestellnummer |
|---|---|---|
| Hepes | Merck | 1.10110 |
| EDTA | VWR | E-7889 |
| EDTA | Merck | 1.08418 |
| EGTA | Merck | 1.08435 |
| DMSO | Merck | 1.02952 |
| Staurosporin | LC Labs | S-9300 |
| [33P]-ATP | Perkin Elmer | NEG302H |
| Spec. radioact. 3000 Ci/mmol | | |
| 1 mCi/0.1ml | | |
| BSA, 30% | Sigma | A9205 |
| $MgCl_2$ | Merck | 1.05833 |
| ATP | Calbiochem | 1191 |
| I RAK-1 | Merck | Merck, Lab. Dr.Jaekel |
| | | Pool B, 232Y08C1.IRK |
| Triton X-100 | Sigma | X-100 |

(fortgesetzt)

| Material | Hersteller | Bestellnummer |
|---|---|---|
| NaCl | Merck | 1.06404 |

APCI-MS (atmospheric pressure chemical ionization - mass spectrometry) $(M+H)^+$.
ESI-MS (electrospray ionization - mass spectrometry) $(M+H)^+$.
HPLC-APCI-MS & HPLC-ESI-MS Conditions
Solvent A:Wasser + 0.1%HCOOH
Solvent B:Acetonitril + 0.1% HCOOH
Solvent C:C
Solvent D:D
Min pressure (Bar):0
Max pressure (Bar):300
Delay volume (ml):0.00
Equilibration time (min):0.00
Gradient curve:Linear

Gradient program:

| Time(min) | Flow(mL/min) | A(%) | B(%) | C(%) | D(%) |
|---|---|---|---|---|---|
| 0.00 | 0.50 | 98 | 2 | 0 | 0 |
| 5.00 | 0.50 | 2 | 98 | 0 | 0 |
| 8.00 | 0.50 | 2 | 98 | 0 | 0 |
| 8.10 | 0.50 | 98 | 2 | 0 | 0 |
| 13.00 | 0.50 | 98 | 2 | 0 | 0 |

Säule: Purosphere RP-18, 55-2, Art. 1.50241.0001, Lot.: 641047

| | | |
|---|---|---|
| Pumpe: | Flux Instruments Rheos 2000<br>Finnigan MAT Spectra System P4000 | |
| Detektion : | Finnigan Surveyor PDA Detector<br>Finnigan MAT Spectra System UV 6000LP | |
| MS: | Finnigan LCQ Deca XP Plus<br>ESI interface<br>Positive/negative ionisation | Finnigan LCQ DECA<br>APCI interface<br>Positive ionisation |

EI-MS (electon impact - mass spectrometry) $M^+$

- EI Ionisierung 70 eV
- Ionenquellentemperatur 220°C, Direktverdampfung
- Massenspektrometer: VG Autospec
  LC-MS (high performance liquid chromotagraphy - mass spectrometry) $(M+H)^+$ .
  Die MS Daten werden wie folgt erhalten: Massenspectrum: $(M+H)^+$; Agilent System der 1100 Serie (Ionenquelle: Electrospray (positive mode); Scan: 85-1000 m/z; Fragmentation-voltage: variabel; Gastemperatur: 300°C, DAD: 220 nm. Flußrate: 2.4 ml/Min. Der verwendete Splitter reduziert die flow rate nach dem DAD für die MS auf 0,6 ml/Min; Säule: Chromolith Speed ROD RP-18e 50-4.6; Lösungsmittel: LiChrosolv Merck KGaA; Lösungsmittel A: $H_2O$ (0.05% Ameisensäure); Lösungsmittel B: $CH_3CN$ (0.04% Ameisensäure); Gradient: In 2.8 min von 96 % A bis 100 % B; gefolgt von 0.5 min 100 % B.

<u>Bedingungen präparative HPLC:</u>

Säule: Chromolith-prep RP-18e 100-25
Anlage: Shimadzu LC 8A

Eluent A: Wasser + 0,1% TFA
Eluent B: Acetonitril + 0,1% TFA
Gradient: 99:1 -->1:99 in 15 min.
Fluss: 30 ml/min
Detektion: UV 220 nm
$^1$H-NMR: Bruker DPX-300, DRX-500, DRX-400 oder AVII-400.

Die Mikrowellenchemie wird durchgeführt mit einem "single mode microwave reactor Emrys™ Optimiser" von Personal Chemistry.

Allgemeine Syntheseschemata zur Herstellung offenbarter Verbindungen

Weg 1:

Route A

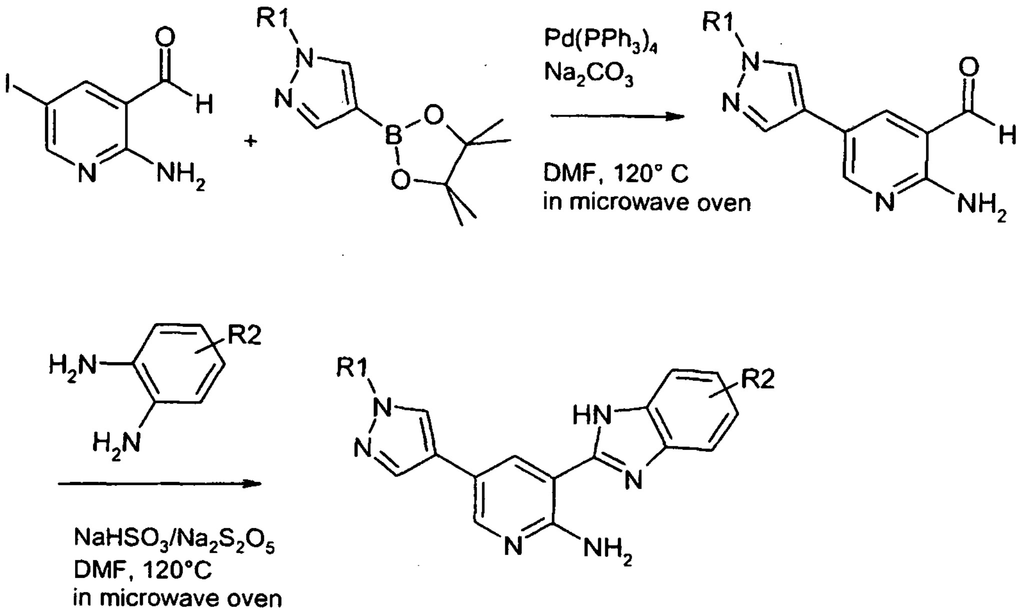

Route B

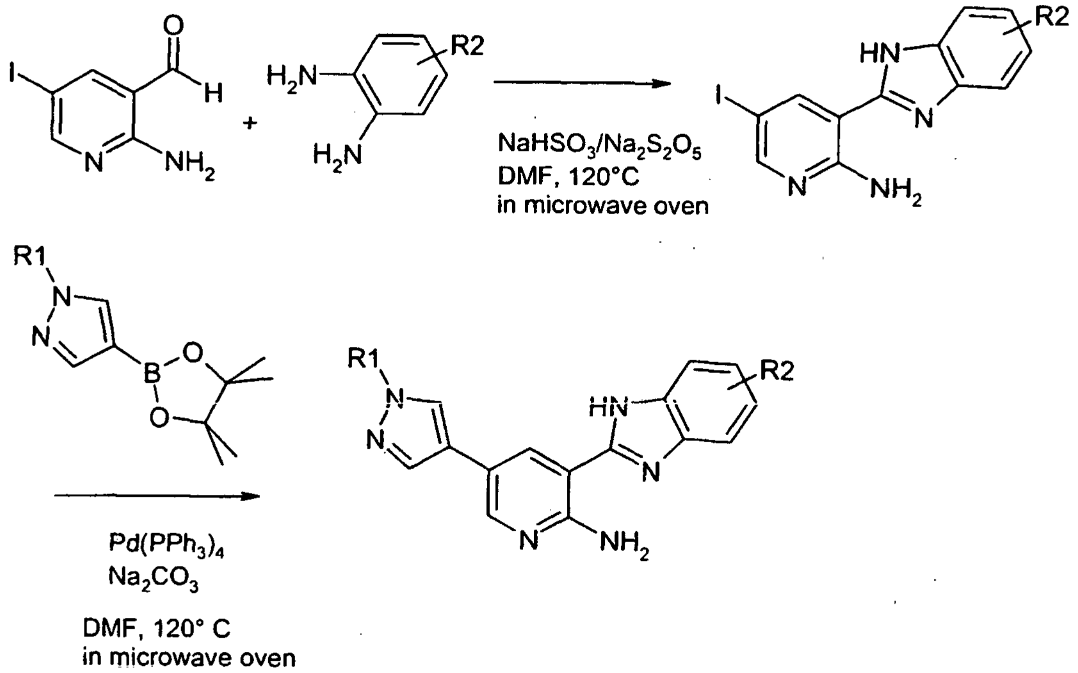


Weg 2:

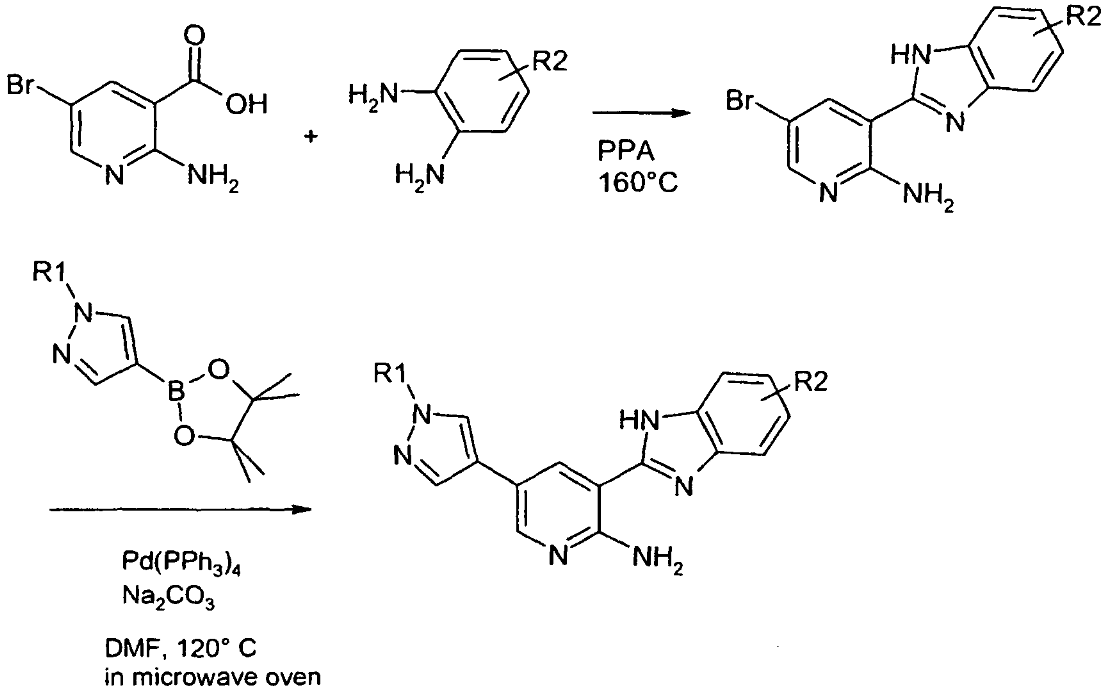

Beispiel 1

Herstellung von 6-{2-[2-Amino-5-(1-methyl-1H-pyrazol-4-yl)-pyridin-3-yl]-3H-benzimidazol-5-yl}-5-methyl-4,5-dihydro-2H-pyridazin-3-on ("A1")

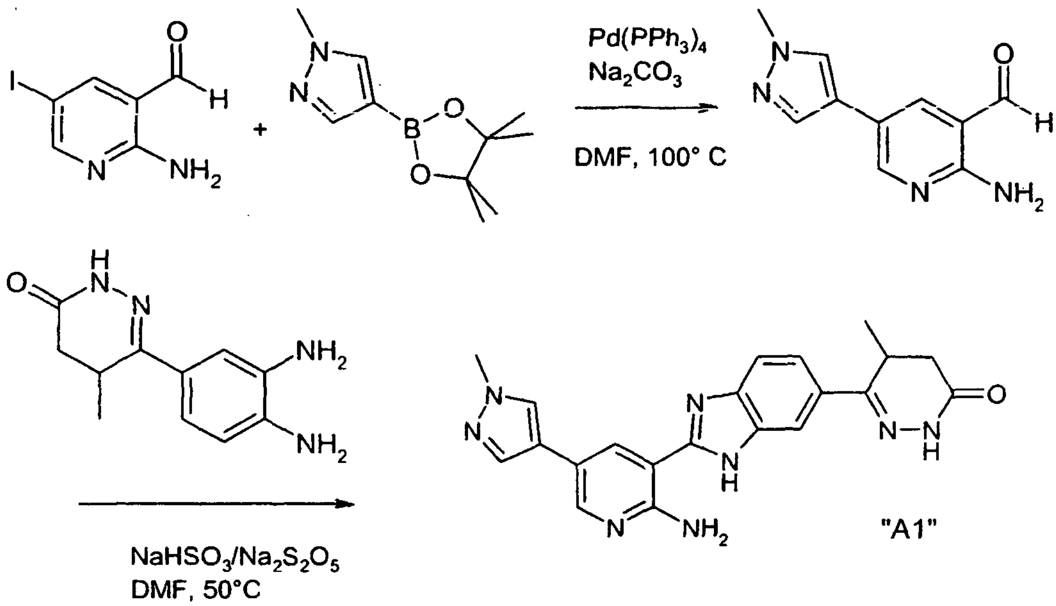


"A1"

1.1 Eine unter Stickstoff gehaltene Lösung von 2 g (8.064 mmol) 2-Amino-5-iod-pyridin-3-carbaldehyd und 3.017g (14.5 mmol) 1-Methyl-4-(4,4,5,5-tetramethyl-[1,3,2]dioxaborolan-2-yl)-1H-pyrazol in 20 ml N,N-Dimethylformamid wird mit 20 ml (40 mmol) 2 M Natriumkarbonat-Lösung und 466 mg (0.403 mmol) Tetrakis(triphenylphosphin)-palladium(0) versetzt, auf 100° C erhitzt und 1 Stunde bei dieser Temperatur gerührt. Das Reaktionsgemisch wird auf Raumtemperatur gekühlt, filtriert und das Filtrat zwischen Wasser und Ethylacetat verteilt. Die organische Phase wird über Natriumsulfat getrocknet, eingedampft und der Rückstand mit Ether verrieben und abgesaugt: 2-Amino-5-(1-methyl-1H-pyrazol-4-yl)-pyridine-3-carbaldehyde als weisser Feststoff; ESI 203.

1.2 Eine Lösung von 100 mg (0.495 mmol) 2-Amino-5-(1-methyl-1H-pyrazol-4-yl)-pyridin-3-carbaldehyd und 108 mg (0.495 mmol) 6-(3,4-Diamino-phenyl)-5-methyl-4,5-dihydro-2H-pyridazin-3-on in 4 ml DMF wird mit 300 μl (1.485 mmol) Natriumhydrogensulfit-Lösung 38-40% versetzt und die entstandene Suspension 72 Stunden bei 50°C gerührt. Das Reaktionsgemisch wird auf RT abgekühlt, mit Wasser versetzt und das ausgefallene Produkt abgesaugt, mit Wasser gewaschen und getrocknet. Man erhält 6-{2-[2-Amino-5-(1-methyl-1H-pyrazol-4-yl)-pyridin-3-yl]-3H-benzimidazol-5-yl}-5-methyl-4,5-dihydro-2H-pyridazin-3-on als gelben Feststoff; APCI 401;

$^1$H NMR (400 MHz, DMSO-$d_6$) δ [ppm] 8.96 (d, *J* = 1.9, 1H), 8.32 (d, *J* = 1.8, 1 H), 8.12 (s, 1 H), 8.01 (s, 1 H), 7.96 - 7.77 (m, 2H), 7.67 (d, *J* = 8.6, 1 H), 3.84 (d, *J* = 28.4, 3H), 3.53 - 3.36 (m, 1 H), 2.70 (dd, *J* = 15.6, 7.9, 1 H), 2.27 (d, *J* = 16.1, 1 H), 1.13 (d, *J* = 7.3, 3H).

Synthese von 6-(3,4-Diamino-phenyl)-5-methyl-4,5-dihydro-2H-pyridazin-3-on ist in Jonas, R.; Klockow, M.; Lues, I.; Prücher, H.; Schliep, H. J.; Wurziger, H. ***Eur. J. Med. Chem.*** 1993, 28, 129, beschrieben.

Die "Pyridazinon" Bausteine werden analog zur Vorschrift in ***Eur. J. Med. Chem.*** 1993,28, 129 hergestellt.

Die Trennung des Racemats (R,S)-6-{2-[2-Amino-5-(1-methyl-1H-pyrazol-4-yl)-pyridin-3-yl]-3H-benzimidazol-5-yl}-5-methyl-4,5-dihydro-2H-pyridazin-3-on in die beiden Enantiomeren (R)-6-{2-[2-Amino-5-(1-methyl-1H-pyrazol-4-yl)-pyridin-3-yl]-3H-benzimidazol-5-yl}-5-methyl-4,5-dihydro-2H-pyridazin-3-on ("A2") und (S)-6-{2-[2-Amino-5-(1-methyl-1H-pyrazol-4-yl)-pyridin-3-yl]-3H-benzimidazol-5-yl}-5-methyl-4,5-dihydro-2H-pyridazin-3-on ("A3") erfolgt mittels präparativer chiraler HPLC und einem Polarimeter als Detektor.

Präparative HPLC: bei Raumtemperatur

Gerät: VWR LaPrep
Mobile Phase. MeOH/EtOH 1/1
Fluß. 100ml/min

Säule: ChiralPAK ® AD 2x(25x5cm)
Wellenlänge 270nM
Racemat: 100mg Racemat/Injection (injection solvent. EtOH/MeOH/DEA 5:3:2)
Enantiomer 1 ("A2"): $t_R$=23.4min (-) MeOH/EtOH 1:1
Enantiomer 2 ("A3"): $t_R$=33.8min (+) MeOH/EtOH 1:1

Analytische HPLC: bei Raumtemperatur

Gerät: HPLC LaChrom 7000
Mobile Phase. MeOH/EtOH 1/1
Fluß. 0.8ml/min
Säule: ChiralPAK ® AD-H (25x0.46cm)
Wellenlänge 270nM
Enantiomer 1 ("A2"): $t_R$=14.6min
Enantiomer 2 ("A3"): $t_R$=20.5min

Analog Beispiel 1 erhält man die nachstehenden Verbindungen

| Nr. | Name und/oder Struktur | Analytik |
|---|---|---|
| "A4" | 6-{2-[2-Amino-5-(1-methyl-1H-pyrazol-4-yl)-pyridin-3-yl]-1H-benzimidazol-5-yl}-5-ethyl-4,5-dihydro-2H-pyridazin-3-on, Trifluoracetat | APCI-MS $[M+H]^+$ 415.3 |
| $^1$H NMR (500 MHz, DMSO-$d_6$ + TFA-$d_1$) δ [ppm] 9.07 (d, *J* = 2.0, 1H), 8.43 (d, *J* = 2.0, 1H), 8.23 (s, 1H), 8.10 (d, *J* = 0.9, 1H), 8.00 (s, 1H), 7.91 (dd, *J* = 8.7, 1.5, 1H), 7.78 (d, *J* = 8.6, 1H), 3.95 (s, 3H), 3.41 (d, J = 6.8, 1H), 2.73 (dd, *J* = 16.9, 7.0, 1H), 2.47 (d, *J* = 16.1, 1H), 1.56 (ddd, *J* = 22.8, 15.1, 7.2, 2H), 0.94 (t, *J* = 7.4, 3H) | | |
| "A5" | 6-{2-[2-Amino-5-(1-methyl-1H-pyrazol-4-yl)-pyridin-3-yl]-3H-benzimidazol-5-yl}-5,5-dimethyl-4,5-dihydro-2H-pyridazin-3-on | ESI-MS $[M+H]^+$ 415.3 |
| | | |
| "A6" | 6-{2-[2-Amino-5-(1-methyl-1H-pyrazol-4-yl)-pyridin-3-yl]-3H-benzimidazol-5-yl}-2,5-dimethyl-4,5-dihydro-2H-pyridazin-3-on | EI-MS $[M]^+$ 414.3 |
| $^1$H NMR (400 MHz, DMSO-$d_6$ + TFA-$d_1$) δ [ppm] 9.06 (d, J = 1.8, 1H), 8.44 (d, J = 1.8, 1H), 8.23 (s, 1H), 8.13 (s, 1H), 8.02 - 7.98 (m, 1H), 7.93 (d, J = 7.7, 1H), 7.77 (s, 1H), 3.95 (s, 3H), 3.62 - 3.51 (m, 1H), 3.40 (s, 3H), 2.80 (dd, J = 16.5, 6.6, 1H), 2.40 (d, J = 16.4, 1H), 1.17 (d, J = 7.2, 3H) | | |
| "A7" | 6-{2-[2-Amino-5-(1-methyl-1H-pyrazol-4-yl)-pyridin-3-yl]-3H-benzimidazol-5-yl}-4,5-dihydro-2H-pyridazin-3-on | EI-MS $[M]^+$ 386.2 |
| $^1$H NMR (500 MHz, DMSO-$d_6$ + TFA-$d_1$) δ [ppm] 9.00 (d, *J* = 2.0, 1H), 8.35 (d, *J* = 2.0, 1H), 8.14 (s, 1H), 7.99 (s, 1H), 7.94 (s, 1H), 7.81 (dd, *J* = 8.6, 1.5, 1H), 7.69 (d, *J* = 8.6, 1H), 3.89 (s, 3H), 3.03 (t, *J* = 8.2, 2H), 2.46 (d, *J* = 8.2, 2H) | | |
| "A8" | 6-{2-[2-Amino-5-(1-methyl-1H-pyrazol-4-yl)-pyridin-3-yl]-3H-benzimidazol-5-yl}-2H-pyridazin-3-on | ESI-MS $[M+H]^+$ 485.3 |
| $^1$H NMR (500 MHz, DMSO-$d_6$ + TFA-$d_1$) δ [ppm] 9.01 (s, 1H), 8.39 (s, 1H), 8.18 (d, *J* = 27.8, 2H), 8.11 (d, *J* = 9.9, 1H), 7.98 (s, 1H), 7.82 (dd, *J* = 40.7, 8.5, 2H), 7.03 (d, *J* = 9.8, 1H), 3.94 (s, 3H) | | |

Beispiel 2

Herstellung von 6-(2-{2-Amino-5-[1-(2-morpholin-4-yl-ethyl)-1 H-pyrazol-4-yl]-pyridin-3-yl}-1H-benzimidazol-5-yl)-5-methyl-4,5-dihydro-2H-pyridazin-3-on ("A9") nach Weg 1 und Route B

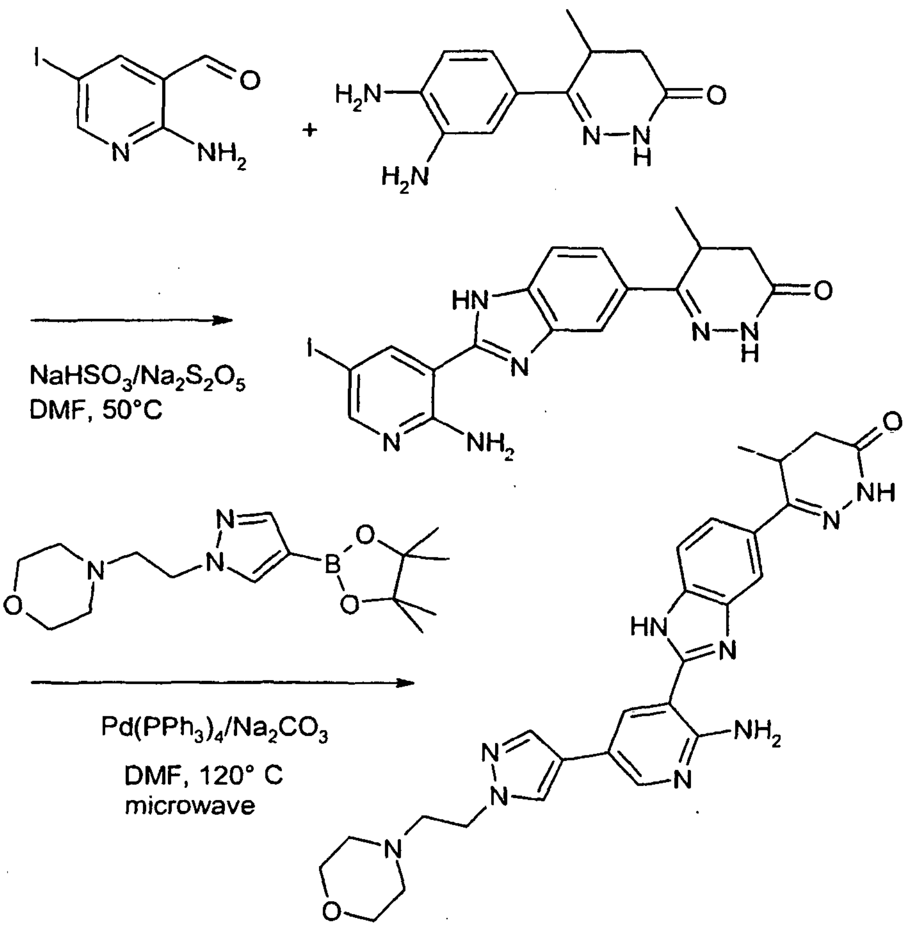


2.1 Eine Lösung von 2.8g (11.289 mol) 2-Amino-5-iod-pyridin-3-carbaldehyd und 2.464 g (11.290 mmol) 6-(3,4-Diamino-phenyl)-5-methyl-4,5-dihydro-2H-pyridazin-3-on in 30 ml DMF wird mit 6.75 ml (33.9 mmol) Natriumhydrogensulfit-Lösung (38-40%) versetzt und 14 h bei 50°C gerührt. Das Reaktionsgemisch wird auf Raumtemperatur gekühlt und mit Wasser versetzt. Der gelbe Niederschlag wird abfiltriert, mit Wasser und Diethylether gewaschen und anschließend getrocknet: 2-Amino-5-(1-methyl-1 H-pyrazol-4-yl)-pyridin-3-carbaldehyd als gelber Feststoff; HPLC-MS $[M+H]^+$ 447.

2.2 In einem mit Stickstoff befüllten Mikrowellengefäss wird 100 mg (0.224 mmol) 2-Amino-5-(1-methyl-1 H-pyrazol-4-yl)-pyridin-3-carbaldehyd und 135 mg (0.440 mmol) 4-{2-[4-(4,4,5,5-Tetramethyl-[1,3,2]dioxaborolan-2-yl)-pyrazol-1-yl]-ethyl}-morpholin in 1.2 ml N,N-Dimethylformamid gelöst und mit 0.666 ml (1.32 mmol) 2M Natriumcarbonat-Lösung und 23 mg (0.02 mmol) Tetrakis(triphenylphosphin)-palladium(0) versetzt. Die Reaktionslösung wird 30 min bei 120°C mit Mikrowellen in Biotage SmithSynthesizer bestrahlt. Das Reaktionsgemisch wird auf Raumtemperatur gekühlt, und mit Essigester/Wasser versetzt. Die wässrige Phase noch einmal mit Essigester ausgeschüttelt. Die vereinigten organischen Phasen werden 3 x mit Wasser gewaschen, mit Natriumsulfat getrocknet, und zum Rückstand eingedampft. Der Rückstand wird mittels RP-HPLC aufgereinigt: 6-(2-{2-Amino-5-[1-(2-morpholin-4-yl-ethyl)-1H-pyrazol-4-yl]-pyrdin-3-yl}-1H-benzimidazol-5-yl)-5-methyl-4,5-dihydro-2H-pyridazin-3-on Trifluoracetat als gelbliche Kristalle; APCI-MS $[M+H]^+$ 500;

$^1$H NMR (500 MHz, DMSO-$d_6$ + TFA-$d_1$) $\delta$ [ppm] 9.09 (d, $J$ = 2.0, 1 H), 8.44 (d, $J$ = 2.0, 1 H), 8.36 (s, 1 H), 8.12 (s, 1 H), 8.08 (s, 1 H), 7.88 (dd, $J$ = 8.7, 1.6, 1 H), 7.75 (d, $J$ = 8.6, 1 H), 4.66 (t, $J$ = 6.4, 2H), 3.97 (s, 2H), 3.71 (dd, $J$ = 15.0, 8.5, 4H), 3.51 (dd, $J$ = 14.6, 7.4, 3H), 3.19 (s, 2H), 2.74 (dd, $J$ = 16.8, 6.9, 1 H), 2.29 (d, $J$ = 15.5, 1 H), 1.15 (d, $J$ = 7.3, 3H).

Analog Beispiel 2 werden folgende Verbindungen hergestellt; sofern nicht anders erwähnt sind die Boronsäure bzw. Boronsäureester kommerziell erhältlich oder in der Literatur beschrieben:

| Nr. | Name und/oder Struktur | Analytik |
|---|---|---|
| "A10" | 6-(2-{2-Amino-5-[1-(3-fluor-benzyl)-1H-pyrazol-4-yl]-pyridin-3-yl}-1H-benzimidazol-5-yl)-5-methyl-4,5-dihydro-2H-pyridazin-3-on Trifluoroacetat | APCI-MS $[M+H]^+$ 495 |

(fortgesetzt)

| Nr. | Name und/oder Struktur | Analytik |
|---|---|---|
| | 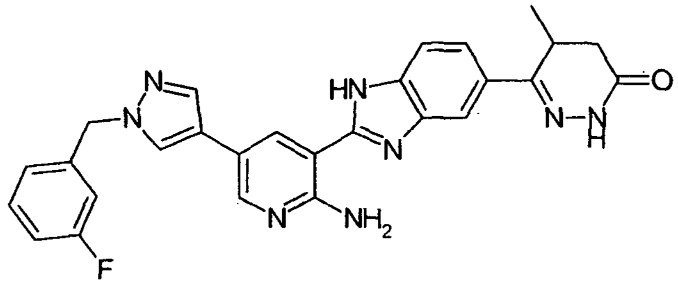 | |
| $^1$H NMR (500 MHz, DMSO-$d_6$ + TFA-$d_1$) δ [ppm] 9.08 (d, J = 2.0, 1H), 8.47 (d, J = 2.0, 1H), 8.37 (s, 1H), 8.11 (s, 1H), 8.09 (s, 1H), 7.91 (dd, J = 8.7, 1.5, 1H), 7.78 (d, J = 8.6, 1H), 7.47 - 7.40 (m, 1H), 7.19 - 7.08 (m, 3H), 5.49 (s, 2H), 3.60 - 3.51 (m, 1H), 2.77 (dd, J = 16.7, 6.8, 1H), 2.32 (d, J = 15.6, 1H), 1.18 (d, J = 7.3, 3H) | | |
| "A11" | 6-{2-[2-Amino-5-(1-isopropyl-1H-pyrazol-4-yl)-pyridin-3-yl]-1 H-benzimidazol-5-yl}-5-methyl-4,5-dihydro-2H-pyridazin-3-on, Trifluoracetat | APCI-MS [M+H]$^+$ 429 |
| $^1$H NMR (500 MHz, DMSO-$d_6$ + TFA-$d_1$) δ [ppm] 9.05 (d, J = 2.0, 1H), 8.42 (d, J = 2.0, 1H), 8.30 (s, 1H), 8.08 (d, J = 1.1, 1H), 7.99 (s, 1H), 7.88 (dd, J = 8.6, 1.6, 1H), 7.76 (d, J = 8.6, 1H), 4.56 (hept, J = 6.7, 1H), 3.59 - 3.45 (m, 1H), 2.75 (dd, J = 16.7, 6.9, 1H), 2.29 (d, J = 15.5, 1H), 1.48 (d, J = 6.7, 6H), 1.15 (d, J = 7.3, 3H) | | |
| "A 12" | 6-(2-{2-Amino-5-[1-(2-methoxy-ethyl)-1H-pyrazol-4-yl]-pyridin-3-yl}-1H-benzimidazol-5-yl)-5-methyl-4,5-dihydro-2H-pyridazin-3-on, Trifluoracetat | APCI-MS [M+H]$^+$ 445 |
| | 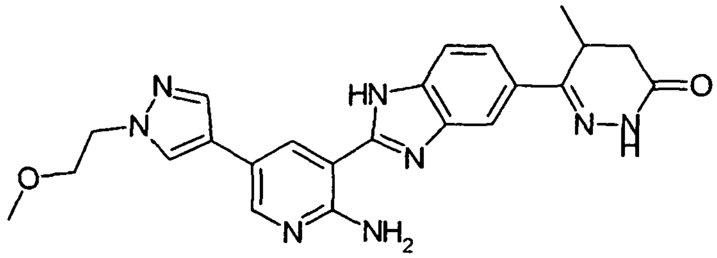 | |
| $^1$H NMR (500 MHz, DMSO-$d_6$ + TFA-$d_1$) δ [ppm] 9.07 (d, J = 2.0, 1H), 8.46 (d, J = 2.0, 1H), 8.25 (s, 1H), 8.10 (s, 1H), 8.04 (s, 1H), 7.90 (dd, J = 8.7, 1.6, 1H), 7.78 (d, J = 8.6, 1H), 4.36 (t, J = 5.1, 2H), 3.76 (t, J = 5.2, 2H), 3.63 - 3.49 (m, 1H), 3.28 (s, 3H), 2.77 (dd, J = 16.7, 6.8, 1H), 2.31 (d, J = 15.6, 1H), 1.17 (d, J = 7.3, 3H) | | |
| "A13" | 6-(2-{2-Amino-5-[1-(3-methoxy-propyl)-1H-pyrazol-4-yl]-pyridin-3-yl}-1H-benzimidazol-5-yl)-5-methyl-4,5-dihydro-2H-pyridazin-3-on, Trifluoroacetat | APCI-MS [M+H]$^+$ 459 |
| $^1$H NMR (400 MHz, DMSO-$d_6$ + TFA-$d_1$) δ [ppm] 9.08 (d, J = 2.0, 1H), 8.46 (d, J = 2.1, 1H), 8.27 (s, 1H), 8.11 (s, 1H), 8.04 (s, 1H), 7.92 (dd, J = 8.7, 1.6, 1H), 7.79 (d, J = 8.7, 1H), 4.26 (t, J = 7.0, 2H), 3.62 - 3.51 (m, 1H), 3.36 (t, J = 6.0, 2H), 3.28 (s, 3H), 2.78 (dd, J = 16.7, 6.8, 1H), 2.33 (d, J = 15.6, 1H), 2.10 (p, J = 6.5, 2H), 1.19 (d, J = 7.3, 3H) | | |
| "A14" | 6-(2-{2-Amino-5-[1-(2-pyrrolidin-1-yl-ethyl)-1H-pyrazol-4-yl]-pyridin-3-yl}-1H-benzimidazol-5-yl)-5-methyl-4,5-dihydro-2H-pyridazin-3-on, Trifluoracetat 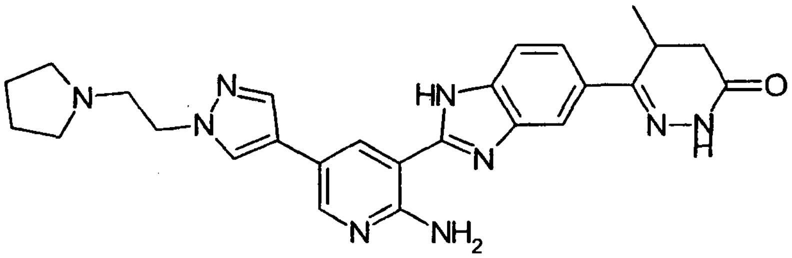 | APCI-MS [M+H]$^+$ 484 |
| $^1$H NMR (400 MHz, DMSO-$d_6$ + TFA-$d_1$) δ [ppm] 9.08 (d, J = 2.0, 1H), 8.44 (d, J = 2.0, 1H), 8.35 (s, 1H), 8.12 (s, 1H), 8.08 (s, 1H), 7.88 (dd, J = 8.7, 1.6, 1H), 7.75 (d, J = 8.7, 1H), 4.59 (t, J = 6.1, 2H), 3.73 (t, J = 6.1, 2H), 3.61 - 3.47 (m, 3H), 3.11 - 2.99 (m, 2H), 2.74 (dd, J = 16.8, 6.8, 1H), 2.29 (d, J = 15.7, 1H), 2.08 - 1.93 (m, 2H), 1.93 - 1.79 (m, 2H), 1.15 (d, J = 7.3, 3H) | | |

(fortgesetzt)

| Nr. | Name und/oder Struktur | Analytik |
|---|---|---|
| "A15" | 6-(2-{2-Amino-5-[1-(2-dimethylamino-ethyl)-1H-pyrazol-4-yl]-pyridin-3-yl}-1H-benzimidazol-5-yl)-5-methyl-4,5-dihydro-2H-pyridazin-3-on, Trifluoracetat | APCI-MS $[M+H]^+$ 458 |
| $^1H$ NMR (400 MHz, DMSO-$d_6$ + TFA-d) δ [ppm] 9.12 (d, *J* = 2.0, 1H), 8.49 (d, *J* = 2.0, 1H), 8.40 (s, 1H), 8.16 (s, 1H), 8.12 (s, 1H), 7.92 (dd, *J* = 8.7, 1.5, 1H), 7.80 (d, *J* = 8.6, 1H), 4.66 (t, *J* = 6.2, 2H), 3.69 (t, *J* = 6.2, 2H), 3.61 - 3.51 (m, 1H), 2.90 (s, 6H), 2.78 (dd, *J* = 16.7, 6.8, 1H), 2.33 (d, *J* = 15.7, 1H), 1.19 (d, *J* = 7.3, 3H) | | |
| "A16" | 6-(2-{2-Amino-5-[1-(1-methyl-piperidin-4-yl)-1H-pyrazol-4-yl]-pyridin-3-yl}-1H-benzimidazol-5-yl)-5-methyl-4,5-dihydro-2H-pyridazin-3-on, Trifluoracetat 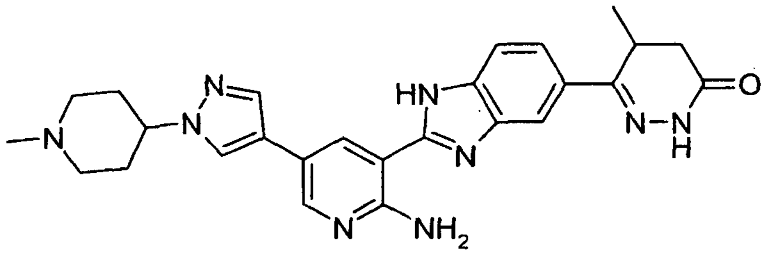 | APCI-MS $[M+H]^+$ 484 |
| $^1H$ NMR (500 MHz, DMSO-$d_6$ + TFA-$d_1$) δ [ppm] 9.10 (d, *J* = 2.0, 1H), 8.48 (d, *J* = 2.0, 1H), 8.37 (s, 1H), 8.12 (s, 1 H), 8.10 (s, 1H), 7.92 (dd, *J* = 8.7, 1.5, 1H), 7.79 (d, *J* = 8.6, 1H), 4.57 (tt, *J* = 11.7, 3.9, 1H), 3.65 (d, *J* = 12.6, 2H), 3.59 - 3.52 (m, 1H), 3.26 (t, *J* = 11.9, 2H), 2.89 (s, 3H), 2.78 (dd, *J* = 16.8, 6.9, 1H), 2.39 - 2.18 (m, 5H), 1.19 (d, *J* = 7.3, 3H) | | |
| "A17" | Nach der Suzuki-Kopplung des Boc-geschützen Piperidin-Pyrazolboronsäuresters, wird die Boc-Schutzgruppe nach Standardbedingungen in Dioxan/HCl abgespalten. Man erhält 6-{2-[2-Amino-5-(1-piperidin-4-yl-1H-pyrazol-4-yl)-pyridin-3-yl]-1H-benzimidazol-5-yl}-5-methyl-4,5-dihydro-2H-pyridazin-3-on, Trifluoracetat | APCI-MS $[M+H]^+$ 470 |
| $^1H$ NMR (500 MHz, DMSO-$d_6$ + TFA-$d_1$) δ [ppm] 9.11 (d, *J* = 2.0, 1H), 8.48 (d, *J* = 2.0, 1H), 8.37 (s, 1H), 8.12 (s, 1H), 8.10 (s, 1H), 7.92 (dd, *J* = 8.7, 1.5, 1H), 7.79 (d, *J* = 8.6, 1H), 4.66 - 4.57 (m, 1H), 3.60 - 3.52 (m, 1H), 3.52 - 3.45 (m, 2H), 3.19 (td, *J* = 12.7, 3.0, 2H), 2.78 (dd, *J* = 16.7, 6.8, 1H), 2.38 - 2.27 (m, 3H), 2.27 - 2.17 (m, 2H), 1.19 (d, *J* = 7.3, 3H) | | |
| "A18" | 6-(2-{2-Amino-5-[1-(tetrahydro-furan-2-ylmethyl)-1H-pyrazol-4-yl]-pyridin-3-yl}-1H-benzimidazol-5-yl)-5-methyl-4,5-dihydro-2H-pyridazin-3-on, Trifluoracetat 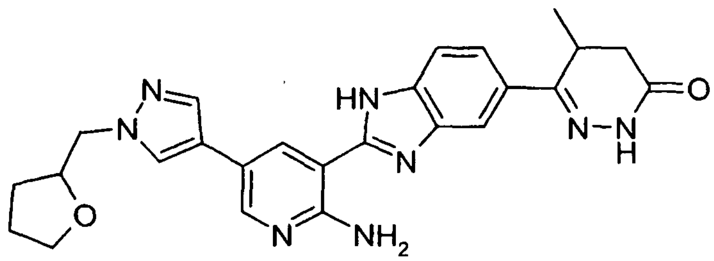 | APCI-MS $[M+H]^+$ 471 |
| | | |

(fortgesetzt)

| Nr. | Name und/oder Struktur | Analytik |
|---|---|---|
| "A19" | Nach Abspaltung der Schutzgruppe aus der BOCgeschützten Verbindung erhält man 6-[2-(6-Amino-6'-piperazin-1-yl-[3,3']bipyridinyl-5-yl)-1H-benzimidazol-5-yl]-5-methyl-4,5-dihydro-2H-pyridazin-3-on, Trifluoracetat 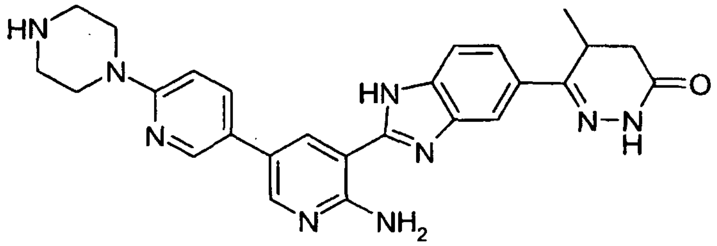 | ESI-MS $[M+H]^+$ 482.3 |
| $^1H$ NMR (500 MHz, DMSO-$d_6$ + TFA-$d_1$) δ [ppm] 9.12 (d, *J* = 2.1, 1H), 8.57 (t, *J* = 2.1, 2H), 8.30 (dd, *J* = 9.3, 2.4, 1H), 8.08 (s, 1H), 7.88 (dd, *J* = 8.7, 1.5, 1H), 7.75 (d, *J* = 8.7, 1H), 7.40 (d, *J* = 9.3, 1H), 3.95 - 3.87 (m, 4H), 3.52 (p, *J* = 7.7, 1H), 3.34 - 3.27 (m, 4H), 2.73 (dd, *J* = 16.8, 6.8, 1H), 2.29 (d, *J* = 15.8, 1H), 1.15 (d, *J* = 7.3, 3H) | | |
| "A20" | 6-{2-[2-Amino-5-(2-dimethylamino-thiazol-4-yl)-pyridin-3-yl]-1H-benzimidazol-5-yl}-5-methyl-4,5-dihydro-2H-pyridazin-3-on | HPLC-MS $[M+H]^+$ 447.2 |
| | 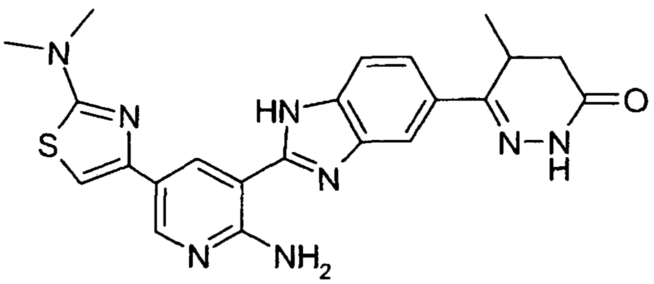 | |
| | | |
| "A21" | 6-[2-(6-Amino-6'-methoxy-[3,3']bipyridinyl-5-yl)-1H-benzimidazol-5-yl]-5-methyl-4,5-dihydro-2H-pyridazin-3-on, Trifluoracetat 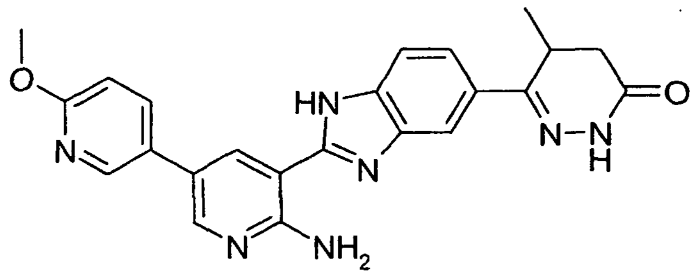 | ESI-MS $[M+H]^+$ 428.3 |
| $^1H$ NMR (500 MHz, DMSO-$d_6$ + TFA-$d_1$) δ [ppm] 9.14 (d, *J* = 2.1, 1H), 8.64 (d, *J* = 2.5, 1H), 8.53 (d, *J* = 2.1, 1H), 8.15 (dd, *J* = 8.7, 2.6, 1H), 8.07 (d, *J* = 1.0, 1H), 7.88 (dd, *J* = 8.7, 1.6, 1H), 7.74 (d, *J* = 8.6, 1H), 7.03 (d, *J* = 8.6, 1H), 3.93 (s, 3H), 3.52 (p, *J* = 7.1, 1H), 2.73 (dd, *J* = 16.8, 6.9, 1H), 2.29 (d, *J* = 15.6, 1H), 1.15 (d, *J* = 7.3, 3H) | | |

(fortgesetzt)

| Nr. | Name und/oder Struktur | Analytik |
|---|---|---|
| "A22" | 6-[2-(6,6'-Diamino-[3,3']bipyridinyl-5-yl)-1H-benzimidazol-5-yl]-5-methyl-4,5-dihydro-2H-pyridazin-3-on, Trifluoracetat 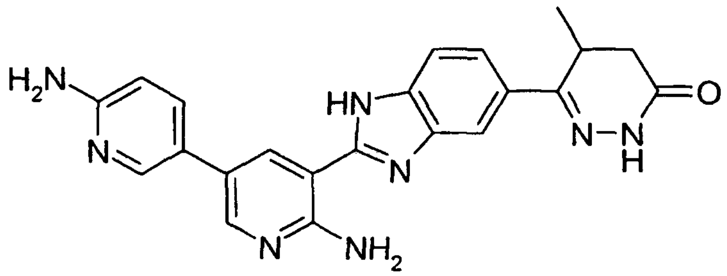 | ESI-MS $[M+H]^+$ 413.3 |
| $^1H$ NMR (500 MHz, DMSO-$d_6$ + TFA-$d_1$) $\delta$ [ppm] 9.14 (d, $J$ = 2.1, 1H), 8.59 (d, J = 2.1, 1H), 8.44 (d, J = 2.2, 1H), 8.40 (dd, J = 9.3, 2.3, 1H), 8.13 (s, 1H), 7.94 (dd, $J$ = 8.7, 1.5, 1H), 7.80 (d, $J$ = 8.7, 1H), 7.24 (d, $J$ = 9.3, 1H), 3.61 - 3.51 (m, 1H), 2.79 (dd, $J$ = 16.8, 6.9, 1H), 2.35 (d, $J$ = 15.6, 1H), 1.21 (d, $J$ = 7.3, 3H) | | |

<u>Beispiel 2a</u>

Herstellung von 6-{2-[2-Amino-5-(4-fluor-2H-pyrazol-3-yl)-pyridin-3-yl]-1H-benzimidazol-5-yl}-5-methyl-4,5-dihydro-2H-pyridazin-3-on ("A23")

Alternativ zu den Standardbedingungen der Suzuki-Kopplung in Weg 1 Route A, kann man die Synthesesequenz auch unter Bedingungen einer Stille-Kopplung durchführen.

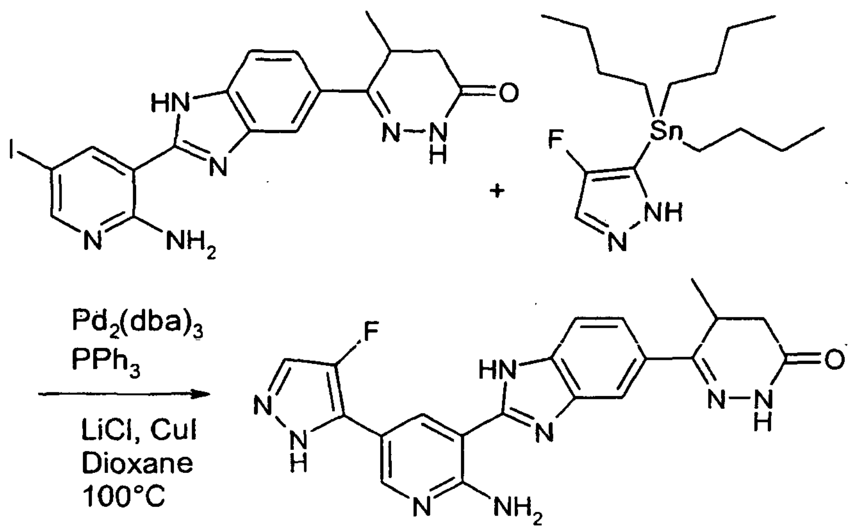


In einem mit Stickstoff befüllten Reaktionsgefäss werden 100 mg (0.224 mmol) 6-[2-(2-Amino-5-iod-pyridin-3-yl)-1 H-benzimidazol-5-yl]-5-methyl-4,5-dihydro-2H-pyridazin-3-on, 252.2 mg (0.672 mmol) 4-Fluor-5-tributylstannanyl-1H-pyrazol, 41.5 mg (0.045 mmol) Tris(dibenzyliden-aceton)dipalladium(0), 23.5 mg (0.090 mmol) Triphenylphosphin, 28.5 mg (0.672 mmol) Lithiumchlorid und 8.5 mg (0.045 mmol) Kupferiodid in 2 ml Dioxan gegeben. Das Gemisch wird 14 h bei 100°C unter Stickstoffschutzatmosphäre gerührt. Das Reaktionsgemisch wird auf Raumtemperatur gekühlt und das Lösungsmittel entfernt. Der Rückstand wird mit DCM und HCl 2N versetzt. Der Niederschlag wird abgetrennt und mit DCM/Wasser gewaschen. Der Niederschlag wird weiter über präparative RP-HPLC aufgereinigt und man erhält 6-{2-[2-Amino-5-(4-fluor-2H-pyrazol-3-yl)-pyridin-3-yl]-1H-benzimidazol-5-yl}-5-methyl-4,5-dihydro-2H-pyridazin-3-on; HPLC-MS $[M+H]^+$ 405.2;
$^1H$ NMR (500 MHz, DMSO-$d_6$ + TFA-$d_1$) $\delta$ [ppm] 9.14 (d, $J$ = 1.9, 1 H), 8.42 (d, $J$ = 1.9, 1 H), 8.06 (d, $J$ = 4.4, 2H), 7.88 (dd, $J$ = 8.7, 1.6, 1 H), 7.74 (d, $J$ = 8.6, 1 H), 3.54 (p, $J$ = 7.6, 1H), 2.75 (dd, $J$ = 16.7, 6.8, 1 H), 2.30 (d, $J$ = 15.6, 1 H), 1.15 (d, $J$ = 7.3, 3H).

Beispiel 3

Herstellung von (4-{6-Amino-5-[5-(4-methyl-6-oxo-1,4,5,6-tetrahydropyridazin-3-yl)-1H-benzimidazol-2-yl]-pyridin-3-yl}-pyrazol-1-yl)-essigsäure ("A24")

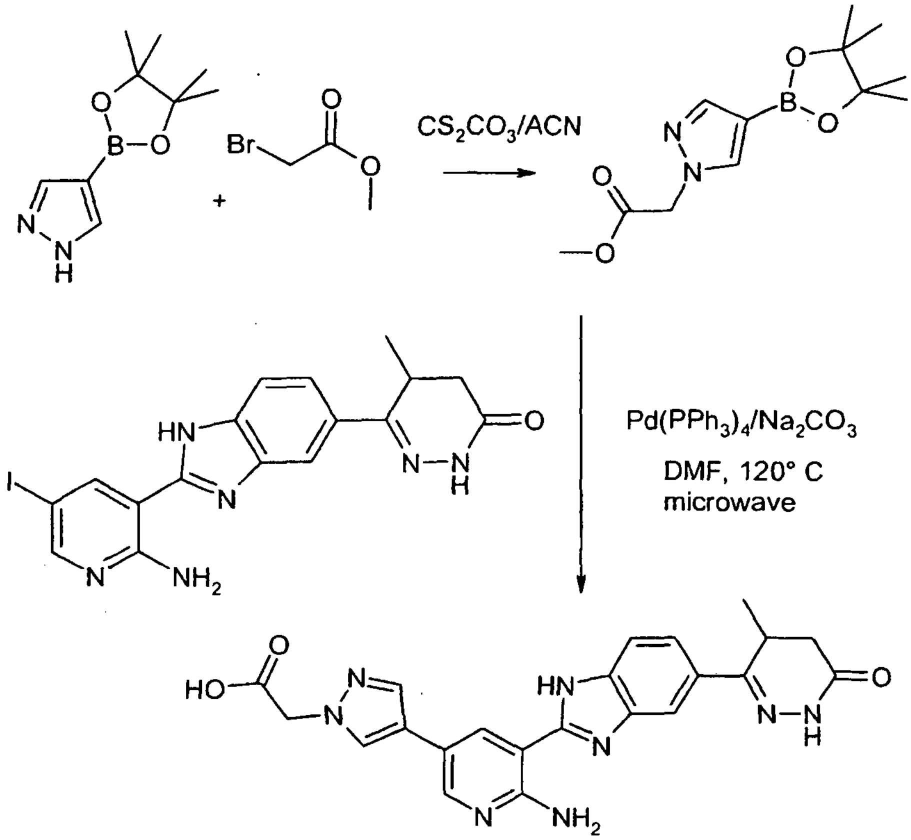


3.1 1 g (5.051 mmol) 4-(4,4,5,5-Tetramethyl-[1,3,2]dioxaborolan-2-yl)-1H-pyrazol und 1.808 g (5.550 mmol) Cäsiumcarbonat werden in 20 ml ACN suspendiert. 468 μl (5.050 mmol) Bromessigsäure-methylester werden zugegeben und 72 Stunden bei RT gerührt. Der Niederschlag wird abgesaugt und mit ACN nachgewaschen. Die Mutterlauge wird zum Rückstand abdestilliert, mit Essigester versetzt und zügig 2x mit Wasser gewaschen. Die organische Phase wird sofort mit Natriumsulfat getrocknet und zum Rückstand abdestilliert: [4-(4,4,5,5-Tetramethyl-[1,3,2]dioxaborolan-2-yl)-pyrazol-1-yl]-essigsäure-methylester als Feststoff; EI-MS $[M]^+$ 266.

3.2 In einem mit Stickstoff befüllten Mikrowellengefäss wird 100 mg (0.224 mmol) 2-Amino-5-(1-methyl-1H-pyrazol-4-yl)-pyridin-3-carbaldehyd und 117 mg (0.440 mmol) 4-(4,4,5,5-Tetramethyl-[1,3,2]dioxaborolan-2-yl)-pyrazol-1-yl]-essigsäure-methylester in 1.2 ml N,N-Dimethylformamid gelöst und mit 0.666 ml (1.32 mmol) 2M Natriumcarbonat-Lösung und 23 mg (0.02 mmol) Tetrakis(triphenylphosphin)-palladium(0) versetzt. Die Reaktionslösung wird 30 min bei 120°C mit Mikrowellen im Biotage SmithSynthesizer bestrahlt. Das Reaktionsgemisch wird auf Raumtemperatur gekühlt und mit Essigester/Wasser versetzt. Die wässrige Phase noch einmal mit Essigester ausgeschüttelt. Die wässrige Phase wird mit HCl 1 N sauer gestellt und 2x mit Dichlormethan/wenig Isopropanol ausgeschüttelt. Die organische Phase wird mit Natriumsulfat getrocknet, und zum Rückstand eingedampft. Der Rückstand wird mittels RP-HPLC aufgereinigt; da der Ester während der Umsetzung hydrolysiert wird erhält man: (4-{6-Amino-5-[5-(4-methyl-6-oxo-1,4,5,6-tetrahydro-pyridazin-3-yl)-1H-benzimidazol-2-yl]-pyridin-3-yl}-pyrazol-1-yl)-essigsäure, trifluoracetat als gelbliche Kristalle; HPLC-MS $[M+H]^+$ 445.3;

$^1H$ NMR (400 MHz, DMSO-$d_6$ + TFA-$d_1$) δ [ppm] 9.10 (d, *J* = 1.9, 1H), 8.49 (d, *J* = 1.9, 1 H), 8.29 (s, 1 H), 8.11 (s, 1 H), 8.07 (s, 1 H), 7.92 (dd, *J* = 8.7, 1.3, 1 H), 7.79 (d, *J* = 8.7, 1 H), 5.12 (s, 2H), 3.65 - 3.50 (m, 1 H), 2.78 (dd, *J* = 16.8, 6.9, 1 H), 2.33 (d, *J* = 16.2, 1 H), 1.19 (d, *J* = 7.3, 3H).

Beispiel 4

Herstellung von 2-(4-{6-Amino-5-[5-(4-methyl-6-oxo-1,4,5,6-tetrahydropyridazin-3-yl)-1H-benzimidazol-2-yl]-pyridin-3-yl}-pyrazol-1-yl)-N,N-dimethylacetamid ("A25")

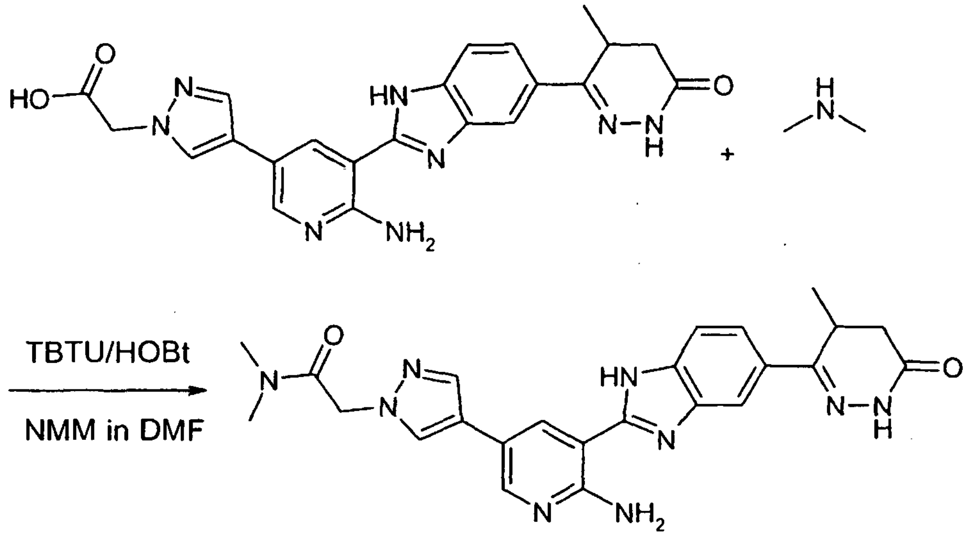


50 mg "A24" (0.112 mmol) werden in 5 ml DMF gelöst. 35 mg (0.110 mmol) TBTU, 4.458mg (0.033mmol) HOBt, 83 μl (0.165 mmol) Dimethylamin 2M in THF und 61 μl (0.550 mmol) N-Methylmorpholin werden zugegeben. Das Reaktionsgemisch wird 14 h bei Raumtemperatur gerührt, mit Wasser versetzt und gefriergetrocknet. Der Rückstand wird mittels RP-HPLC aufgereinigt: 2-(4-{6-Amino-5-[5-(4-methyl-6-oxo-1,4,5,6-tetrahydropyridazin-3-yl)-1H-benzimidazol-2-yl]-pyridin-3-yl}-pyrazol-1-yl)-N,N-dimethyl-acetamid, Trifluoracetat als gelbliche Kristalle; APCI-MS $[M+H]^+$ 472; $^1H$ NMR (500 MHz, DMSO-$d_6$ + TFA-$d_1$) δ [ppm] 9.09 (d, *J* = 2.0, 1 H), 8.47 (d, *J* = 2.1, 1H), 8.21 (s, 1H), 8.11 (s, 1H), 8.03 (s, 1H), 7.91 (dd, *J* = 8.7, 1.5, 1 H), 7.78 (d, *J* = 8.7, 1 H), 5.24 (s, 2H), 3.61 - 3.51 (m, 1 H), 3.10 (s, 3H), 2.91 (s, 3H), 2.78 (dd, *J* = 16.7, 6.9, 1 H), 2.32 (d, *J* = 15.6, 1 H), 1.18 (d, *J* = 7.3, 3H).

Analog erhält man 6-[2-(2-Amino-5-{1-[2-(4-methyl-piperazin-1-yl)-2-oxo-ethyl]-1H-pyrazol-4-yl}-pyridin-3-yl)-1H-benzimidazol-5-yl]-5-methyl-4,5-dihydro-2H-pyridazin-3-on ("A26"), Trifluoracetat als gelbliche Kristalle

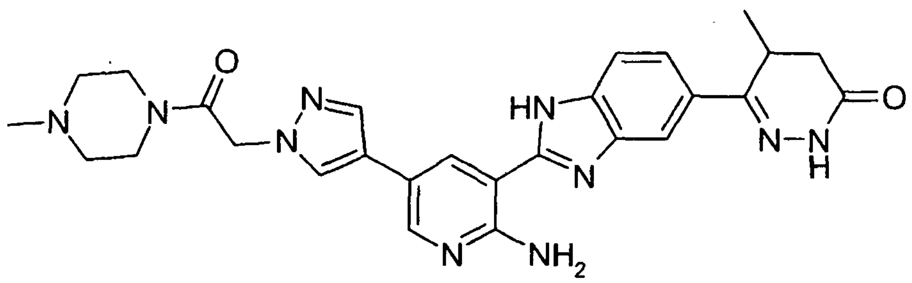

APCI-MS $[M+H]^+$ 527;
$^1H$ NMR (500 MHz, DMSO-$d_6$ + TFA-$d_1$) δ [ppm] 9.11 (d, *J* = 2.0, 1 H), 8.49 (d, *J* = 2.0, 1 H), 8.22 (s, 1 H), 8.11 (s, 1 H), 8.06 (s, 1 H), 7.91 (dd, *J* = 8.6, 1.6, 1 H), 7.79 (d, *J* = 8.6, 1H), 5.36 (q, *J* = 16.1, 2H), 4.35 (dd, *J* = 125.8, 13.9, 2H), 3.61 - 3.46 (m, 4H), 3.23 - 3.00 (m, 3H), 2.90 (s, 3H), 2.78 (dd, *J* = 16.8, 6.9, 1 H), 2.33 (d, *J* = 15.7, 1 H), 1.19 (d, *J* = 7.3, 3H).

Beispiel 4a

Herstellung von 2-{4-[6-Amino-5-(6-methoxy-1H-benzimidazol-2-yl)-pyridin-3-yl]-pyrazol-1-yl}-N-(3,4-difluor-benzyl)-acetamid ("A27")

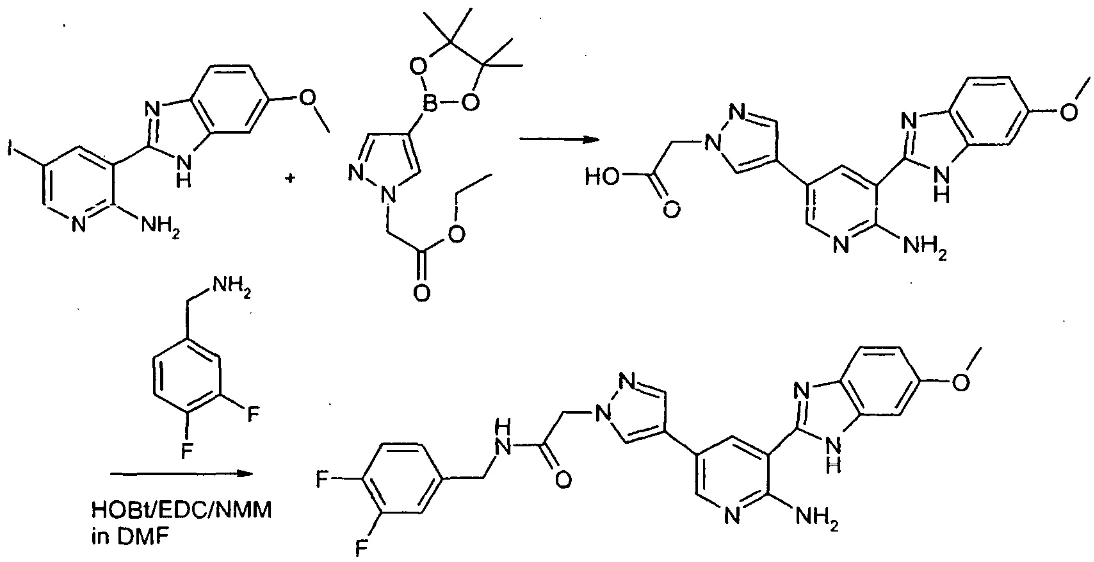


4a.1 {4-[6-Amino-5-(6-methoxy-1H-benzimidazol-2-yl)-pyridin-3-yl]-pyrazol-1-yl}-essigsäure wird nach Route 1 Weg B hergestellt (120°C im Kolben 14 h, statt Mikrowellen), dabei wird der Ethylester hydrolysiert und das Rohprodukt so weiter umgesetzt; HPLC-MS $[M+H]^+$ 365.2.

4a.2 146 mg {4-[6-Amino-5-(6-methoxy-1H-benzimidazol-2-yl)-pyridin-3-yl]-pyrazol-1-yl}-essigsäure (0.120 mmol) werden in 1.5 ml DMF gelöst. 17.5 mg (0.120 mmol) 3,4-Difluor-benzylamin, 16.2 mg (0.120 mmol) HOBt, 23 mg (0120 mmol) EDC und 26.4 μl (0.240 mmol) N-Methylmorpholin werden zugegeben. Das Reaktionsgemisch wird 14 h bei Raumtemperatur gerührt, und anschließend mit Wasser/Essigester versetzt. Die wässrige Phase wird 2x mit Essigester extrahiert. Die vereinigten organischen Phasen werden mit Wasser gewaschen, mit Natriumsulfat getrocknet, filtriert und zum Rückstand eingeengt. Der Rückstand wird mittels präparativer RP-HPLC aufgereinigt. Man erhält 2-{4-[6-Amino-5-(6-methoxy-1H-benzimidazol-2-yl)-pyridin-3-yl]-pyrazol-1-yl}-N-(3,4-difluorbenzyl)-acetamid; HPLC-MS $[M+H]^+$ 490.2;

Beispiel 5

Herstellung von 6-(2-{2-Amino-5-[1-(2-oxo-2-piperidin-1-yl-ethyl)-1H-pyrazol-4-yl]-pyridin-3-yl}-3H-benzimidazol-5-yl)-5-methyl-4,5-dihydro-2H-pyridazin-3-on ("A28")

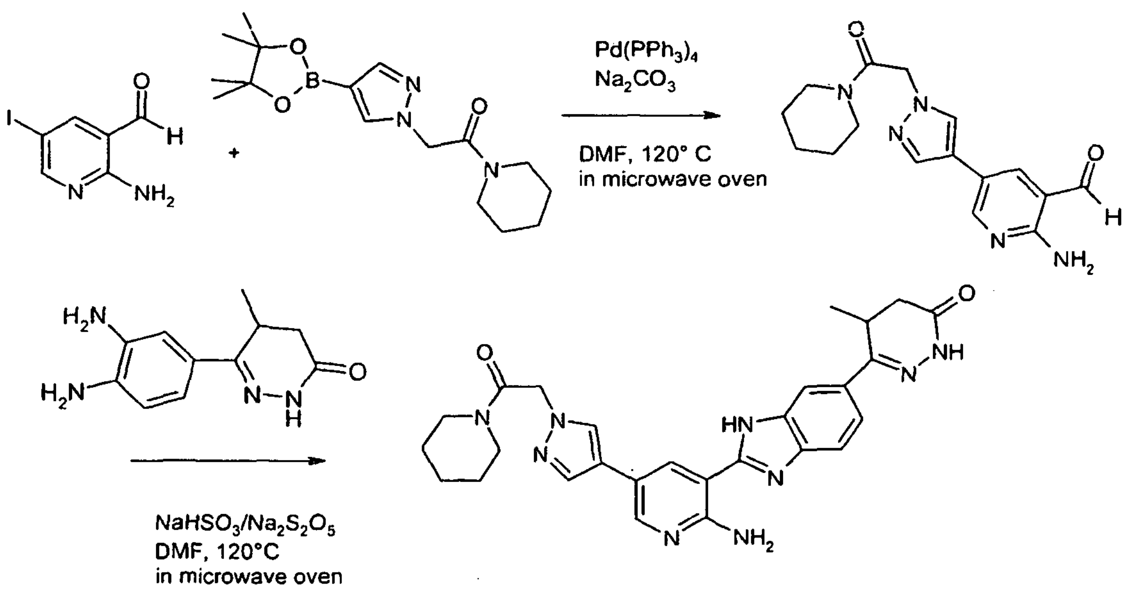

5.1 In einem mit Argon befüllten Mikrowellengefäss werden 200 mg (0.806 mmol) 2-Amino-5-iod-pyridine-3-carbaldehyd und 501 mg (1.570 mmol) 1-Piperidin-1-yl-2-[4-(4,4,5,5-tetramethyl-[1,3,2]dioxaborolan-2-yl)-pyrazol-1-yl]-ethanon in 4 ml N,N-Dimethylformamid gelöst und mit 2.5 ml (5 mmol) 2M Natriumcarbonat-Lösung und 93.2 mg (0.081 mmol) Tetrakis(triphenylphosphin)-palladium(0) versetzt. Die Reaktionslösung wird 30 min bei 120°C mit Mikrowellen im Biotage SmithSynthesizer bestrahlt. Das Reaktionsgemisch wird auf Raumtemperatur gekühlt, mit Wasser versetzt und filtriert. Das Filtrat wird dreimal mit Essigester extrahiert und die kombinierten organischen Phasen 2x mit Wasser gewaschen, mit Natriumsulfat getrocknet, und zum Rückstand eingedampft. Der ölige Rückstand wird mittels RP-HPLC aufgereinigt und man erhält als gelbliches Pulver 2-Amino-5-[1-(2-oxo-2-piperidin-1-yl-ethyl)-1H-pyrazol-4-yl]-pyridin-3-carbaldehyd; ESI-MS $[M+H]^+$ 314.2.

5.2 Eine Lösung von 70 mg (0.223 mmol) 2-Amino-5-[1-(2-oxo-2-piperidin-1-yl-ethyl)-1H-pyrazol-4-yl]-pyridin-3-carbaldehyd und 58.51 mg (0.268 mmol) 6-(3,4-Diamino-phenyl)-5-methyl-4,5-dihydro-2H-pyridazin-3-on in 3 ml DMF wird mit 110.07 μl (0.558 mmol) Natriumhydrogensulfit-Lösung 38-40% versetzt und 30 min bei 120°C mit Mikrowellen im Biotage SmithSynthesizer bestrahlt. Das Reaktionsgemisch wird auf Raumtemperatur gekühlt, und mit Wasser versetzt. Der Niederschlag wird abfiltriert und mit Wasser gewaschen und anschließend getrocknet. Der Rückstand wird mittels RP-HPLC aufgereinigt: 6-(2-{2-Amino-5-[1-(2-oxo-2-piperidin-1-yl-ethyl)-1H-pyrazol-4-yl]-pyridin-3-yl}-3H-benzimidazol-5-yl)-5-methyl-4,5-dihydro-2H-pyridazin-3-on als gelber Feststoff; HPLC-MS $[M+H]^+$ 512.2;
$^1H$ NMR (400 MHz, DMSO-$d_6$ + TFA-$d_1$) δ [ppm] 9.05 (d, *J* = 2.1, 1H), 8.41 (d, *J* = 2.0, 1 H), 8.17 (s, 1 H), 8.08 - 8.03 (m, 1 H), 7.98 (d, *J* = 0.6, 1 H), 7.87 (dd, *J* = 8.7, 1.6, 1 H), 7.72 (d, *J* = 8.7, 1 H), 5.19 (s, 2H), 3.54 - 3.40 (m, 5H), 2.76 - 2.69 (m, 1H), 2.28 *(d, J* = 15.7, 1 H), 1.63 - 1.40 (m, 6H), 1.14 (d, *J* = 7.3, 3H).

Analog erhält man

6-(2-{2-Amino-5-[1-(2-hydroxy-ethyl)-1H-pyrazol-4-yl]-pyridin-3-yl}-3H-benzimidazol-5-yl)-5-methyl-4,5-dihydro-2H-pyridazin-3-on ("A29")

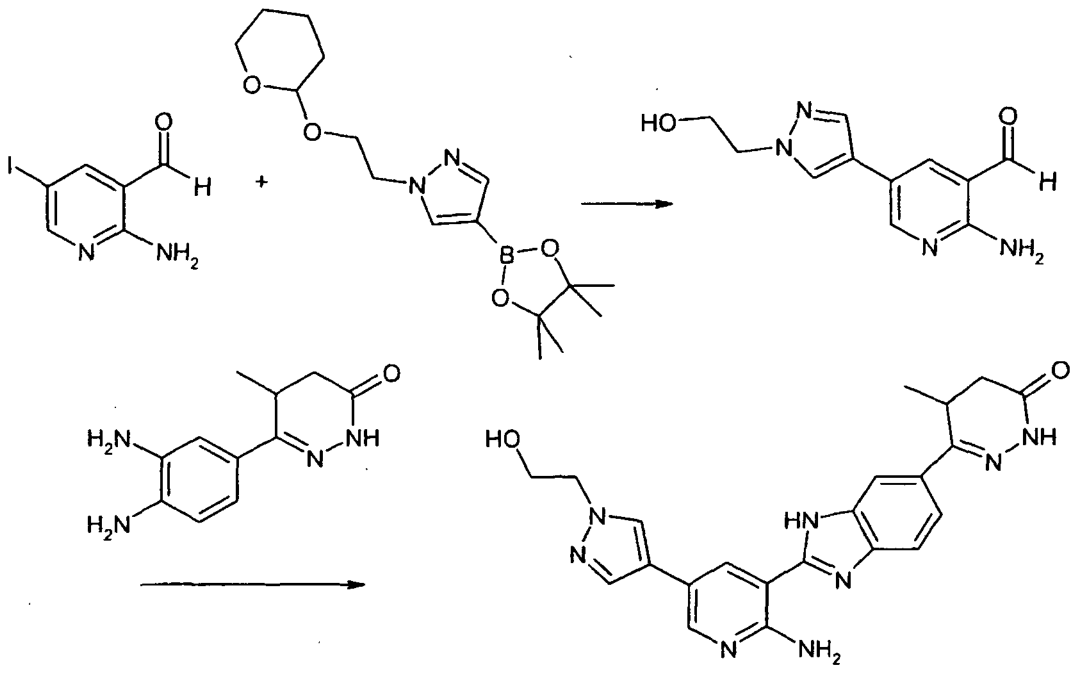

HPLC-MS $[M+H]^+$ 431.2;
$^1H$ NMR (500 MHz, DMSO-$d_6$ + TFA-$d_1$) δ [ppm] 9.09 (d, *J* = 2.0, 1H), 8.47 (d, *J* = 2.0, 1 H), 8.28 (s, 1 H), 8.11 (d, *J* = 1.0, 1 H), 8.04 (s, 1 H), 7.92 (dd, *J* = 8.7, 1.6, 1 H), 7.79 (d, *J* = 8.6, 1 H), 4.26 (t, *J* = 5.4, 2H), 3.83 (t, *J* = 5.4, 2H), 3.56 (p, *J* = 7.1, 1 H), 2.78 (dd, *J* = 16.8, 6.8, 1H), 2.33 (d, *J* = 15.6, 1 H), 1.19 (d, *J* = 7.3, 3H).

Analog erhält man

2-{4-[6-Amino-5-(5-fluor-6-methoxy-1H-benzimidazol-2-yl)-pyridin-3-yl]-pyrazol-1-yl}-1-piperidin-1-yl-ethanon ("A30")

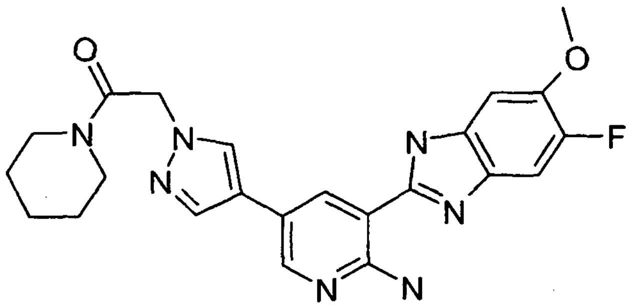

HPLC-MS $[M+H]^+$ 450.2;
$^1H$ NMR (500 MHz, DMSO-$d_6$ + TFA-$d_1$) δ [ppm] 9.05 (d, *J* = 2.0, 1 H), 8.44 (d, *J* = 2.0, 1H), 8.23 (s, 1 H), 8.03 (s, 1 H), 7.60 (d, *J* = 10.9, 1 H), 7.40 (d, *J* = 7.7, 1 H), 5.25 (s, 2H), 3.97 (s, 3H), 3.51 (s, 4H), 1.69 - 1.47 (m, 6H).

Beispiel 6

Herstellung von 3-(1H-Benzimidazol-2-yl)-5-(1-piperidin-4-yl-1 H-pyrazol-4-yl)-pyridin-2-ylamin ("A31", Vgl.-Bsp.) [Weg 1 Route A]

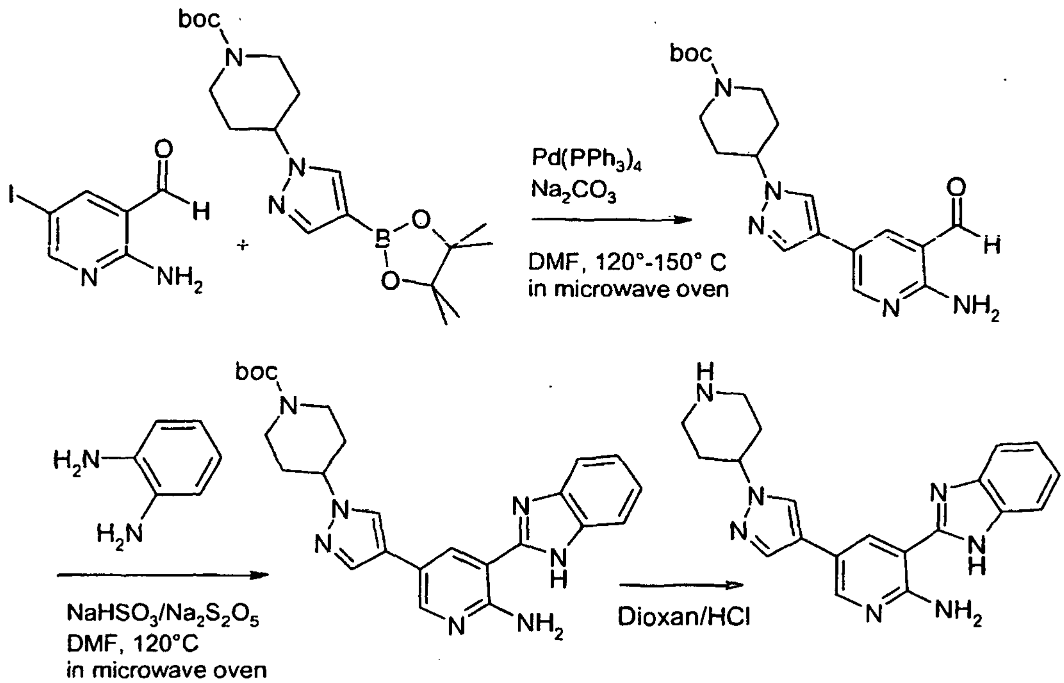


6.1 Suzuki-Kopplung:

In einem mit Stickstoff befüllten Mikrowellengefäss werden 150 mg (0.605 mmol) 2-Amino-5-iod-pyridin-3-carbaldehyd und 413 mg (1.089 mmol) 4-[4-(4,4,5,5-Tetramethyl-[1,3]dioxolan-2-yl)-pyrazol-1-yl]-piperidin-1-carbonsäure-tert.-butylester in 2.3 ml N,N-Dimethylformamid gelöst und mit 1.7 ml (3.4 mmol) 2M Natriumcarbonat-Lösung und 35 mg (0.030 mmol) Tetrakis(triphenylphosphin)-palladium(0) versetzt. Die Reaktionslösung wird 30 min bei 120°C mit Mikrowellen im Biotage SmithSynthesizer bestrahlt. Das Reaktionsgemisch wird auf Raumtemperatur gekühlt und

filtriert. Das Filtrat wird zum Rückstand eingedampft. Der ölige Rückstand wird mittels RP-HPLC aufgereinigt und man erhält 4-[4-(6-Amino-5-formyl-pyridin-3-yl)-pyrazol-1-yl]-piperidin-1-carbonäure-tert.-butylester; HPLC-MS $[M+H]^+$ 372.2.

6.2 Cyclisierung zum Benzimidazol:

Eine Lösung von 84.1 mg (0.226 mmol) 4-[4-(6-Amino-5-formyl-pyridin-3-yl)-pyrazol-1-yl]-piperidin-1-carbonsäure-tert.-butylester und 29.4 mg (0.272 mmol) o-Phenylen-diamin in 4 ml DMF wird mit 133.8 μl (0.679 mmol) Natriumhydrogensulfit-Lösung 38-40% versetzt und 30 min bei 120°C mit Mikrowellen im Biotage SmithSynthesizer bestrahlt. Das Reaktionsgemisch wird auf Raumtemperatur gekühlt und zum Rückstand eingedampft. Der Rückstand wird mittels RP-HPLC aufgereinigt: 4-{4-[6-Amino-5-(1H-benzimidazol-2-yl)-pyridin-3-yl]-pyrazol-1-yl}-piperidin-1-carbonsäure-tert.-butylester; HPLC-MS $[M+H]^+$ 460.2.

6.3 BOC-Abspaltung:

41.8 mg (0.091 mmol) 4-{4-[6-Amino-5-(1H-benzimidazol-2-yl)-pyridin-3-yl]-pyrazol-1-yl}-piperidin-1-carbonsäure-tert.-butylester werden mit 5 ml HCl in Dioxan 4M versetzt und 14 h bei Raumtemperatur gerührt. Das Reaktionsgemisch wird zum Rückstand eingedampft und mit Wasser versetzt. Die wässrige Phase wird mit NaOH 2N auf pH>10 gestellt und 3x mit Essigester extrahiert. Die organischen Phasen werden mit Natriumsulfat getrocknet, filtriert und eingedampft. Der Rückstand wird lyophilisiert. Man erhält 3-(1H-Benzimidazol-2-yl)-5-(1-piperidin-4-yl-1H-pyrazol-4-yl)-pyridin-2-ylamin als freie Base; HPLC-MS $[M+H]^+$ 360.2;
$^1H$ NMR (500 MHz, DMSO-$d_6$ + TFA-$d_1$) $\delta$ [ppm] 9.04 (d, *J*= 2.1, 1H), 8.45 (d, *J* = 2.1, 1H), 8.32 (s, 1H), 8.06 (s, 1H), 7.75 (dd, *J* = 6.1, 3.1, 2H), 7.36 (dd, *J* = 6.1, 3.1, 2H), 4.62 - 4.54 (m, 1H), 3.45 (d, *J* = 13.0, 2H), 3.15 (td, *J* = 12.6, 2.9, 2H), 2.27 (dd, *J* = 13.3, 2.9, 2H), 2.23 - 2.12 (m, 2H).

Im Allgemeinen wird die Boc-Abspaltung mit den Standardmethoden HCl in Dioxan 4N oder TFA/DCM durchgeführt und die Substanzen als freie Base oder als HCl- oder TFA-Salz isoliert.

Analog erhält man die nachstehenden Verbindungen

| Nr. | Name und/oder Struktur | Analytik |
|---|---|---|
| "A32" (Vgl.-Bsp.) | 5-(1 H-Benzimidazol-2-yl)-[3,4']bipyridinyl-6-ylamin 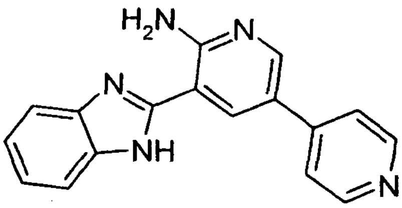 | HPLC-MS $[M+H]^+$ 288.0 |
| "A33" (Vgl.-Bsp.) | 3-(1 H-Benzimidazol-2-yl)-5-furan-2-yl-pyridin-2-ylamin 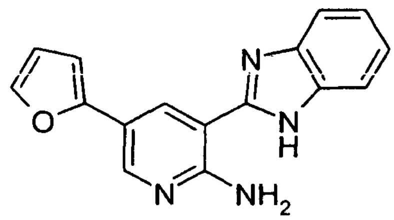 | HPLC-MS $[M+H]^+$ 277.1 |
| "A34" (Vgl.-Bsp.) | 3-(1H-Benzimidazol-2-yl)-5-(1-methyl-1H-pyrazol-4-yl)-pyridin-2-ylamin 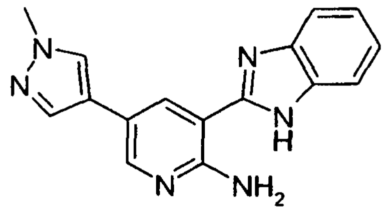 | HPLC-MS $[M+H]^+$ 291.2 |

(fortgesetzt)

| Nr. | Name und/oder Struktur | Analytik |
|---|---|---|
| $^1H$ NMR (400 MHz, DMSO-$d_6$ + TFA-$d_1$) $\delta$ [ppm] 9.01 (d, $J$ = 2.1, 1H), 8.39 (d, $J$ = 2.1, 1H), 8.17 (s, 1H), 7.96 (d, $J$ = 0.6, 1H), 7.74 (dd, $J$ = 6.1, 3.1, 2H), 7.35 (dd, $J$= 6.1, 3.2, 2H), 3.91 (s, 3H). | | |
| "A35" (Vgl.-Bsp.) | 5-(1H-Benzimidazol-2-yl)-[3,3']bipyridinyl-6,6'-diamin 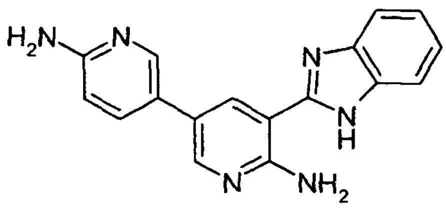 | HPLC-MS $[M+H]^+$ 303.1 |
| "A36" (Vgl.-Bsp.) | 3-(5-Isopropyl-1H-benzimidazol-2-yl)-5-(1-methyl-1H-pyrazol-4-yl)-pyridin-2-ylamin 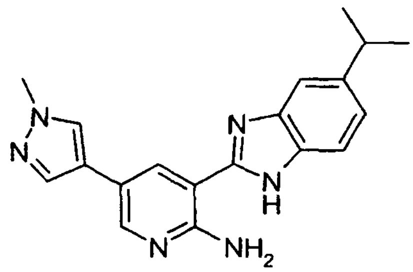 | HPLC-MS $[M+H]^+$ 333.2 |
| "A37" (Vgl.-Bsp.) | 5-(1H-Benzimidazol-2-yl)-[3,3']bipyridinyl-6-ylamin 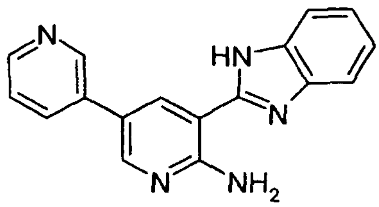 | HPLC-MS $[M+H]^+$ 288.2 |
| "A38" (Vgl.-Bsp.) | 3-(1H-Benzimidazol-2-yl)-5-furan-3-yl-pyridin-2-ylamin 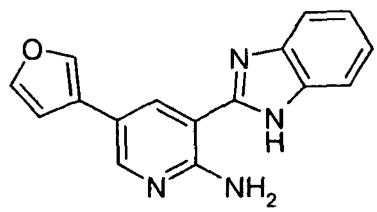 | HPLC-MS $[M+H]^+$ 277.2 |
| "A39" (Vgl.-Bsp.) | 3-(1H-Benzimidazol-2-yl)-5-(1H-pyrazol-4-yl)-pyridin-2-ylamin | HPLC-MS $[M+H]^+$ 277.2 |
| | 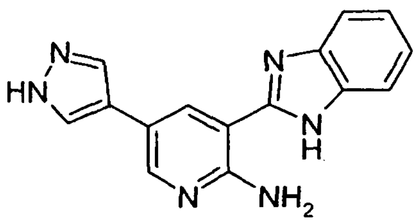 | |
| $^1H$ NMR (500 MHz, DMSO-$d_6$ + TFA-$d_1$) $\delta$ [ppm] 9.05 (d, $J$ = 2.1, 1H), 8.45 (d, $J$ = 2.1, 1H), 8.20 (s, 2H), 7.73 (dd, $J$ = 6.1, 3.1, 2H), 7.36 (dd, $J$ = 6.1, 3.1, 2H) | | |

(fortgesetzt)

| Nr. | Name und/oder Struktur | Analytik |
|---|---|---|
| "A40" (Vgl.-Bsp.) | 3-(1H-Benzimidazol-2-yl)-5-[1-(2-pyrrolidin-1-yl-ethyl)-1H-pyrazol-4-yl]-pyridin-2-ylamin 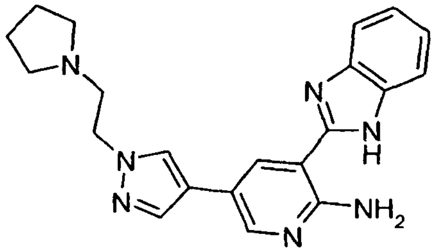 | HPLC-MS $[M+H]^+$ 374.2 |
| "A41" (Vgl.-Bsp.) | 3-(1H-Benzimidazol-2-yl)-5-pyrimidin-5-yl-pyridin-2-ylamin 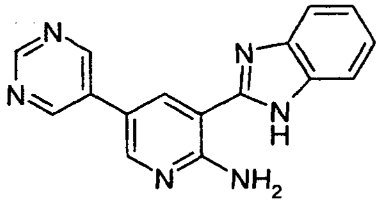 | HPLC-MS $[M+H]^+$ 289.2 |
| "A42" (Vgl.-Bsp.) | 3-(7-Methyl-1H-benzimidazol-2-yl)-5-(1-methyl-1H-pyrazol-4-yl)-pyridin-2-ylamin 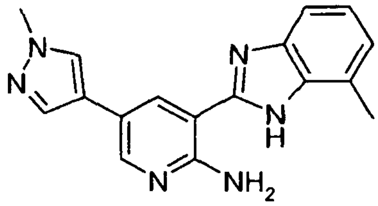 | HPLC-MS $[M+H]^+$ 305.2 |
| $^1H$ NMR (500 MHz, DMSO-$d_6$ + TFA-$d_1$) δ [ppm] 9.03 (d, *J* = 2.1, 1H), 8.41 (d, *J* = 2.1, 1H), 8.19 (s, 1H), 7.97 (d, *J* = 0.5, 1H), 7.54 (d, *J* = 8.1, 1H), 7.27 (t, *J* = 7.7, 1H), 7.16 (d, *J* = 7.3, 1H), 3.92 (s, 3H), 2.62 (s, 3H) | | |
| "A43" (Vgl.-Bsp.) | 3-(6-Methoxy-1H-benzimidazol-2-yl)-5-(1-methyl-1H-pyrazol-4-yl)-pyridin-2-ylamin | HPLC-MS $[M+H]^+$ 321.2 |
| | 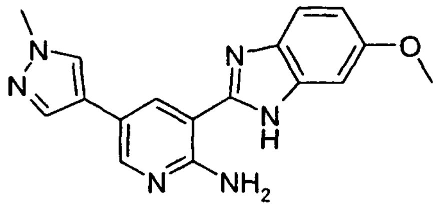 | |
| $^1H$ NMR (500 MHz, DMSO-$d_6$ + TFA-$d_1$) δ [ppm] 8.95 (d, *J* = 2.1, 1H), 8.37 (d, *J* = 2.1, 1H), 8.17 (s, 1H), 7.95 (d, *J* = 0.6, 1H), 7.64 (d, *J* = 8.8, 1H), 7.19 (d, *J* = 2.3, 1H), 7.00 (dd, *J* = 8.9, 2.4, 1H), 3.91 (s, 3H), 3.84 (s, 3H) | | |
| "A44" (Vgl.-Bsp.) | 3-(1H-Benzimidazol-2-yl)-5-(1-propyl-1H-pyrazol-4-yl)-pyridin-2-ylamin 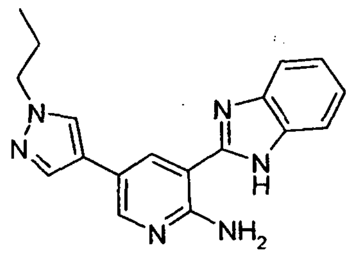 | HPLC-MS $[M+H]^+$ 319.2 |

(fortgesetzt)

| Nr. | Name und/oder Struktur | Analytik |
|---|---|---|
| "A45" (Vgl.-Bsp.) | 5-(1-Isobutyl-1H-pyrazol-4-yl)-3-(7-methyl-1H-benzimidazol-2-yl)-pyridin-2-ylamin 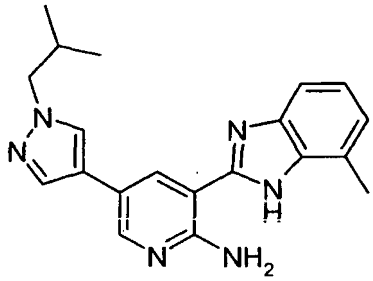 | HPLC-MS [M+H]$^+$ 347.2 |
| "A46" (Vgl.-Bsp.) | 3-(7-Methyl-1H-benzimidazol-2-yl)-5-[1-(3-methyl-butyl)-1H-pyrazol-4-yl]-pyridin-2-ylamin 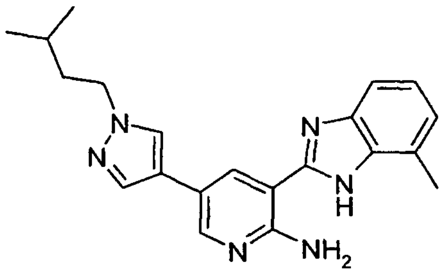 | HPLC-MS [M+H]$^+$ 361.2 |
| "A47" (Vgl.-Bsp.) | 5-(7-Methyl-1H-benzimidazol-2-yl)-[3,3']bipyridinyl-6-ylamin | HPLC-MS [M+H]$^+$ 302.0 |
| | 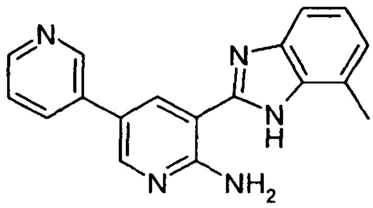 | |
| "A48" (Vgl.-Bsp.) | 3-(7-Methyl-1H-benzimidazol-2-yl)-5-[1-(2-morpholin-4-yl-ethyl)-1H-pyrazol-4-yl]-pyridin-2-ylamin 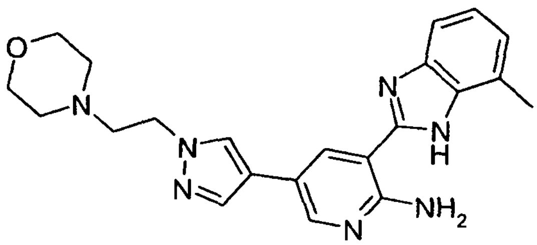 | HPLC-MS [M+H]$^+$ 404.2 |

(fortgesetzt)

| Nr. | Name und/oder Struktur | Analytik |
|---|---|---|
| "A49" | 3-(5H-[1,3]Dioxolo[4',5':4,5]benzo[1,2-d]imidazol-6-yl)-5-(1-piperidin-4-yl-1H-pyrazol-4-yl)-pyridin-2-ylamine 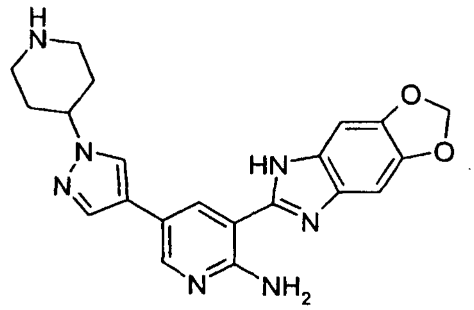 | HPLC-MS $[M+H]^+$ 404.2 |
| "A50" (Vgl.-Bsp.) | 3-(6-Fluor-1H-benzimidazol-2-yl)-5-(1-piperidin-4-yl-1H-pyrazol-4-yl)-pyridin-2-ylamin 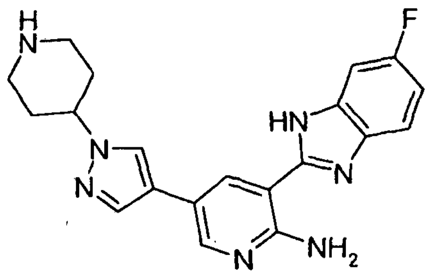 Hydrochlorid | HPLC-MS $[M+H]^+$ 378.2 |
| "A51" (Vgl.-Bsp.) | 2-[2-Amino-5-(1-methyl-1H-pyrazol-4-yl)-pyridin-3-yl]-3H-benzimidazol-5-carbonsäure | HPLC-MS $[M+H]^+$ 335.2 |
| | 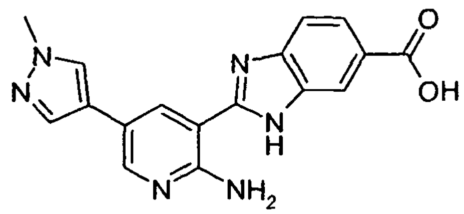 | |
| $^1H$ NMR (400 MHz, DMSO-$d_6$ + TFA-$d_1$) δ [ppm] 9.07 (d, *J* = 2.1, 1H), 8.42 (d, *J* = 2.1, 1H), 8.33 (dd, *J* = 1.5, 0.6, 1H), 8.20 (s, 1H), 8.00 - 7.95 (m, 2H), 7.78 (dd, *J* = 8.5, 0.6, 1H), 3.92 (s, 3H) | | |
| "A52" (Vgl.-Bsp.) | {2-[2-Amino-5-(1-methyl-1H-pyrazol-4-yl)-pyridin-3-yl]-3H-benzimidazol-4-yl}-methanol 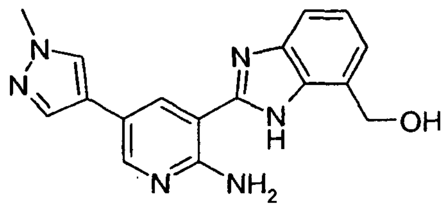 | HPLC-MS $[M+H]^+$ 321.2 |

(fortgesetzt)

| Nr. | Name und/oder Struktur | Analytik |
|---|---|---|
| "A53" (Vgl.-Bsp.) | 3-(6-tert.-Butyl-1H-benzimidazol-2-yl)-5-(1-piperidin-4-yl-1H-pyrazol-4-yl)-pyridin-2-ylamin 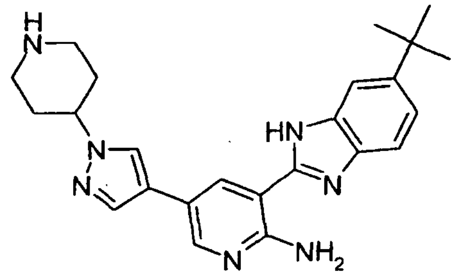 | HPLC-MS [M+H]+ 416.2 |
| "A54" | 3-(2,2-Difluor-5H[1,3]dioxolo[4',5'5]benzo[1,2-d]imidazol-6-yl)-5-(1-piperidin-4-yl-1H-pyrazol-4-yl)-pyridin-2-ylamin 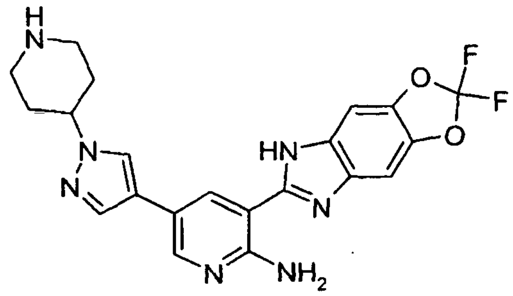 Hydrochlorid | HPLC-MS [M+H]+ 440.2 |
| "A55" | 6-[2-Amino-5-(1-piperidin-4-yl-1H-pyrazol-4-yl)-pyridin-3-yl]-3,5-dihydro-1H-benzo[1,2-d;4,5-d']diimidazol-2-on 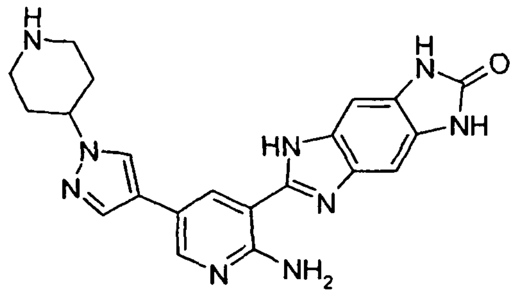 Hydrochlorid | HPLC-MS [M+H]+ 489.2 |
| "A56" (Vgl.-Bsp.) | 3-(7-Fluor-1H-benzimidazol-2-yl)-5-(1-methyl-1H-pyrazol-4-yl)-pyridin-2-ylamin 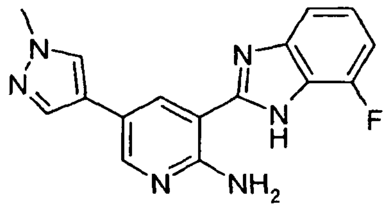 | HPLC-MS [M+H]+ 309.2 |

(fortgesetzt)

| Nr. | Name und/oder Struktur | Analytik |
|---|---|---|
| "A57" (Vgl.-Bsp.) | 3-(5,6-Difluor-1H-benzimidazol-2-yl)-5-(1-piperidin-4-yl-1H-pyrazol-4-yl)-pyridin-2-ylamin 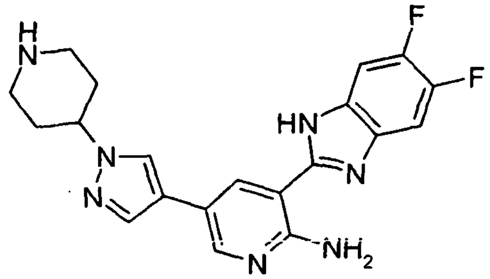 Hydrochlorid | HPLC-MS $[M+H]^+$ 396.2 |
| "A58" (Vgl.-Bsp.) | 2-[2-Amino-5-(1-methyl-1H-pyrazol-4-yl)-pyridin-3-yl]-3H-benzimidazol-5-carbonsäure-methylester 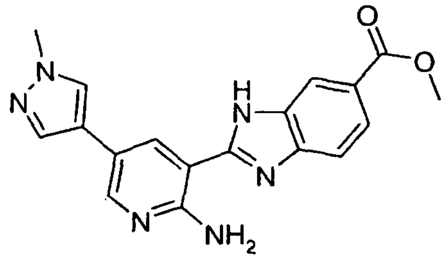 | ESI-MS $[M+H]^+$ 349.3 |
| "A59"(Vgl.-Bsp.) | 5-(1-Piperidin-4-yl-1H-pyrazol-4-yl)-3-(6-trifluormethyl-1H-benzimidazol-2-yl)-pyridin-2-ylamin | HPLC-MS $[M+H]^+$ 428.2 |
| | 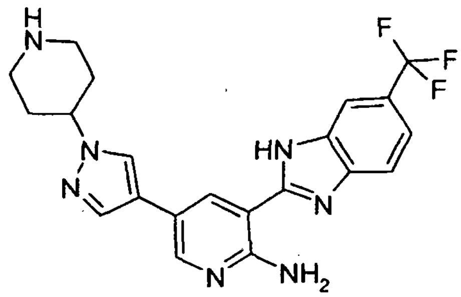 Hydrochlorid | |
| "A60" (Vgl.-Bsp.) | 3-(4-Methoxy-1H-benzimidazol-2-yl)-5-(1-piperidin-4-yl-1H-pyrazol-4-yl)-pyridin-2-ylamin 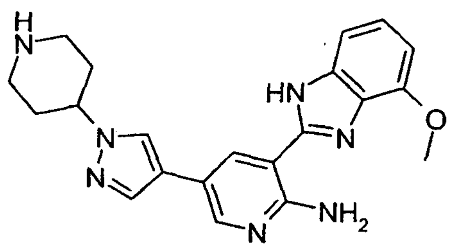 Hydrochlorid | HPLC-MS $[M+H]^+$ 390.2 |
| $^1H$ NMR (500 MHz, DMSO-$d_6$ + TFA-$d_1$) $\delta$ [ppm] 9.16 (d, *J* = 2.0, 1H), 8.47 (d, *J* = 1.8, 1H), 8.33 (s, 1H), 8.11 (d, *J* = 0.7, 1H), 7.39 - 7.29 (m, 2H), 6.95 (d, *J* = 7.0, 1H), 4.67 - 4.57 (m, 1H), 4.06 (s, 3H), 3.51 (dt, *J* = 6.2, 2.8, 2H), 3.20 (td, *J* = 12.6, 3.5, 2H), 2.36 - 2.20 (m, 4H) | | |

(fortgesetzt)

| Nr. | Name und/oder Struktur | Analytik |
|---|---|---|
| "A61" | 3-(6,7-Dihydro-1H-5,8-dioxa-1,3-diaza-cyclopenta[b]naphthalin-2-yl)-5-(1-piperidin-4-yl-1H-pyrazol-4-yl)-pyridin-2-ylamin 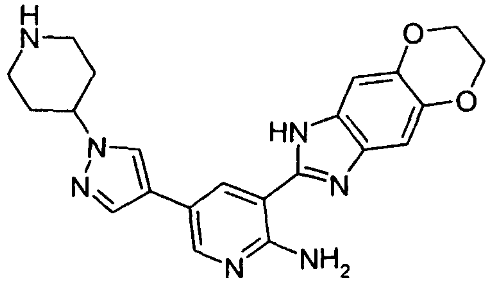 Hydrochlorid | HPLC-MS $[M^{+}H]^{+}$ 418.3 |
| "A62" (Vgl.-Bsp.) | 2-[2-Amino-5-(1-piperidin-4-yl-1H-pyrazol-4-yl)-pyridin-3-yl]-1H-benzimidazole-5-sulfonsäure-amid | HPLC-MS $[M^{+}H]^{+}$ |
| | 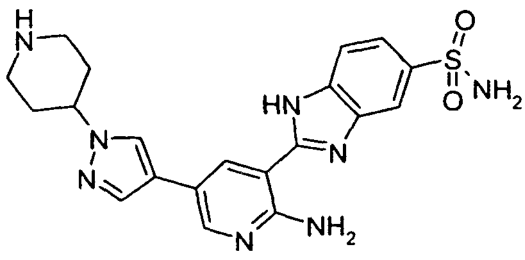 Formiat | 439.2 |
| "A63" (Vgl.-Bsp.) | N-(4-{2-[2-Amino-5-(1-piperidin-4-yl-1H-pyrazol-4-yl)-pyridin-3-yl]-3H-benzimidazol-5-yloxy}-phenyl)-acetamid 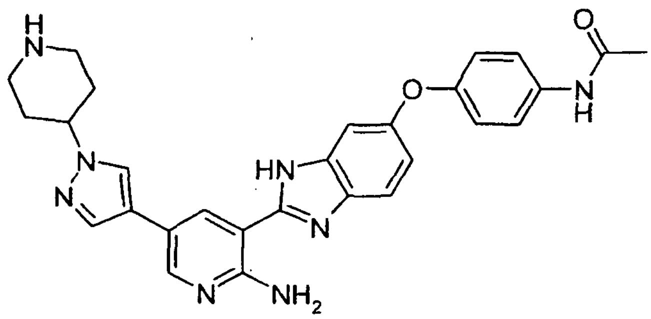 Formiat | HPLC-MS $[M+H]^{+}$ 509.2 |
| "A64" (Vgl.-Bsp.) | 3-[6-(2-Amino-ethyl)-1H-benzimidazol-2-yl]-5-(1-methyl-1H-pyrazol-4-yl)-pyridin-2-ylamin 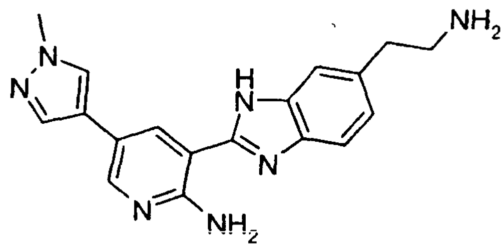 Trifluoracetat | APCI-MS $[M+H]^{+}$ 334.3 |

(fortgesetzt)

| Nr. | Name und/oder Struktur | Analytik |
|---|---|---|
| "A65" (Vgl.-Bsp.) | 2-[2-Amino-5-(1-methyl-1H-pyrazol-4-yl)-pyridin-3-yl]-3H-benzimidazole-5-carbonsäure-ethylester 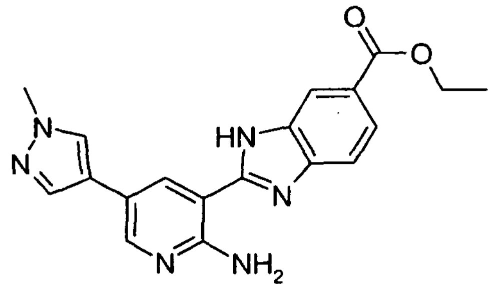 Trifluoracetat | APCI-MS $[M+H]^+$ 363.2 |
| "A66" (Vgl.-Bsp.) | 3-(5,6-Dimethoxy-1H-benzimidazol-2-yl)-5-(1-piperidin-4-yl-1H-pyrazol-4-yl)-pyridin-2-ylamin 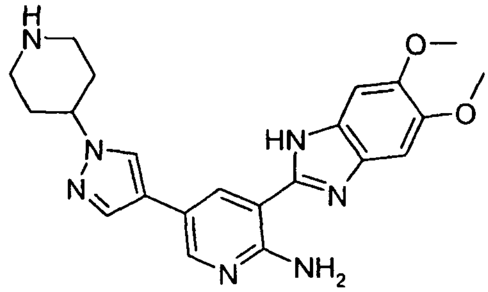 Hydrochlorid | HPLC-MS $[M+H]^+$ 420.2 |
| "A67" (Vgl.-Bsp.) | 5-(1-Piperidin-4-yl-1H-pyrazol-4-yl)-3-(5-trifluormethoxy-1H-benzimidazol-2-yl)-pyridin-2-ylamin 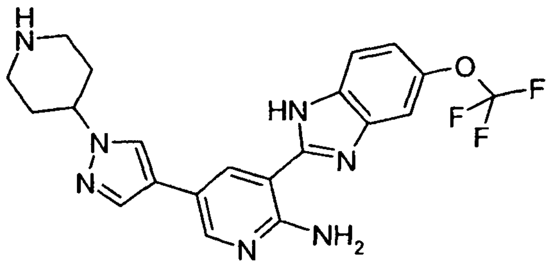 Formiat | HPLC-MS $[M+H]^+$ 444.2 |
| "A68" (Vgl.-Bsp.) | {2-[2-Amino-5-(1-methyl-1H-pyrazol-4-yl)-pyridin-3-yl]-3H-benzimidazol-5-yl}-dimethyl-amin 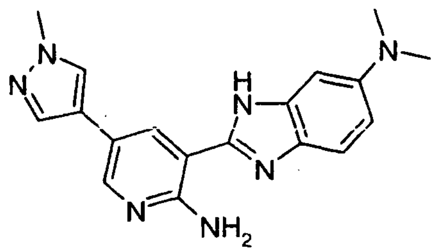 Trifluoracetat | APCI-MS $[M+H]^+$ 334.4 |
| "A69" (Vgl.-Bsp.) | 3-(5-Fluor-6-methoxy-1H-benzimidazol-2-yl)-5-(1-piperidin-4-yl-1H-pyrazol-4-yl)-pyridin-2-ylamin | HPLC-MS $[M+H]^+$ 408.2 |

(fortgesetzt)

| Nr. | Name und/oder Struktur | Analytik |
|---|---|---|
| | 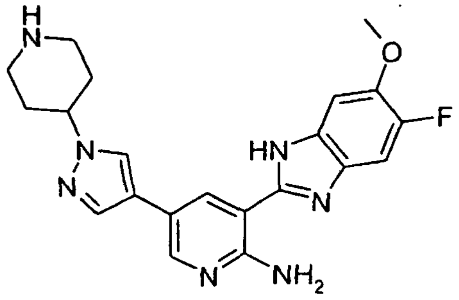 Hydrochlorid | |
| $^1$H NMR (400 MHz, DMSO-$d_6$ + TFA-$d_1$) $\delta$ [ppm] 9.10 (d, $J$ = 2.0, 1H), 8.45 (d, $J$ = 2.0, 1H), 8.39 (s, 1H), 8.10 (s, 1H), 7.63 (d, $J$ = 11.1, 1H), 7.41 (d, $J$ = 7.7, 1H), 4.65 - 4.55 (m, 1H), 3.96 (s, 3H), 3.47 (dd, $J$ = 9.6, 3.6, 2H), 3.17 (td, $J$ = 12.6, 2.9, 2H), 2.23 (dtd, $J$ = 25.5, 13.4, 3.2, 4H) | | |
| "A70" (Vgl.-Bsp.) | 3-(6-Chlor-1H-benzimidazol-2-yl)-5-(1-piperidin-4-yl-1H-pyrazol-4-yl)-pyridin-2-ylamin 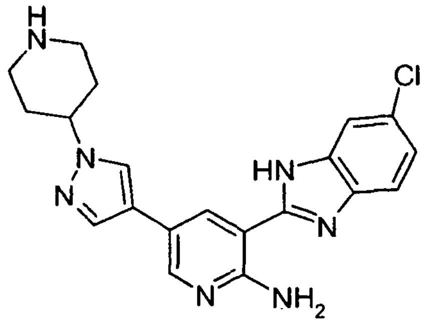 Hydrochlorid | HPLC-MS [M+H]+ 394.2 |
| "A71" | 3-[6-Fluor-5-(4-methyl-piperazin-1-yl)-1H-benzimidazol-2-yl]-5-(1-piperiden-4-yl-1H-pyrazol-4-yl)-pyridin-2-ylamin 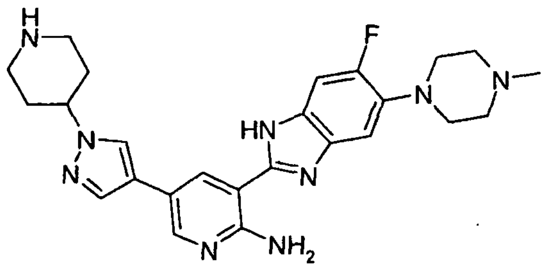 Hydrochlorid | APCI-MS [M+H]+ 476.3 |
| "A72" (Vgl.-Bsp.) | 3-(5-Chlor-7-methyl-1H-benzimidazol-2-yl)-5-(1-piperidin-4-yl-1H-pyrazol-4-yl)-pyridin-2-ylamin | HPLC-MS [M+H]+ 408.2 |
| | 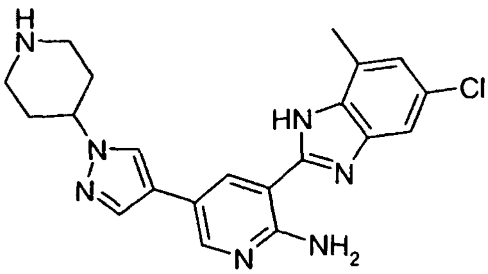 Hydrochlorid | |

(fortgesetzt)

| Nr. | Name und/oder Struktur | Analytik |
|---|---|---|
| "A73" | 3-(6-Fluor-5-morpholin-4-yl-1H-benzimidazol-2-yl)-5-(1-piperidin-4-yl-1H-pyrazol-4-yl)-pyridin-2-ylamin 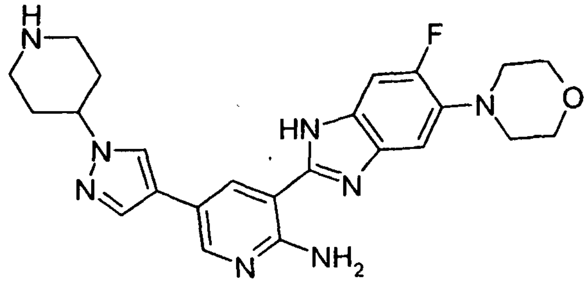 Hydrochlorid | HPLC-MS $[M+H]^+$ 463.2 |
| "A74" (Vgl.-Bsp.) | 3-(6-Fluor-1H-benzimidazol-2-yl)-5-(1-methyl-1H-pyrazol-4-yl)-pyridin-2-ylamin 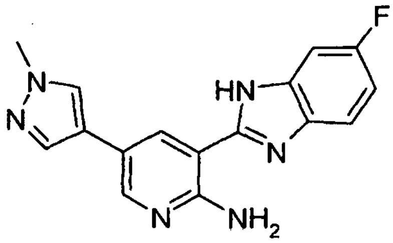 | HPLC-MS $[M+H]^+$ 309.2 |
| "A75" (Vgl.-Bsp.) | 5-(1-Methyl-1H-pyrazol-4-yl)-3-(4,5,6-trifluor-1-benzimidazol-2-yl)-pyridin-2-ylamin 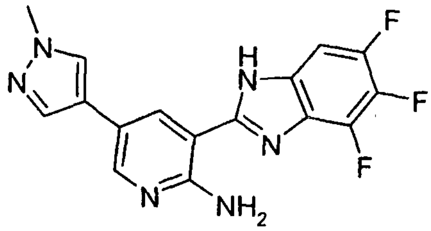 | HPLC-MS $[M+H]^+$ 345.2 |
| "A76" (Vgl.-Bsp.) | 3-(4,6-Difluor-1H-benzimidazol-2-yl)-5-(1-methyl-1H-pyrazol-4-yl)-pyridin-2-ylamin | HPLC-MS $[M+H]^+$ 327.2 |
| | 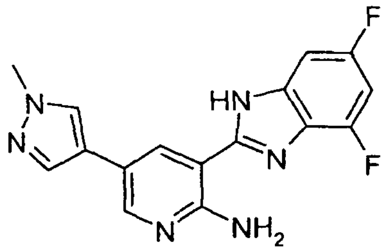 | |

(fortgesetzt)

| Nr. | Name und/oder Struktur | Analytik |
|---|---|---|
| "A77" (Vgl.-Bsp.) | {2-[2-Amino-5-(1-methyl-1H-pyrazol-4-yl)-pyridin-3-yl]-3H-benzimidazol-5-yl}-phenyl-methanon 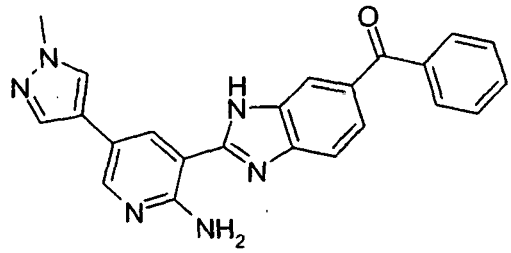 Trifluoracetat | ESI-MS $[M+H]^+$ 395.2 |
| "A78" (Vgl.-Bsp.) | 3-(6-Fluor-5-methoxy-1H-benzimidazol-2-yl)-5-(1-methyl-1H-pyrazol-4-yl)-pyridin-2-ylamin 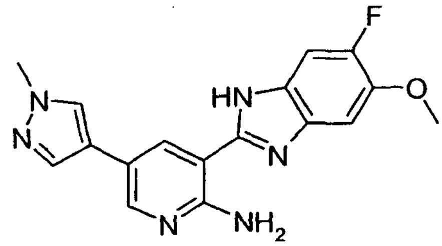 | HPLC-MS $[M+H]^+$ 339.2 |
| $^1H$ NMR (500 MHz, DMSO-$d_6$ + TFA-$d_1$) δ [ppm] 9.02 (d, *J* = 2.0, 1H), 8.39 (d, *J* = 2.0, 1H), 8.21 (s, 1H), 7.99 (s, 1H), 7.61 (d, *J* = 11.0, 1H), 7.41 (d, *J* = 7.7, 1H), 3.96 (d, J = 4.7, 6H) | | |
| "A79" (Vgl.-Bsp.) | 5-(1-Methyl-1H-pyrazol-4-yl)-3-(4,5,6,7-tetrafluor-1H-benzimidazol-2-yl)-pyridin-2-ylamin 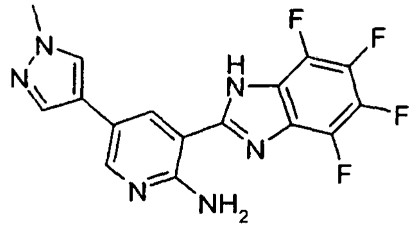 | HPLC-MS $[M+H]^+$ 363.2 |
| "A80" (Vgl.-Bsp.) | {2-[2-Amino-5-(1-methyl-1H-pyrazol-4-yl)-pyridin-3-yl]-3H-benzimidazol-5-yl}-phenyl-methanol | ESI-MS $[M+H]^+$ |
| | 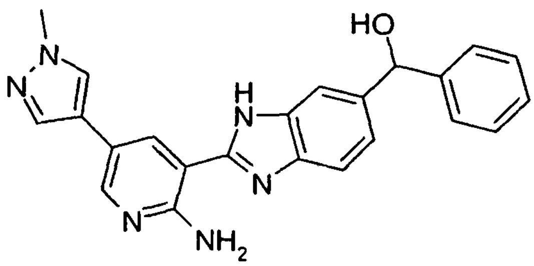 Trifluoracetat | 397.2 |

(fortgesetzt)

| Nr. | Name und/oder Struktur | Analytik |
|---|---|---|
| "A81" (Vgl.-Bsp.) | 3-(6-Benzyl-1H-benzimidazol-2-yl)-5-(1-methyl-1H-pyrazol-4-yl)-pyridin-2-ylamin 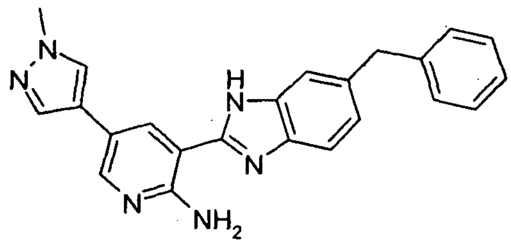 Trifluoracetat | ES-MM $[M+H]^+$ 381.3 |
| "A82" (Vgl.-Bsp.) | 3-(6-Methoxy-1H-benzimidazol-2-yl)-5-(1H-pyrazol-4-yl)-pyridin-2-ylamin 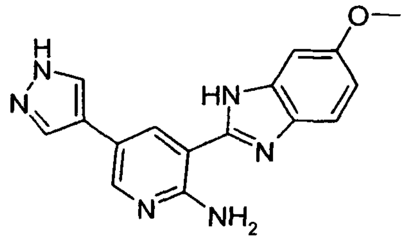 | HPLC-MS $[M+H]^+$ 307.2 |
| "A83" (Vgl.-Bsp.) | 2-[2-Amino-5-(1-methyl-1H-pyrazol-4-pyridin-3-yl]-1H-benzimidazol-5-sulfonsäure-amid 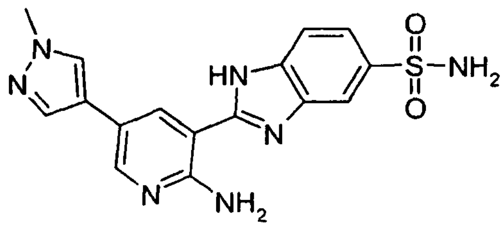 | HPLC-MS $[M+H]^+$ 370.0 |

Beispiel 7

Herstellung von 5-(6-Methoxy-1H-benzimidazol-2-yl)-6'-piperazin-1-yl-[3,3']bipyridinyl-6-ylamin ("A84", Vgl.-Bsp.) [Weg 1 Route B]

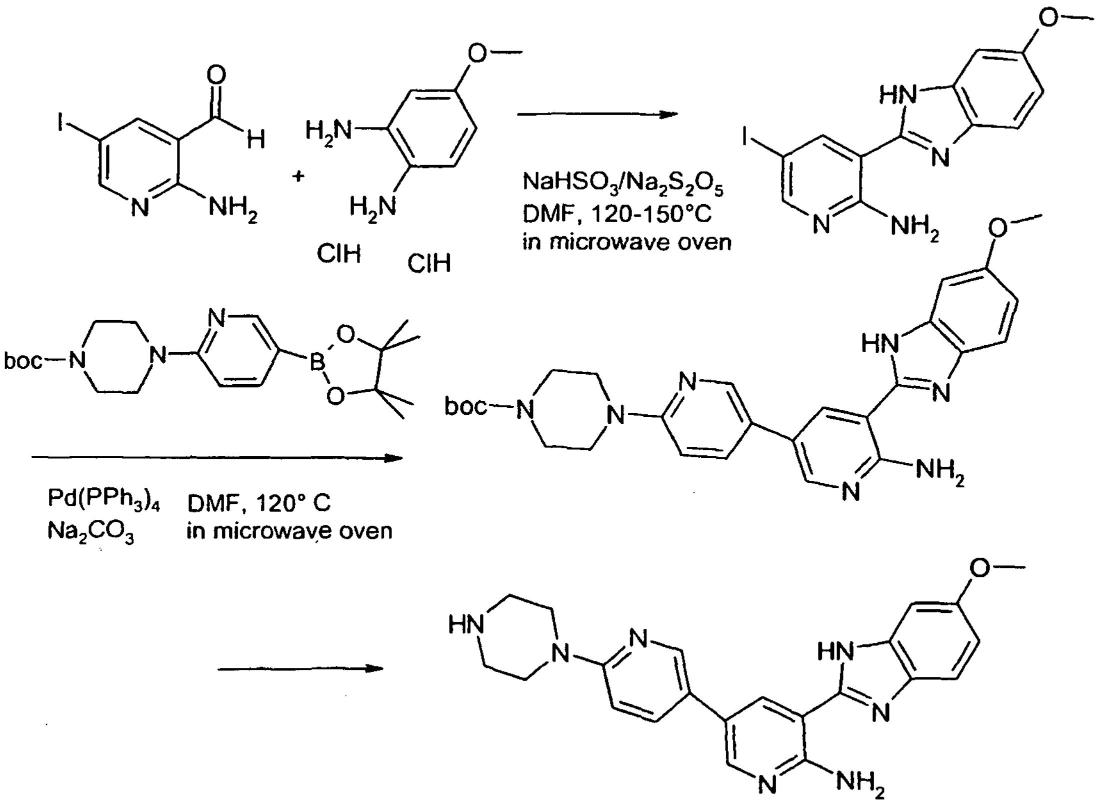


7.1 Cyclisierung zum Benzimidazol:

Eine Lösung von 1.355 g (5.462 mmol) 2-Amino-5-iod-pyridin-3-carbaldehyd und 1.412 g (6.554 mmol) 4-Methoxy-phenylen-1,2-diamin Dihydrochlorid in 17 ml DMF wird mit 3 ml (15.222 mmol) Natrium-hydrogensulfit-Lösung 38-40% versetzt und 30 min bei 150°C mit Mikrowellen im Biotage SmithSynthesizer bestrahlt. Das Reaktionsgemisch wird auf Raumtemperatur gekühlt und zum Rückstand eingedampft. Der Rückstand wird mit Wasser versetzt und 3 Mal mit Essigester extrahiert. Die vereinigten organischen Phasen werden mit Natriumsulfat getrocknet, filtriert und zum Rückstand eingedampft. Es wird dann aus MTBE kristallisiert: 5-Iod-3-(5-methoxy-1H-benzimidazol-2-yl)-pyridin-2-ylamin; HPLC-MS $[M+H]^+$ 367.0.

7.2 Suzuki-Kopplung:

In einem mit Stickstoff befüllten Mikrowellengefäss werden 128.87 mg (0.273 mmol) 5-Iod-3-(5-methoxy-1 H-benzimidazol-2-yl)-pyridin-2-ylamin und 191.38 mg (0.492 mmol) 4-[5-(4,4,5,5-Tetramethyl-[1,3,2]dioxaborolan-2-yl)-pyridin-2-yl]-piperazin-1-carbonsäure-tert.-butylester in 2.5 ml N,N-Dimethylformamid suspendiert und mit 0.6 ml (1.2 mmol) 2M Natriumcarbonat-Lösung und 31.5 mg (0.027 mmol) Tetrakis(triphenylphosphin)-palladium(0) versetzt. Die Reaktionslösung wird 30 min bei 120°C mit Mikrowellen im Biotage SmithSynthesizer bestrahlt. Das Reaktionsgemisch wird auf Raumtemperatur gekühlt und mit Wasser/Essigester verdünnt und filtriert. Die wässrige Phase wird noch 2x mit Essigester extrahiert und die vereinigten organischen Phasen mit Natriumsulfat getrocknet, filtriert und zum Rückstand eingedampft. Der Rückstand wird mittels RP-HPLC aufgereinigt und man erhält 4-[6'-Amino-5'-(6-methoxy-1H-benzimidazol-2-yl)-[3,3']bipyridinyl-6-yl]-piperazin-1-carbonsäure-tert.-butylester.

7.3 BOC-Abspaltung:

4-[6'-Amino-5'-(6-methoxy-1H-benzimidazol-2-yl)-[3,3']bipyridinyl-6-yl]-piperazine-1-carbonsäure-tert.-butylester werden mit 3 ml HCl in Dioxan 4M versetzt und 14 h bei Raumtemperatur gerührt. Das Reaktionsgemisch wird zum Rückstand eingedampft und mit Wasser versetzt. Die wässrige Phase wird mit NaOH 2N auf pH>10 gestellt und mit Essigester extrahiert. Die organischen Phasen werden mit Natriumsulfat getrocknet, filtriert und eingedampft. Der Rückstand wird mittels RP-HPLC aufgereinigt und lyophilisiert. Man erhält 5-(6-Methoxy-1H-benzimidazol-2-yl)-6'-piperazin-1-yl-[3,3']bipyridinyl-6-ylamin als freie Base; HPLC-MS $[M+H]^+$ 402.2.

Im Allgemeinen wird die Boc-Abspaltung mit den Standardmethoden HCl in Dioxan 4N oder TFA/DCM durchgeführt und die Substanzen als freie Base, HCl oder TFA Salz isoliert.

Analog erhält man die nachstehenden Verbindungen

| Nr. | Name und/oder Struktur | Analytik |
|---|---|---|
| "A85" (Vgl.-Bsp.) | 3-(1 H-Benzimidazol-2-yl)-5-(2H-pyrazol-3-yl)-pyridin-2-ylamin 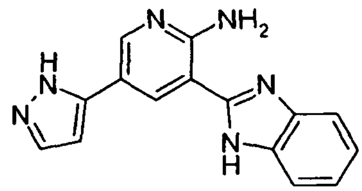 | ESI-MS $[M+H]^+$ 277.2 |
| "A86" (Vgl.-Bsp.) | 3-(1H-Benzimidazol-2-yl)-5-(2-methyl-2H-pyrazol-3-yl)-pyridin-2-ylamin 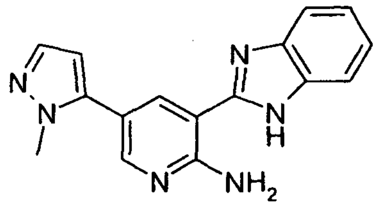 | HPLC-MS $[M+H]^+$ 291.2 |
| "A87" (Vgl.-Bsp.) | 3-(4-Ethoxy-1H-benzimidazol-2-yl)-5-(2H-pyrazol-3-yl)-pyridin-2-ylamin 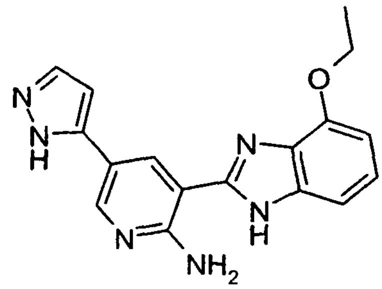 | HPLC-MS $[M+H]^+$ 321.0 |
| "A88" (Vgl.-Bsp.) | 3-(4-Ethoxy-1H-benzimidazol-2-yl)-5-(1-methyl-1H-pyrazol-4-yl)-pyridin-2-ylamin 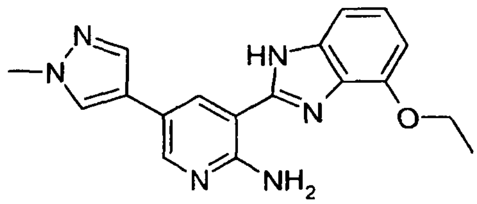 | HPLC-MS $[M+H]^+$ 335.2 |
| "A89" (Vgl.-Bsp.) | 3-(7-Methyl-1H-benzimidazol-2-yl)-5-(1-piperidin-4-yl-1H-pyrazol-4-yl)-pyridin-2-ylamin | HPLC-MS $[M+H]^+$ 374.2 |

(fortgesetzt)

| Nr. | Name und/oder Struktur | Analytik |
|---|---|---|
| | 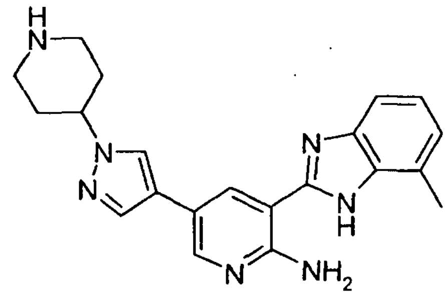<br>Hydrochlorid | |
| $^1H$ NMR (500 MHz, DMSO-$d_6$ + TFA-$d_1$) δ [ppm] 9.03 (d, *J* = 2.1, 1H), 8.45 (d, *J* = 2.1, 1H), 8.30 (s, 1H), 8.04 (s, 1H), 7.54 (d, *J* = 8.0, 1H), 7.29 - 7.25 (m, 1H), 7.16 (d, *J* = 7.3, 1H), 4.61 - 4.53 (m, 1H), 3.44 (d, *J* = 13.1, 2H), 3.14 (td, *J* = 12.7, 3.2, 2H), 2.62 (s, 3H), 2.29 - 2.15 (m, 4H) | | |
| "A90" | 3-[5-(4-Methyl-piperazin-1-yl)-1H-benzimidazol-2-yl]-5-(1-methyl-1H-pyrazol-4-yl)-pyridin-2-ylamin<br>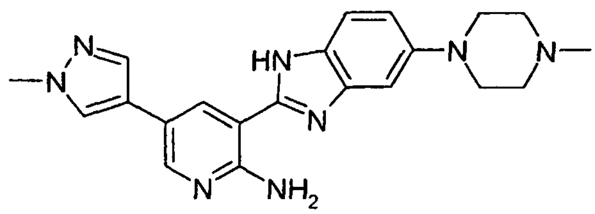 | HPLC-MS $[M+H]^+$ 389.2 |
| "A91" (Vgl.-Bsp.) | {2-[2-Amino-5-(1-methyl-1H-pyrazol-4-yl)-pyridin-3-yl]-1H-benzimidazol-5-yl}-essigsäure-ethylester<br>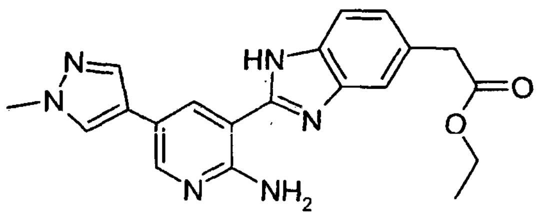 | HPLC-MS $[M+H]^+$ 377.2 |
| "A92" (Vgl.-Bsp.) | 3-(6-Methoxy-1H-benzimidazol-2-yl)-5-(1-piperidin-4-yl-1H-pyrazol-4-yl)-pyridin-2-ylamin<br>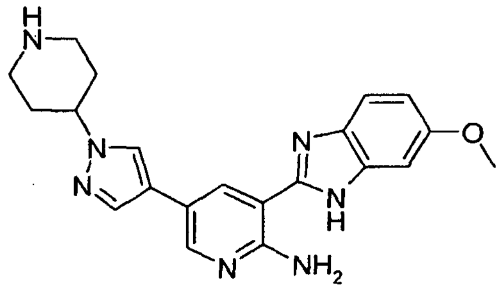 | HPLC-MS $[M+H]^+$ 390.2 |
| $^1H$ NMR (400 MHz, DMSO-$d_6$ + TFA-$d_1$) δ [ppm] 9.01 (d, *J* = 2.0, 1H), 8.45 (d, *J* = 2.1, 1H), 8.35 (s, 1H), 8.08 (s, 1H), 7.68 (d, *J* = 8.9, 1H), 7.23 (d, *J* = 2.3, 1H), 7.03 (dd, *J* = 8.9, 2.4, 1H), 4.66 - 4.55 (m, 1H), 3.88 (s, 3H), 3.52 - 3.43 (m, 2H), 3.18 (td, *J* = 12.8, 3.1, 2H), 2.23 (dtd, *J* = 15.9, 13.6, 3.6, 4H) | | |

(fortgesetzt)

| Nr. | Name und/oder Struktur | Analytik |
|---|---|---|
| "A93" (Vgl.-Bsp.) | 2-[2-Amino-5-(1-methyl-1H-pyrazol-4-yl)-pyridin-3-yl]-1H-benzimidazol-4-carbonsäure 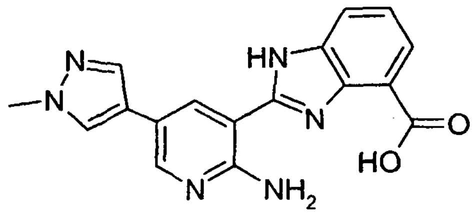 | HPLC-MS [M+H]+ 335.2 |
| "A94" (Vgl.- Bsp.) | 5-[1-(2-Dimethylamino-ethyl)-1H-pyrazol-4-yl]-3-(6-methoxy-1H-benzimidazol-2-yl)-pyridin-2-ylamin 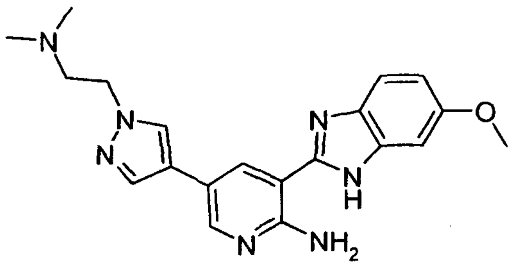 | HPLC-MS [M+H]+ 378.2 |
| "A95" (Vgl.-Bsp.) | 6'-Methoxy-5-(6-methoxy-1H-benzimidazol-2-yl)-[3,3']bipyridinyl-6-ylamine 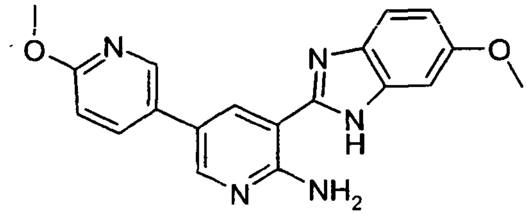 | HPLC-MS [M+H]+ 348.2 |
| "A96" (Vgl.-Bsp.) | 5-[1-(3-Dimethylamino-propyl)-1H-pyrazol-4-yl]-3-(6-methoxy-1H-benzimidazol-2-yl)-pyridin-2-ylamin | HPLC-MS [M+H]+ 392.2 |
| | 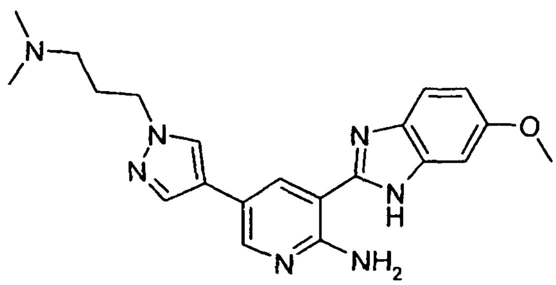 | |
| "A97" (Vgl.-Bsp.) | 3-(6-Methoxy-1H-benzimidazol-2-yl)-5-(1H-pyrrol-2-yl)-pyridin-2-ylamin 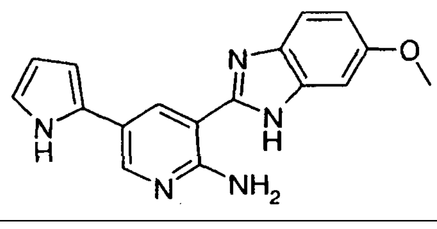 | HPLC-MS [M+H]+ 306.2 |

(fortgesetzt)

| Nr. | Name und/oder Struktur | Analytik |
|---|---|---|
| "A98" (Vgl.-Bsp.) | 3-(6-Methoxy-1H-benzimidazol-2-yl)-5-(4-methyl-3,4-dihydro-2H-pyrido[3,2-b][1,4]oxazin-7-yl)-pyridin-2-ylamin 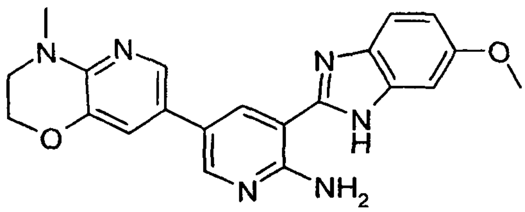 | HPLC-MS $[M+H]^+$ 389.2 |
| "A99" (Vgl.-Bsp.) | 5-(6-Methoxy-1H-benzimidazol-2-yl)-[3,3']bipyridinyl-6,6'-diamin 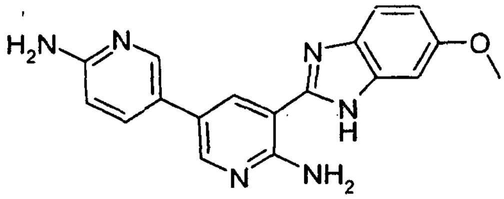 | HPLC-MS $[M+H]^+$ 333.2 |
| "A100" (Vgl.-Bsp.) | 5-Benzo[b]thiophen-2-yl-3-(6-methoxy-1H-benzimidazol-2-yl)-pyridin-2-ylamin 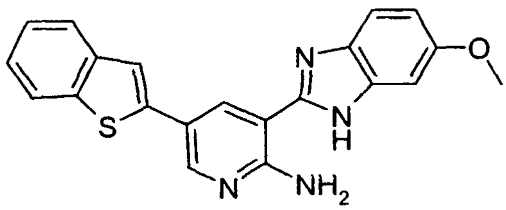 | HPLC-MS $[M+H]^+$ 373.0 |
| "A101" (Vgl.-Bsp.) | 5-Isoxazol-4-yl-3-(6-methoxy-1H-benzimidazol-2-yl)-pyridin-2-ylamin 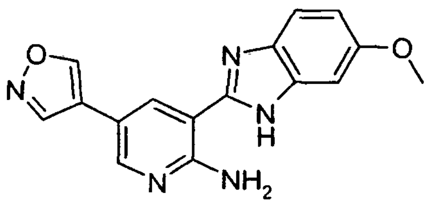 | ESI-MS $[M+H]^+$ 308.2 |
| "A102" (Vgl.-Bsp.) | 2-[2-Amino-5-(1-methyl-1H-pyrazol-4-yl)-pyridin-3-yl]-1H-benzimidazol-4-ol 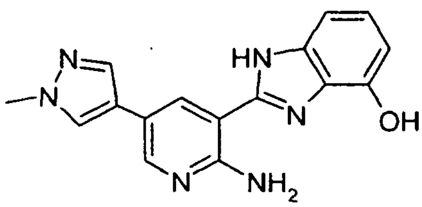 | HPLC-MS $[M+H]^+$ 307.2 |

(fortgesetzt)

| Nr. | Name und/oder Struktur | Analytik |
|---|---|---|
| "A103" (Vgl.-Bsp.) | 5-Benzofuran-2-yl-3-(6-methoxy-1H-benzimidazol-2-y)-pyridin-2-ylamin 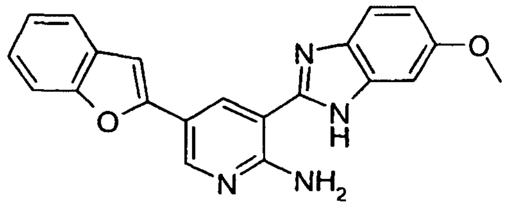 | HPLC-MS $[M+H]^+$ 357.2 |
| "A104" (Vgl.-Bsp.) | 5-(6-Methoxy-1H-benzimidazol-2-yl)-N6',N6'-dimethyl-[3,3']bipyridinyl-6,6'-diamin 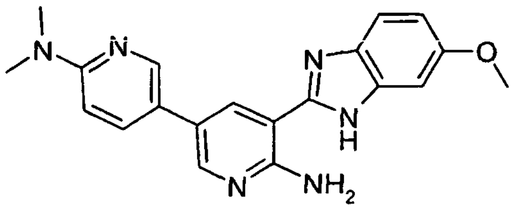 | HPLC-MS $[M+H]^+$ 361.2 |
| "A105" Vgl.-Bsp.) | 5-(1-Ethyl-1H-pyrazol-4-yl)-3-(6-methoxy-1H-benzimidazol-2-yl)-pyridin-2-ylamin 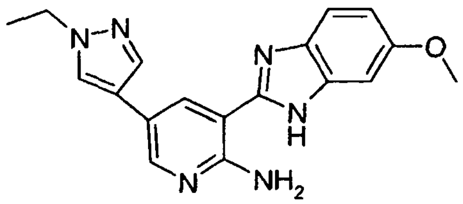 | HPLC-MS $[M+H]^+$ 335.2 |
| | Hydrochlorid | |
| $^1$H NMR (500 MHz, DMSO-$d_6$ + TFA-$d_1$) δ [ppm] 8.99 (d, *J* = 2.0, 1H), 8.40 (d, *J* = 1.9, 1H), 8.27 (s, 1H), 8.00 (s, 1H), 7.67 (d, *J* = 8.9, 1H), 7.22 (d, *J* = 2.3, 1H), 7.02 (dd, *J* = 8.8, 2.4, 1H), 4.23 (q, *J* = 7.3, 2H), 3.87 (s, 3H), 1.46 (t, *J* = 7.3, 3H) | | |
| "A106" (Vgl.-Bsp.) | 3-(6-Methoxy-1H-benzimidazol-2-yl)-5-[1-(1-methylpiperidin-4-yl)-1H-pyrazol-4-yl]-pyridin-2-ylamin 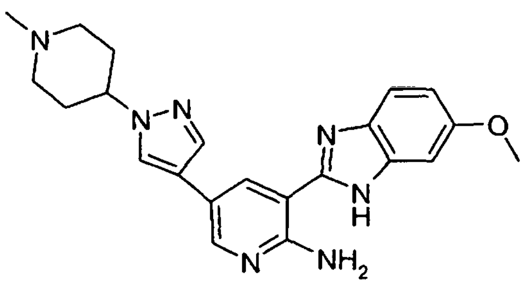 **Hydrochlorid** | HPLC-MS $[M+H]^+$ 404.2 |
| $^1$H NMR (500 MHz, DMSO-$d_6$ + TFA-$d_1$) δ [ppm] 9.01 (d, *J* = 2.0, 1H), 8.46 (d, *J* = 2.0, 1H), 8.36 (s, 1H), 8.09 (s, 1H), 7.69 (d, *J* = 8.8, 1H), 7.24 (d, *J* = 2.2, 1H), 7.04 (dd, *J* = 8.9, 2.4, 1H), 4.61 - 4.53 (m, 1H), 3.88 (s, 3H), 3.65 (d, *J* = 12.8, 2H), 3.26 (t, *J* = 11.9, 2H), 2.91 - 2.86 (m, 3H), 2.40 - 2.19 (m, 4H) | | |

(fortgesetzt)

| Nr. | Name und/oder Struktur | Analytik |
|---|---|---|
| "A107" (Vgl.-Bsp.) | 5-(1-Isopropyl-1H-pyrazol-4-yl)-3-(6-methoxy-1H-benzimidazol-2-yl)-pyridin-2-ylamin 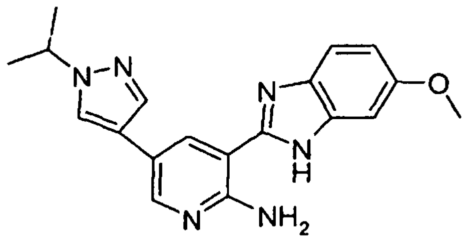 | HPLC-MS $[M+H]^+$ 349.2 |
| $^1H$ NMR (400 MHz, DMSO-$d_6$ + TFA-$d_1$) $\delta$ [ppm] 8.97 (d, $J$ = 2.1, 1H), 8.39 (d, $J$ = 2.1, 1H), 8.29 (s, 1H), 7.98 (d, $J$ = 0.6, 1H), 7.65 (d, $J$ = 8.9, 1H), 7.20 (d, $J$ = 2.3, 1H), 7.00 (dd, $J$ = 8.9, 2.4, 1H), 4.56 (hept, $J$ = 6.7, 1H), 3.85 (s, 3H), 1.48 (d, $J$ = 6.7, 6H) | | |
| "A108" (Vgl.-Bsp.) | 3-(6-Methoxy-1H-benzimidazol-2-yl)-5-(1-propyl-1H-pyrazol-4-yl)-pyridin-2-ylamin | HPLC-MS $[M+H]^+$ |
| | 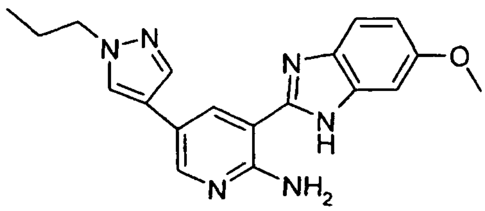 | 349.2 |
| "A109" (Vgl.-Bsp.) | 5-(6-Methoxy-1H-benzimidazol-2-yl)-6'-methyl-[3,3']bipyridinyl-6-ylamin 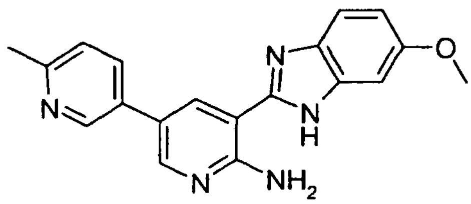 | HPLC-MS $[M+H]^+$ 332.2 |
| "A110" (Vgl.-Bsp.) | 2'-Fluor-5-(6-methoxy-1H-benzimidazol-2-yl)-6'-methyl-[3,3']bipyridinyl-6-ylamin 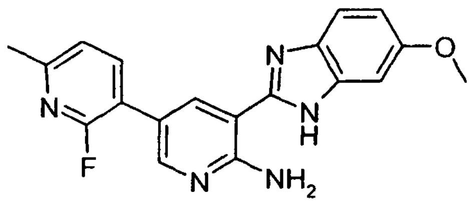 | HPLC-MS $[M+H]^+$ 350.2 |
| "A111" (Vgl.-Bsp.) | 6'-Amino-5'-(1H-benzimidazol-2-yl)-[3,3']bipyridinyl-6-carbonitril 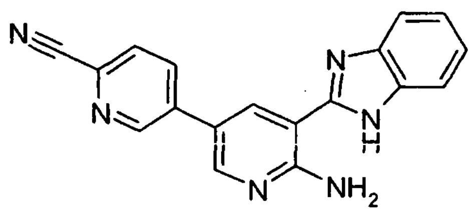 | HPLC-MS $[M+H]^+$ 313.2 |

(fortgesetzt)

| Nr. | Name und/oder Struktur | Analytik |
|---|---|---|
| "A112" (Vgl.-Bsp.) | 4-[6-Amino-5-(6-methoxy-1H-benzimidazol-2-yl)-pyridin-3-yl]-pyrazol-1-carbonsäure-dimethylamid 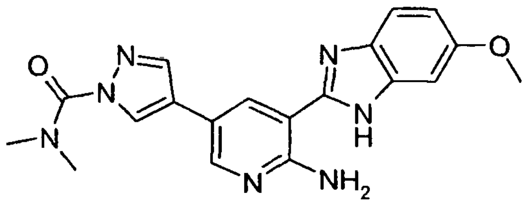 | HPLC-MS $[M+H]^+$ 378.2 |
| "A113" (Vgl.-Bsp.) | 3-(6-Methoxy-1H-benzimidazol-2-yl)-5-(1-thiophen-2-ylmethyl-1H-pyrazol-4-yl)-pyridin-2-ylamin | HPLC-MS $[M+H]^+$ |
| | 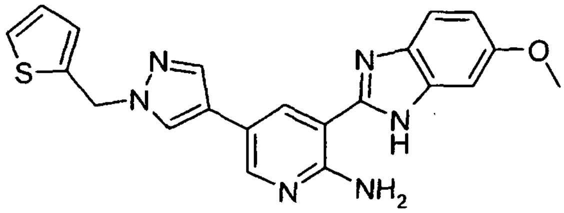 | 403.2 |
| "A114" (Vgl.-Bsp.) | 3-(1H-Benzimidazol-2-yl)-5-(1-piperidin-4-yl-1H-pyrazol-4-yl)-pyridin-2-ylamin 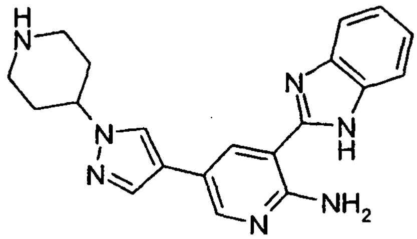 Hydrochlorid | EI-MS $[M]^+$ 359.3 |
| $^1H$ NMR (400 MHz, DMSO-$d_6$ + TFA-$d_1$) $\delta$ [ppm] 9.31 (d, *J* = 2.0, 1H), 8.53 (d, *J* = 1.9, 1H), 8.50 (s, 1H), 8.16 (s, 1H), 7.78 (dd, *J* = 6.1, 3.1, 2H), 7.39 (dd, *J* = 6.1, 3.1, 2H), 4.66 - 4.55 (m, 1H), 3.47 (d, *J* = 13.0, 2H), 3.18 (td, *J* = 12.3, 2.7, 2H), 2.35 - 2.16 (m, 4H) | | |
| "A115" | 3-(1,7-Dihydro-imidazo[4,5-f]indazol-6-yl)-5-(1-methyl-1H-pyrazol-4-yl)-pyridin-2-ylamin 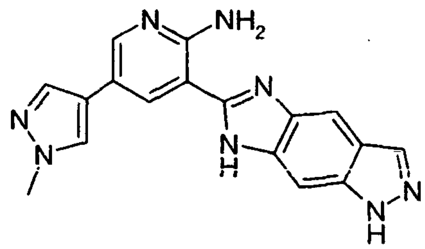 | HPLC-MS $[M+H]^+$ 331.2 |

(fortgesetzt)

| Nr. | Name und/oder Struktur | Analytik |
|---|---|---|
| "A116" (Vgl.-Bsp.) | 3-(5-Methoxy-1H-benzimidazol-2-yl)-5-[2-(4-methylpiperazin-1-yl)-thiazol-4-yl]-pyridin-2-ylamin 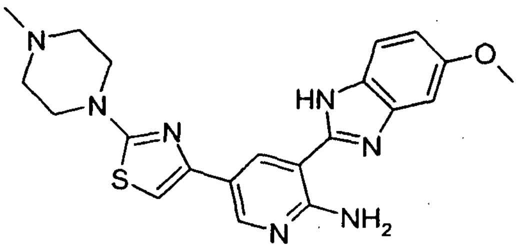 | HPLC-MS $[M+H]^+$ 422.2 |

Beispiel 8

Herstellung von 3-(1H-Imidazo[4,5-b]pyridin-2-yl)-5-(1-methyl-1H-pyrazol-4-yl)-pyridin-2-ylamin ("A117", Vgl.-Bsp.) [Weg 2]

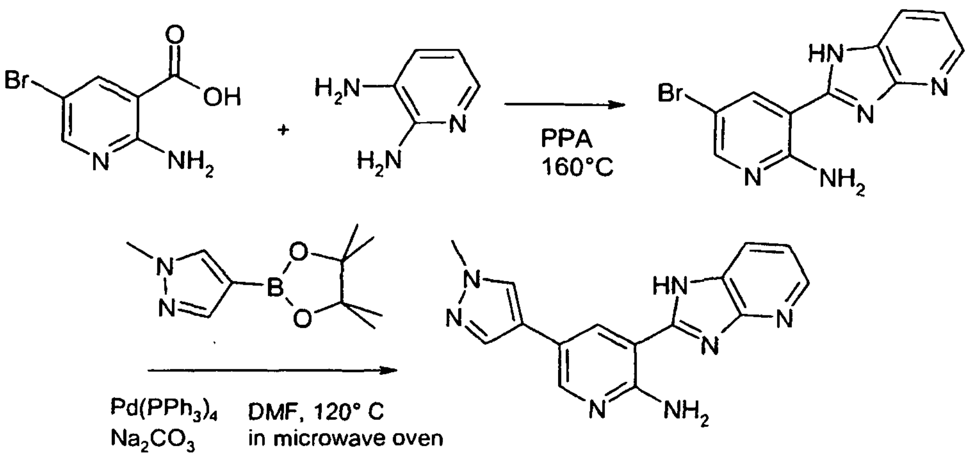


8.1 Cyclisierung zum Benzimidazol:

Zu 451.5 mg (5 mmol) Polyphosphorsäure werden 50 mg (0.230 mmol) I) 2-Amino-5-brom-nicotinsäure und 27.6 mg (0.253 mmol) Pyridin-2,3-diamin zugegeben und 4 Tage bei 160°C gerührt. Das Reaktionsgemisch wird auf Raumtemperatur gekühlt, mit Wasser versetzt und mittels NaOH 1N auf pH 12 gestellt. Der entstehende Niederschlag wird abgesaugt und getrocknet. Man erhält 5-Brom-3-(1H-imidazo[4,5-b]pyridin-2-yl)-pyridin-2-ylamin.

8.2 Suzuki-Kopplung:

In einem mit Stickstoff befüllten Mikrowellengefäss werden 58 mg (0.200 mmol) 5-Brom-3-(1H-imidazo[4,5-b]pyridin-2-yl)-pyridin-2-ylamin und 45.8 mg (0.220 mmol) 1-Methyl-4-(4,4,5,5-tetramethyl-[1,3,2]dioxaborolan-2-yl)-1H-pyrazol in 2 ml N,N-Dimethylformamid suspendiert und mit 0.57 ml (1.142 mmol) 2M Natriumcarbonat-Lösung und 23.1 mg (0.020 mmol) Tetrakis-(triphenylphosphine)-palladium(0) versetzt. Die Reaktionslösung wird 30 min bei 120°C mit Mikrowellen im Biotage SmithSynthesizer bestrahlt. Das Reaktionsgemisch wird zum Rückstand eingedampft und der Rückstand wird mittels RP-HPLC aufgereinigt und man erhält 3-(1H-Imidazo[4,5-b]pyridin-2-yl)-5-(1-methyl-1 H-pyrazol-4-yl)-pyridin-2-ylamin; HPLC-MS $[M+H]^+$ 292.2; $^1H$ NMR (500 MHz, DMSO-$d_6$ + TFA-$d_1$) $\delta$ [ppm] 9.16 (d, $J$ = 2.1, 1H), 8.67 (dd, $J$ = 5.3, 1.1, 1 H), 8.56 (d, $J$ = 7.8, 1H), 8.52 (d, $J$ = 2.1, 1H), 8.20 (s, 1 H), 7.99 (s, 1H), 7.65 (dd, $J$ = 8.0, 5.4, 1H) 3.92 (s, 3H).

Analog erhält man die Verbindung 3-(6-Methyl-7H-purin-8-yl)-5-(1-methyl-1H-pyrazol-4-yl)-pyridin-2-ylamin ("A118", Vgl.-Bsp.)

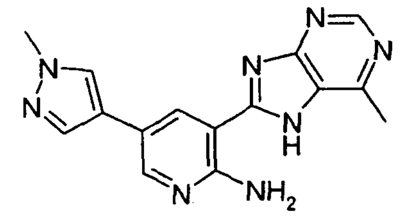

[Suzuki-Bedingungen: tri-Kaliumphosphat Trihydrate anstatt Natriumcarbonat]; HPLC-MS $[M+H]^+$ 307.2;
$^1H$ NMR (400 MHz, DMSO-$d_6$) δ [ppm]

<u>Beispiel 9</u>

Herstellung von 2-{2-[2-Amino-5-(1-methyl-1H-pyrazol-4-yl)-pyridin-3-yl]-1H-benzimidazoi-5-yl}-N-(3,4-difluor-benzyl)-acetamid ("A119")

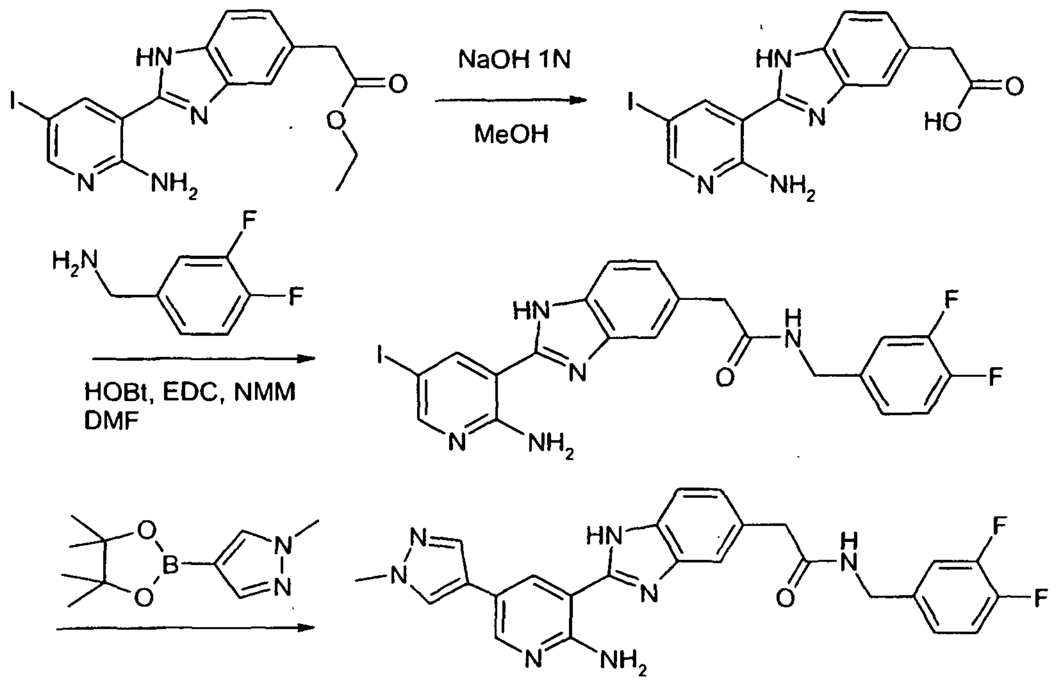


9.1 [2-(2-Amino-5-iod-pyridin-3-yl)-1H-benzimidazol-5-yl]-essigsäure-ethylester wird analog zu Weg1 Route B hergestellt.

9.2 51 mg (0.121 mmol) [2-(2-Amino-5-iod-pyridin-3-yl)-1 H-benzimidazol-5-yl]-essigsäure-ethylester werden in 5 ml Methanol gelöst und mit 242 μl NaOH 1 N (0.242 mmol) versetzt. Das Reaktionsgemisch wird 10 min bei 100°C in einem Mikrowellengerät bestrahlt. Nach Abkühlen wird es zum Rückstand eingedampft und mittels HCl 1 N auf pH 4-5 eingestellt. Der entstehende Niederschlag wird abgesaugt und getrocknet. Man erhält [2-(2-Amino-5-iod-pyridin-3-yl)-1 H-benzimidazol-5-yl]-essigsäure; HPLC-MS $[M+H]^+$ 395.0.

9.3 44mg [2-(2-Amino-5-iod-pyridin-3-yl)-1H-benzimidazol-5-yl]-essigsäure (0.112 mmol) werden in 2 ml DMF gelöst. 17.5 mg (0.123mmol) 3,4-Difluor-benzylamin, 22.4 mg (0.145 mmol) HOBt, 23.5 mg (0.123 mmol) EDC und 12.9 μl (0.117 mmol) N-Methylmorpholin werden zugegeben. Das Reaktionsgemisch wird 3 Tage bei Raumtemperatur gerührt und danach mit Wasser versetzt. Der entstehende Niederschlag wird abgesaugt, mit Wasser gewaschen und gefriergetrocknet. Man erhält: 2-[2-(2-Amino-5-iod-pyridin-3-yl)-1H-benzimidazol-5-yl]-N-(3,4-difluor-benzyl)-acetamid; HPLC-MS $[M+H]^+$ 520.0.

9.4 Unter Standard-Suzuki-Bedingungen (Weg 1 Route B) erhält man 2-{2-[2-Amino-5-(1-methyl-1 H-pyrazol-4-yl)-pyridin-3-yl]-1H-benzimidazol-5-yl}-N-(3,4-difluor-benzyl)-acetamid HPLC-MS $[M+H]^+$ 474.2; $^1H$ NMR (400 MHz, DMSO-$d_6$ + TFA-$d_1$) δ [ppm] 9.05 (d, *J* = 2.1, 1H), 8.46 (d, *J* = 2.1, 1H), 8.24 (s, 1H), 8.02 (d, *J* = 0.7, 1H), 7.76 - 7.71

(m, 2H), 7.39 - 7.23 (m, 3H), 7.13 (s, 1H), 4.33 (s, 2H), 3.98 (s, 3H), 3.72 (s, 2H).

Beispiel 10

Herstellung von 2-{2-[2-Amino-5-(1-methyl-1H-pyrazol-4-yl)-pyridin-3-yl]-1H-benzimidazol-5-yl}-acetamid ("A120")

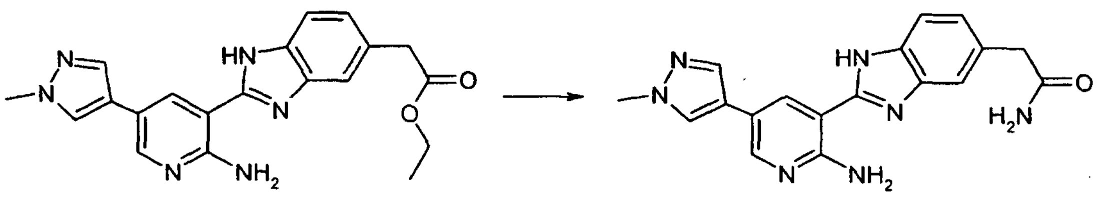

Eine Lösung von "A91" in Methanol wird mit einem Überschuss an Ammoniaklösung versetzt. Das Reaktionsgemisch wird 14 h bei Raumtemperatur gerührt. Danach wird das Lösungsmittel abgetrennt. Der Rückstand wird mittels präparativer HPLC gereinigt. Man erhält 2-{2-[2-Amino-5-(1-methyl-1H-pyrazol-4-yl)-pyridin-3-yl]-1H-benzimidazol-5-yl}-acetamid Trifluoroacetat; APCI-MS $[M+H]^+$ 348;
$^1$H NMR (400 MHz, DMSO-$d_6$ + TFA-$d_1$) $\delta$ [ppm] 8.98 (d, $J$ = 2.0, 1H), 8.39 (d, $J$ = 2.0, 1H), 8.19 (s, 1H), 7.97 (s, 1H), 7.67 (d, $J$ = 8.4, 1H), 7.63 (d, $J$ = 0.7, 1H), 7.28 (dd, $J$ = 8.4, 1.5, 1 H), 3.92 (s, 3H), 3.56 (s, 2H).

Analog erhält man die nachstehenden Verbindungen

2-{2-[2-Amino-5-(1-methyl-1H-pyrazol-4-yl)-pyridin-3-yl]-3H-benzimidazol-5-yl}-N-methyl-acetamid Trifluoracetat ("A121")

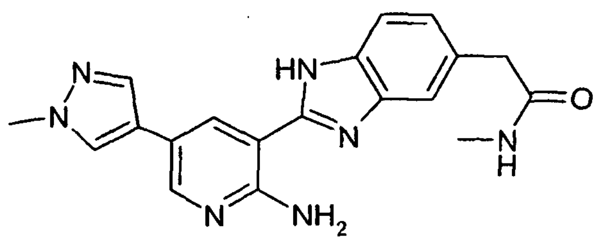

ESI-MS $[M+H]^+$ 362.3;
$^1$H NMR (500 MHz, DMSO-$d_6$ + TFA-$d_1$) $\delta$ [ppm] 9.02 (d, $J$ = 2.1, 1H), 8.44 (d, $J$ = 2.1, 1H), 8.21 (s, 1H), 8.00 (s, 1H), 7.69 (dd, $J$ = 13.8, 4.4, 2H), 7.32 (dd, $J$ = 8.4, 1.4, 1H), 3.95 (s, 3H), 3.62 (s, 2H), 2.64 (s, 3H);

2-{2-[2-Amino-5-(1-methyl-1H-pyrazol-4-yl)-pyridin-3-yl]-3H-benzimidazol-5-yl}-N-methyl-acetamid Trifluoracetat ("A122")

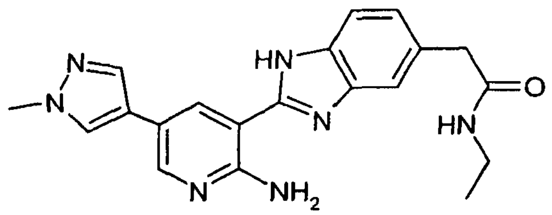

ESI-MS $[M+H]^+$ 376.3;
$^1$H NMR (500 MHz, DMSO-$d_6$ + TFA-$d_1$) $\delta$ [ppm] 9.02 (d, $J$ = 2.1, 1H), 8.44 (d, $J$ = 2.1, 1H), 8.21 (s, 1 H), 8.00 (d, $J$ = 0.5, 1 H), 7.71 (d, $J$ = 8.4, 1 H), 7.68 (s, 1 H), 7.33 (dd, $J$ = 8.4, 1.4, 1 H), 3.95 (s, 3H), 3.61 (s, 2H), 3.13 (q, $J$ = 7.2, 2H),

1.07 (t, *J* = 7.2, 3H).

Beispiel 11

Herstellung von 2-[2-Amino-5-(1-methyl-1H-pyrazol-4-yl)-pyridin-3-yl]-3H-benzimidazol-5-carbonsäure-isopropylamid ("A123")

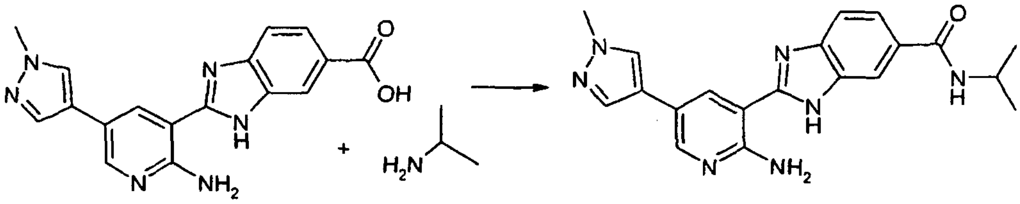

70 mg "A51" (0.209 mmol) werden in 10 ml DMF gelöst. 54 µl (0.630 mmol) Isopropylamin, 28 mg (0.210 mmol) HOBt, 81 mg (0.420 mmol) EDC und 69.2 µl (0.630 mmol) N-Methylmorpholin werden zugegeben. Das Reaktionsgemisch wird 2 Tage bei 80°C gerührt und dann mit Essigester versetzt. Die organische Phase wird 3x mit Wasser gewaschen, mit Natriumsulfat getrocknet, filtriert und das Lösungsmittel abgetrennt. Der Rückstand wird mittels RP-HPLC aufgereinigt und lyophilisiert. Man erhält 2-[2-Amino-5-(1-methyl-1H-pyrazol-4-yl)-pyridin-3-yl]-3H-benzimidazol-5-carbonsäure-isopropylamid Trifluoroacetat; ESI-MS $[M+H]^+$ 376.3;
$^1H$ NMR (500 MHz, DMSO-$d_6$ + TFA-$d_1$) δ [ppm] 9.10 (d, *J* = 2.1, 1 H), 8.45 (d, *J* = 2.1, 1 H), 8.29 (d, *J* = 1.0, 1 H), 8.22 (s, 1 H), 8.01 (d, *J* = 0.5, 1 H), 7.92 (dd, *J* = 8.5, 1.6, 1 H), 7.78 (d, *J* = 8.5, 1 H), 4.23 - 4.14 (m, 1 H), 3.96 (s, 3H), 1.24 (d, *J* = 6.6, 6H).

Analog erhält man die nachstehenden Verbindungen

| Nr. | Name und/oder Struktur | Analytik |
|---|---|---|
| "A124" | 2-[2-Amino-5-(1-methyl-1H-pyrazol-4-yl)-pyridin-3-yl]-3H-benzimidazole-5-carbonsäure-dimethylamid 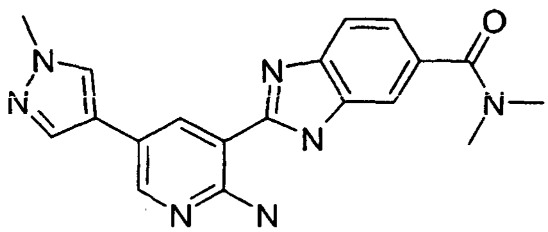 Trifluoracetat | APCI-MS $[M+H]^+$ 362.3 |
| $^1H$ NMR (400 MHz, DMSO-$d_6$ + TFA-$d_1$) δ [ppm] 9.12 (d, *J* = 2.1, 1H), 8.46 (d, *J* = 2.0, 1H), 8.23 (s, 1H), 8.02 (d, *J* = 0.6, 1H), 7.82 (dd, *J* = 10.7, 4.8, 2H), 7.44 (dd, *J* = 8.3, 1.5, 1H), 3.97 (s, 3H), 3.05 (s, 6H) | | |
| "A125" | 4-({2-[2-Amino-5-(1-methyl-1H-pyrazol-4-yl)-pyridin-3-yl]-3H-benzimidazole-5-carbonyl}-amino)-piperidin-1-carbonsäure-tert.-butylester | HPLC-MS $[M+H]^+$ 517.2 |

(fortgesetzt)

| Nr. | Name und/oder Struktur | Analytik |
|---|---|---|
| | 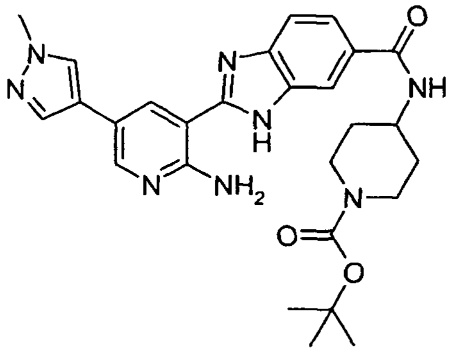 | |
| "A126" | 2-[2-Amino-5-(1-methyl-1H-pyrazol-4-yl)-pyridin-3-yl]-3H-benzimidazol-5-carbonsäure-piperidin-4-ylamid 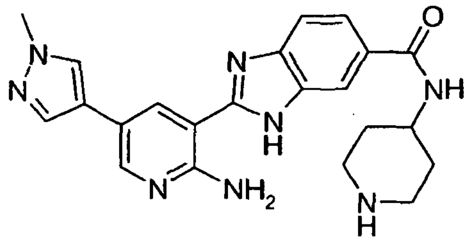 Dihydrochlorid [durch BOC-Abspaltung aus "A125"] | HPLC-MS $[M+H]^+$ 417.2 |
| | | |
| "A127" | 2-[2-Amino-5-(1-methyl-1H-pyrazol-4-yl)-pyridin-3-yl]-1H-benzimidazol-4-carbonsäure-(2-methoxy-ethyl)-amid 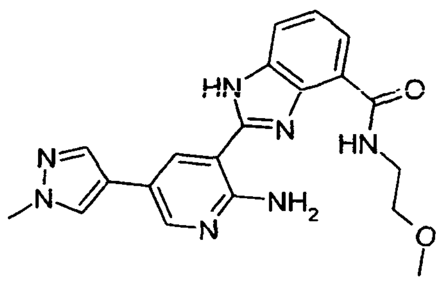 [durch Umsetzung von "A93" mit 2-Methoxy-ethylamin] | HPLC-MS $[M+H]^+$ 392.2 |
| $^1$H NMR (500 MHz, DMSO-$d_6$ + TFA-$d_1$) δ [ppm] 9.16 (d, *J* = 2.0, 1H), 8.46 (d, *J* = 2.0, 1H), 8.23 (s, 1H), 8.01 (s, 1H), 7.97 - 7.87 (m, 2H), 7.46 (t, *J* = 7.8, 1H), 3.92 (s, 3H), 3.60 (dt, *J* = 25.7, 5.2, 4H), 3.35 (s, 3H) | | |

Beispiel 12

Herstellung von 2-[2-Amino-5-(1-methyl-1 H-pyrazol-4-yl)-pyridin-3-yl]-3H-benzimidazol-5-carbonsäure-[2-(3,4-difluorphenyl)-ethyl]-amid ("A128")

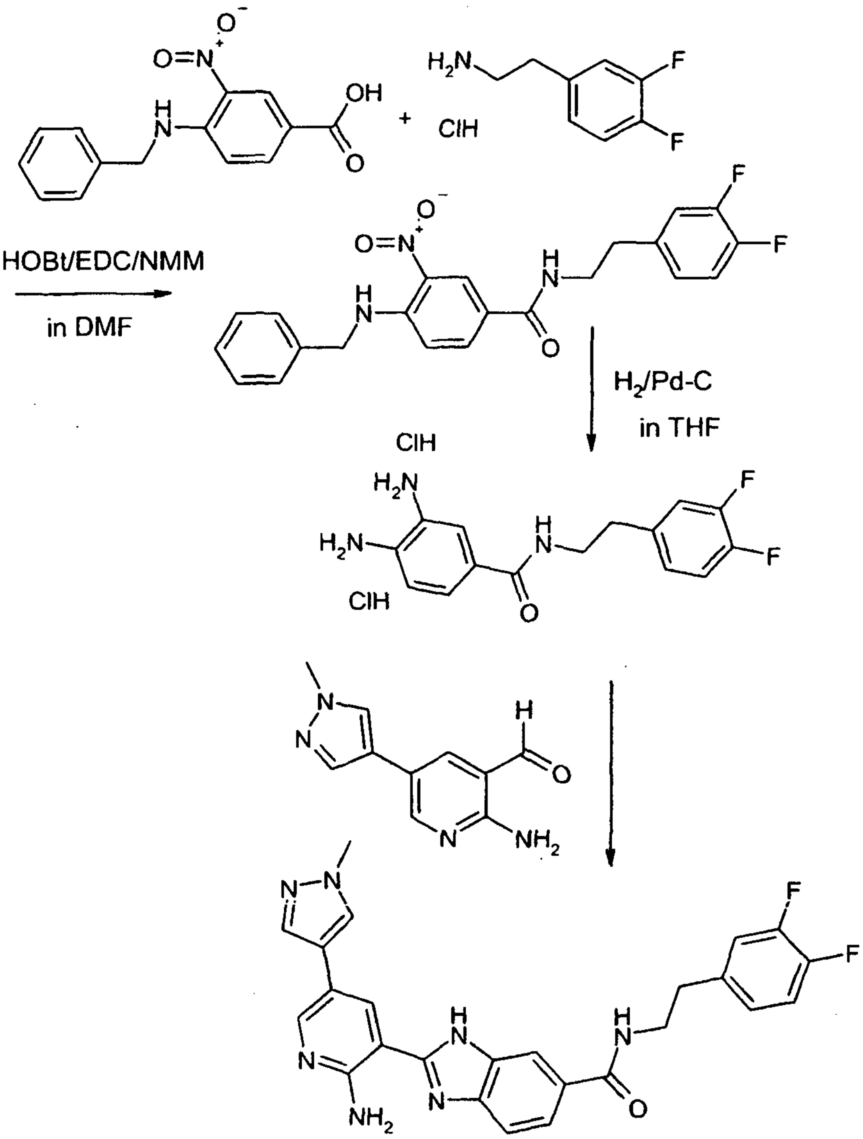


12.1 511 mg (1.877 mmol) 4-Benzylamino-3-nitro-benzoesäure werden in 20 ml DMF gelöst. 1.092 g (5.640mmol) 2-(3,4-Difluor-phenyl)-ethylamin Hydrochlorid, 254 mg (1.88 mmol) HOBt, 729 mg (3.8mmol) EDC und 1.033 ml (9.4 mmol) N-Methylmorpholin werden zugegeben. Das Reaktionsgemisch wird 3 Tage bei Raumtemperatur gerührt und dann mit Essigester versetzt. Die organische Phase wird 3x mit Wasser gewaschen, mit Natriumsulfat getrocknet, filtriert und das Lösungsmittel entfernt.

12.2 4-Benzylamino-N-[2-(3,4-difluor-phenyl)-ethyl]-3-nitro-benzamid wird unter Standardbedingungen hydriert und das Rohprodukt als 3,4-Diamino-N-[2-(3,4-difluor-phenyl)-ethyl]-benzamid Dihydrochlorid isoliert,

12.3 Eine Lösung von 56 mg (0.277 mmol) 2-Amino-5-(1-methyl-1H-pyrazol-4-yl)-pyridin-3-carbaldehyd und 102 mg (0.280 mmol) 3,4-Diamino-N-[2-(3,4-difluor-phenyl)-ethyl]-benzamid Dihydrochlorid in 5 ml DMF wird mit 279 μl (1.4 mmol) Natriumhydrogensulfit-Lösung 38-40% versetzt und die entstandene Suspension 14 h bei 50°C gerührt. Das Reaktionsgemisch wird auf RT abgekühlt, mit Wasser versetzt und 3x mit Dichlormethan/Isopropanol extrahiert. Die organischen Phasen werden 2x mit Wasser gewaschen und mit Natriumsulfat getrocknet, filtriert und das Lösungsmittel entfernt. Der Rückstand wird mittels präparativer RP-HPLC weiter aufgereinigt. Man erhält 2-[2-Amino-5-(1-methyl-1H-pyrazol-4-yl)-pyridin-3-yl]-3H-benzimidazol-5-carbonsäure-[2-(3,4-difluor-phenyl)-ethyl]-amid Trifluoroacetat als gelber Feststoff; APCI-MS $[M+H]^+$ 474.3;

$^1$H NMR (500 MHz, DMSO-$d_6$ +TFA-$d_1$) δ [ppm] 9.09 (d, *J* = 2.1, 1 H), 8.45 (d, J = 2.1, 1H), 8.23 (s, 2H), 8.00 (d, *J* = 0.5, 1H), 7.87 (dd, *J* = 8.5, 1.6, 1H), 7.79 (d, *J* = 8.4, 1 H), 7.36 - 7.28 (m, 2H), 7.14 - 7.08 (m, 1 H), 3.96 (s, 3H), 3.58 (t, *J* = 7.1, 2H), 2.92 (t, *J* = 7.1, 2H).

Beispiel 13

Herstellung von 2-[2-Amino-5-(1-methyl-1H-pyrazol-4-yl)-pyridin-3-yl]-1H-benzimidazol-4-carbonsäure-piperidin-4-ylamid ("A129")

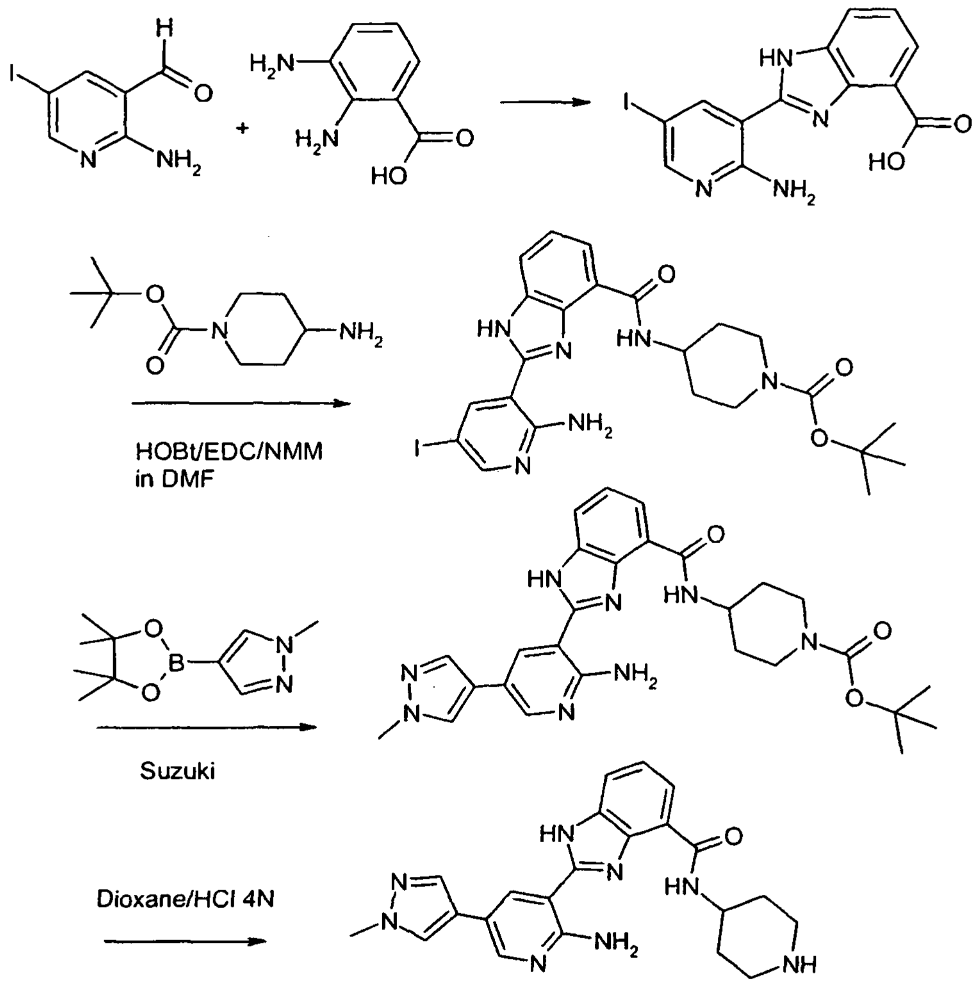


2-(2-Amino-5-iod-pyridin-3-yl)-1 H-benzimidazol-4-carbonsäure wird nach Weg 1 Route B hergestellt und die Amidkopplung wie in Beispiel 14 durchgeführt. Es schließt sich an die Suzuki-Kopplung wie nach Weg 1 Route B und die Boc-Abspaltung mit Dioxan/HCl 4N. Man erhält 2-[2-Amino-5-(1-methyl-1H-pyrazol-4-yl)-pyridin-3-yl]-1H-benzimidazol-4-carbonsäure-piperidin-4-ylamid Hydrochlorid; HPLC-MS $[M+H]^+$ 417.2;
$^1H$ NMR (500 MHz, DMSO-$d_6$ + TFA-$d_1$) $\delta$ [ppm] 9.24 (d, *J* = 2.0, 1H), 8.47 (d, *J* = 2.1, 1H), 8.28 (s, 1H), 8.06 (s, 1H), 7.94 (t, *J* = 7.6, 2H), 7.48 (t, *J* = 7.8, 1H), 4.28 - 4.20 (m, 1H), 3.96 (s, 3H), 3.45 - 3.37 (m, 2H), 3.14 (td, *J* = 12.5, 2.7, 2H), 2.17 (dd, *J*= 14.1, 3.4, 2H), 1.94 - 1.83 (m, 2H).

Beispiel 14

Herstellung von 5-[6-Amino-5-(6-methoxy-1H-benzimidazol-2-yl)-pyridin-3-yl]-thiophen-2-carbonsäure-(2-methoxyethyl)-amid ("A130")

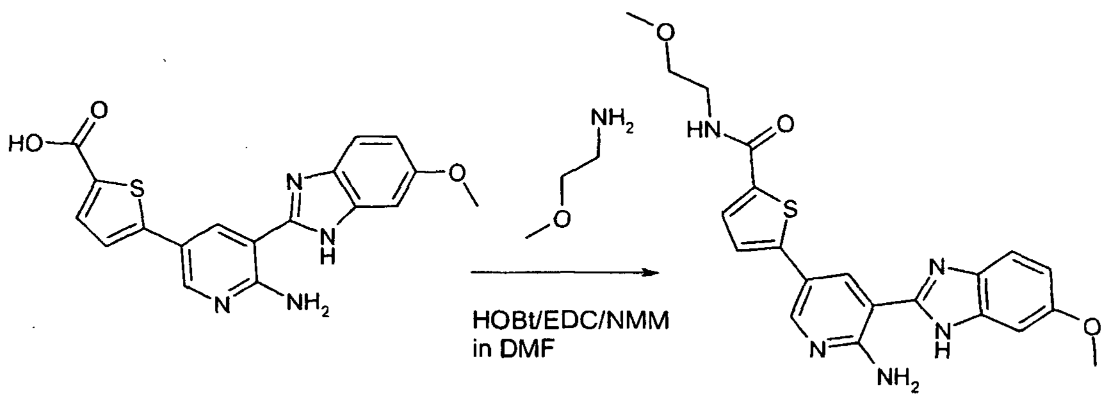


14.1 5-[6-Amino-5-(6-methoxy-1H-benzimidazol-2-yl)-pyridin-3-yl]-thiophee-2-carbonsäure wird analog Weg1 Route B hergestellt und als Rohprodukt weiter umgesetzt.

14.2 157 mg (0.111 mmol) 5-[6-Amino-5-(6-methoxy-1H-benzimidazol-2-yl)-pyridin-3-yl]-thiophen-2-carbonsäure werden in 0.5 ml DMF gelöst. 10 μl (0.114 mmol) 2-Methoxy-ethylamin, 14.9 mg (0.111 mmol) HOBt, 21.2 mg (0.111 mmol) EDC und 24.3 μl (0.221 mmol) N-Methylmorpholin werden zugegeben. Das Reaktionsgemisch wird 14 h bei Raumtemperatur gerührt, mit Wasser versetzt und mit Essigester extrahiert. Die organischen Phasen werden mit Natriumsulfat getrocknet, filtriert und eingedampft. Der Rückstand wird mittels RP-HPLC aufgereinigt und lyophilisiert. Man erhält: 5-[6-Amino-5-(6-methoxy-1H-benzimidazol-2-yl)-pyridin-3-yl]-thiophen-2-carbonsäure-(2-methoxy-ethyl)-amid; HPLC-MS $[M+H]^+$ 424.2.
$^1H$ NMR (400 MHz, DMSO-$d_6$ + TFA-$d_1$) δ [ppm] 9.00 (d, *J* = 2.1, 1 H), 8.60 (d, *J* = 2.0, 1 H), 7.90 (d, *J* = 3.9, 1 H), 7.66 (dd, *J* = 9.7, 6.4, 2H), 7.24 - 7.18 (m, 1 H), 7.07 - 6.99 (m, 1 H), 3.88 (s, 3H), 3.53 - 3.43 (m, 4H), 3.31 (s, 3H).

Analog erhält man die nachstehenden Verbindungen

| Nr. | Name und/oder Struktur | Analytik |
|---|---|---|
| "A131" | 5-[6-Amino-5-(6-methoxy-1H-benzimidazol-2-yl)-pyridin-3-yl]-thiophen-2-carbonsäure-(2-dimethylamino-ethyl)-amid 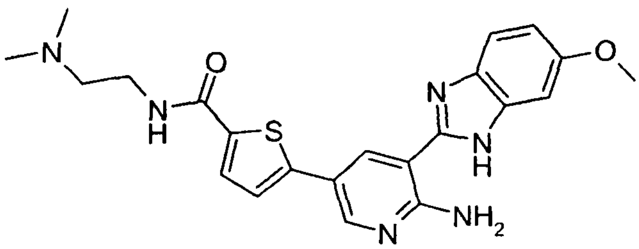 | HPLC-MS $[M+H]^+$ 437.2 |
| | | |
| "A132" | 5-[6-Amino-5-(6-methoxy-1H-benzimidazol-2-yl)-pyridin-3-yl]-thiophen-2-carbonsäure-piperidin-4-ylamid 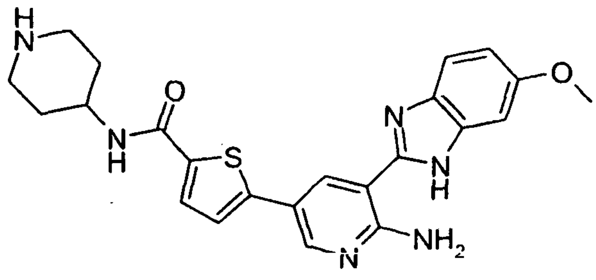 [letzte Stufe: Abspaltung der BOC-Schutzgruppe in HCl/Dioxan] | HPLC-MS $[M+H]^+$ 449.2 |
| "A133" | 5-[6-Amino-5-(6-methoxy-1H-benzimidazol-2-yl)-pyridin-3-yl]-thiophen-2-carbonsäure-(1-methyl-piperidin-4-yl)-amid | HPLC-MS $[M+H]^+$ 463.2 |

(fortgesetzt)

| Nr. | Name und/oder Struktur | Analytik |
|---|---|---|
| | 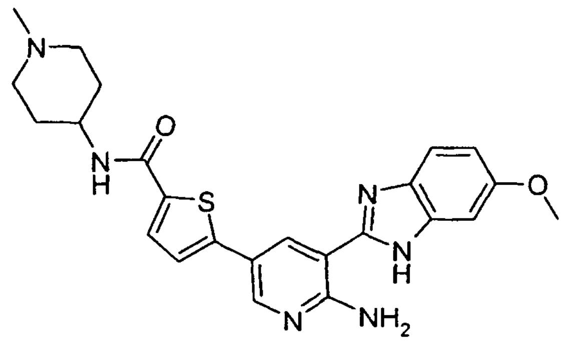 | |
| $^1$H NMR (500 MHz, DMSO-$d_6$ + TFA-$d_1$) δ [ppm] 9.02 (d, *J* = 2.2, 1H), 8.60 (d, *J* = 2.1, 1H), 7.92 (d, *J* = 3.9, 1H), 7.67 (dd, *J* = 6.3, 4.4, 2H), 7.22 (d, *J* = 2.2, 1H), 7.04 (dd, *J* = 8.9, 2.4, 1H), 4.11 - 4.00 (m, 1H), 3.89 (s, 3H), 3.53 (d, *J* = 12.6, 2H), 3.16 (td, *J* = 13.1, 2.2, 2H), 2.83 (s, 3H), 2.10 (dd, *J* = 13.6 2.7, 2H), 1.91 - 1.76 (m, 2H) | | |
| "A134" | 5-[6-Amino-5-(6-methoxy-1H-benzimidazol-2-yl)-pyridin-3-yl]-thiophen-2-carbonsäure-(pyridin-4-ylmethyl)-amid 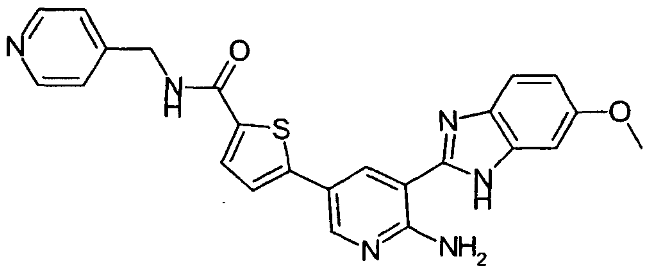 | HPLC-MS [M+H]$^+$ 457.2 |
| $^1$H NMR (500 MHz, DMSO-$d_6$ + TFA-$d_1$) δ [ppm] 9.02 (d, *J* = 2.1, 1H), 8.93 (*d, J* = 6.8, 2H), 8.62 (d, *J* = 2.1, 1H), 8.06 (d, *J* = 6.7, 2H), 7.99 (d, *J* = 3.9, 1H), 7.71 (d, *J* = 3.9, 1H), 7.68 (d, *J* = 8.8, 1H), 7.22 (d, *J* = 2.3, 1H), 7.04 (dd, *J* = 8.9, 2.4, 1H), 4.82 (s, 2H), 3.88 (s, 3H) | | |

Beispiel 15

Herstellung von N-(2-{2-[2-Amino-5-(1-methyl-1H-pyrazol-4-yl)-pyridin-3-yl]-3H-benzimidazol-5-yl}-ethyl)-3,4-difluorbenzamid ("A135")

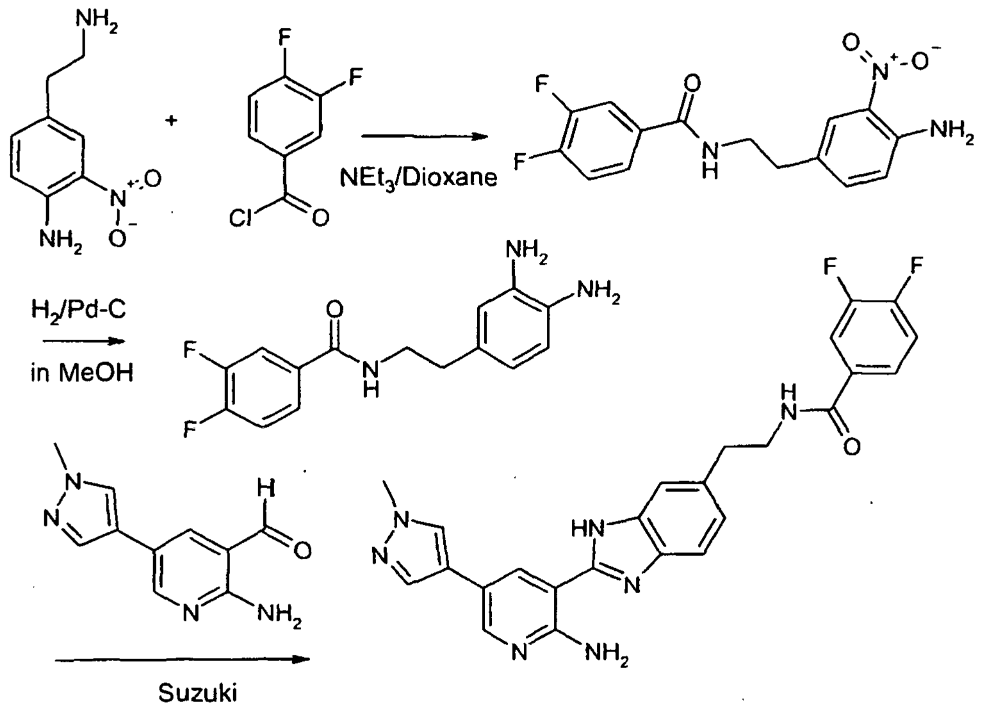


480 mg (2.649 mmol) 4-(2-Amino-ethyl)-2-nitro-phenylamin werden in 30 ml Dioxan gelöst und 1.102 ml (7.950 mmol) Triethylamin zugegeben. Dann werden 0.334 ml (2.650 mmol) 3,4-Difluor-benzoylchloride zugetropft und 30 min bei Raumtemperatur gerührt. Das Reaktionsgemisch wird mit Essigester versetzt und 3x mit Wasser gewaschen, mit Natriumsulfat getrocknet, filtriert und bis zum Rückstand abdestilliert. Man erhält N-[2-(4-Amino-3-nitro-phenyl)-ethyl]-3,4-difluor-benzamid; EI-MS $[M]^+$ 321, das weiter unter Standardbedingungen hydriert wird.
Man erhält N-[2-(3,4-Diamino-phenyl)-ethyl]-3,4-difluor-benzamid; HPLC-MS $[M+H]^+$ 292.2.
Die Cyclisierung zum Benzimidazol erfolgt nach den Bedingungen Weg 1 Route A.
Man erhält N-(2-{2-[2-Amino-5-(1-methyl-1H-pyrazol-4-yl)-pyridin-3-yl]-3H-benzimidazol-5-yl}-ethyl)-3,4-difluor-benzamid Trifluoroacetat; HPLC-MS $[M+H]^+$ 474.3;
$^1$H NMR (500 MHz, DMSO-$d_6$ + TFA-$d_1$) $\delta$ [ppm] 9.02 (d, $J$ = 2.0, 1H), 8.43 (d, $J$ = 2.0, 1 H), 8.21 (s, 1H), 7.99 (s, 1H), 7.92 - 7.83 (m, 1H), 7.78 - 7.72 (m, 1H), 7.70 (d, $J$ = 8.3, 1H), 7.63 (s, 1H), 7.50 (dt, $J$ = 10.3, 8.4, 1H), 7.31 (dd, $J$ = 8.4, 1.2, 1H), 3.95 (s, 3H), 3.62 (t, $J$ = 7.2, 2H), 3.06 (t, $J$ = 7.2, 2H).

Beispiel 16

Herstellung von 2-{2-[2-Amino-5-(1-methyl-1H-pyrazol-4-yl)-pyridin-3-yl]-3H-benzimidazol-4-yl}-ethanol ("A136", (Vgl.-Bsp.)

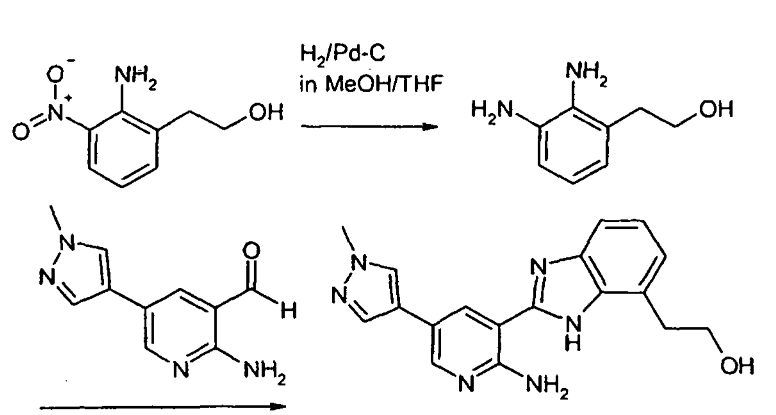

2-(2-Amino-3-nitro-phenyl)-ethanol wird nach Standardbedingungen zu 2-(2,3-Diamino-phenyl)-ethanol hydriert; HPLC-MS $[M+H]^+$ 153.2.

Dieses wird zum Benzimidazol nach Weg 1 Route A cyclisiert, man erhält 2-{2-[2-Amino-5-(1-methyl-1H-pyrazol-4-yl)-pyridin-3-yl]-3H-benzimidazol-4-yl}-ethanol; HPLC-MS $[M+H]^+$ 335.2;

$^1$H NMR (500 MHz, DMSO-$d_6$ + TFA-$d_1$ $\delta$ [ppm] 9.06 (d, $J$ = 2.0, 1H), 8.43 (d, $J$ = 2.1, 1H), 8.22 (s, 1 H), 7.99 (s, 1 H), 7.58 (d, $J$ = 7.5, 1 H), 7.33 - 7.27 (m, 1 H), 7.22 (d, $J$ = 7.2, 1H), 3.94 (s, 3H), 3.82 (t, $J$ = 7.1, 2H), 3.20 (t, $J$ = 7.1, 2H).

Beispiel 17

Herstellung von 3-[5-(4-Methyl-piperazin-1-sulfonyl)-1H-benzimidazol-2-yl]-5-(1-methyl-1H-pyrazol-4-yl)-pyridin-2-ylamin ("A137")

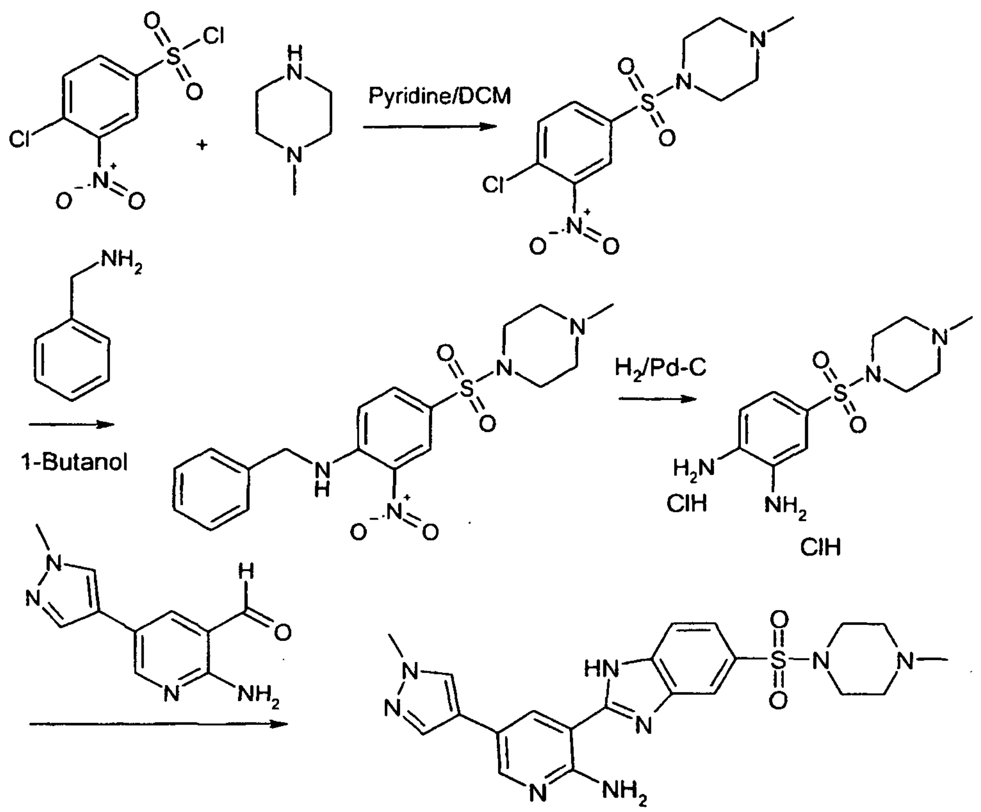


17.1 2.5 g (9.763 mmol) 4-Chlor-3-nitro-benzolsulfonylchlorid werden in 100 ml DCM gelöst. 1.574 ml (19.5 mmol) Pyridin und 1.086 ml (9.760 mmol) N-Methylpiperazin werden zugegeben und 14 h bei Raumtemperatur gerührt. Das Reaktionsgemisch wird 3x mit Wasser gewaschen, die organische Phase mit Natriumsulfat getrocknet, filtriert und das Lösungsmittel entfernt. Man erhält 1-(4-Chlor-3-nitro-benzolsulfonyl)-4-methyl-piperazin; EI-MS $[M]^+$ 319.

17.2 2.8 g (8.756 mmol) 1-(4-Chlor-3-nitro-benzolsulfonyl)-4-methyl-piperazin werden in 10 ml 1-Butanol gelöst und mit 1.913 ml (17.5 mmol) Benzylamin versetzt. Das Reaktionsgemisch wird 14 h unter Rückfluß erhitzt. Nach Abkühlen wird es mit MTBE versetzt, der entstandene Niederschlag wird abgesaugt und mit MTBE gewaschen und am Vakuum getrocknet. Man erhält Benzyl-[4-(4-methyl-piperazin-1-sulfonyl)-2-nitrophenyl]-amin; HPLC-MS $[M+H]^+$ 391.2.

17.3 Benzyl-[4-(4-methyl-piperazin-1-sulfonyl)-2-nitro-phenyl]-amin wird unter Standardbedingungen zu 4-(4-Methyl-piperazin-1-sulfonyl)-benzol-1,2-diamin hydriert und als Dihydrochlorid isoliert; HPLC-MS $[M+H]^+$ 271.2.

17.4 Man cyclisiert zum Benzimidazol nach Weg 1 Route A und erhält 3-[5-(4-Methyl-piperazin-1-sulfonyl)-1H-benzimidazol-2-yl]-5-(1-methyl-1H-pyrazol-4-yl)-pyridin-2-ylamin; ESI-MS $[M+H]^+$; 453.2 $[2M+H]^+$ 905.0;

$^1$H NMR (500 MHz, DMSO-$d_6$ + TFA-$d_1$) $\delta$ [ppm] 9.16 (d, $J$ = 2.1, 1H), 8.50 (d, $J$ = 1.8, 1 H), 8.25 (s, 1 H), 8.23 (d, $J$ = 1.5, 1 H), 8.02 (t, $J$ = 4.2, 2H), 7.78 (dd, $J$ = 8.5, 1.6, 1 H), 3.96 (s, 3H), 3.89 (d, $J$ = 13.2, 2H), 3.53 (d, $J$ = 12.3, 2H), 3.24 (td, $J$ = 12.0, 1.7, 2H), 2.83 (s, 3H), 2.63 (t, $J$ = 12.0, 2H).

Analog werden die nachstehenden Verbindungen erhalten

2-[2-Amino-5-(1-methyl-1H-pyrazol-4-yl)-pyridin-3-yl]-3H-benzimidazol-5-sulfonsäure-dimethylamid ("A138", Vgl.-Bsp.)

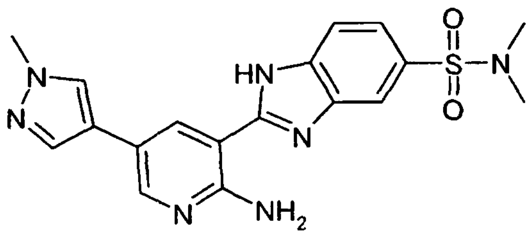

ESI-MS $[M+H]^+$ 398.2;
$^1H$ NMR (500 MHz, DMSO-$d_6$ + TFA-$d_1$) $\delta$ [ppm] 9.13 (d, *J* = 2.0, 1H), 8.48 (d, *J* = 2.0, 1 H), 8.24 (s, 1 H), 8.15 (d, *J* = 1.2, 1 H), 8.01 (s, 1 H), 7.97 (d, *J* = 8.5, 1 H), 7.74 (dd, *J* = 8.5, 1.6, 1 H), 3.96 (s, 3H), 2.67 (s, 6H);

2-[2-Amino-5-(1-methyl-1 H-pyrazol-4-yl)-pyridin-3-yl]-1 H-benzimidazol-5-sulfonsäure-ethylamid ("A139", Vgl.-Bsp.)

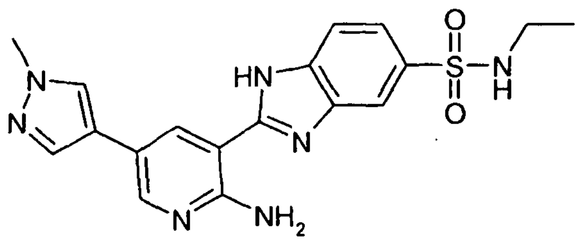

EI-MS $[M]^+$ 397.1;
$^1H$ NMR (500 MHz, DMSO-$d_6$ + TFA-$d_1$) $\delta$ [ppm] 9.08 (d, *J* = 1.9, 1H), 8.43 (d, *J* = 2.0, 1 H), 8.19 (s, 1 H), 8.16 (d, *J* = 1.4, 1 H), 7.97 (d, *J* = 2.3, 1 H), 7.89 (d, *J* = 8.5, 1H), 7.78 (dd, *J* = 8.5, 1.5, 1H), 3.92 (s, 3H), 2.80 (q, *J* = 7.2, 2H), 0.97 (t, *J* = 7.2, 3H).

Beispiel 18

Herstellung von 2-[2-Amino-5-(1-methyl-1H-pyrazol-4-yl)-pyridin-3-yl]-5-fluor-3H-benzimidazol-4-carbonsäure-(pyridin-4-ylmethyl)-amid ("A140")

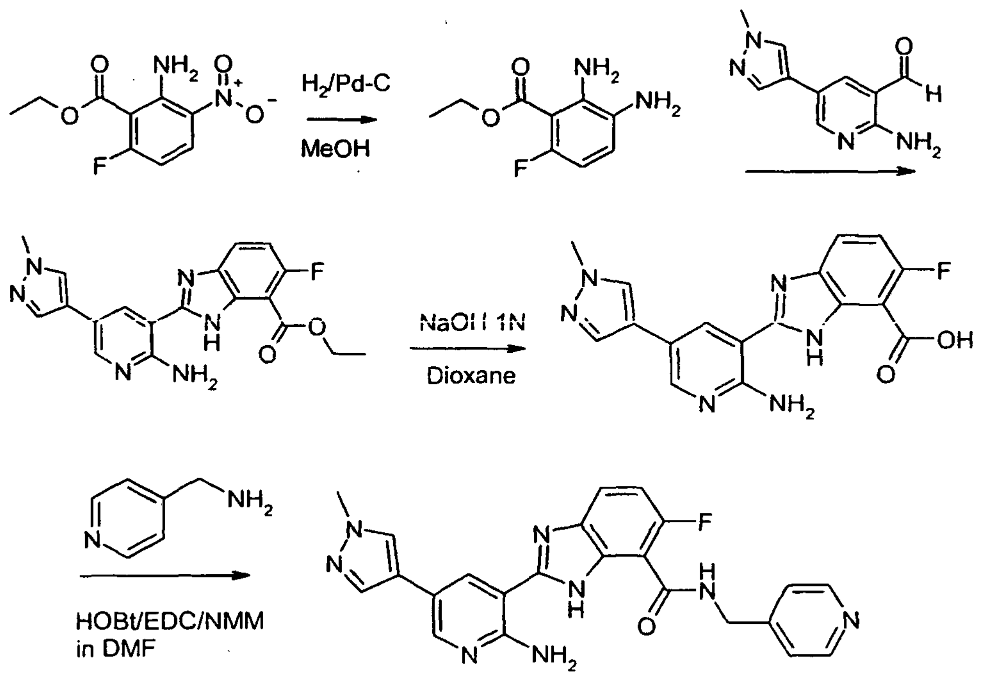


18.1 2-Amino-6-fluor-3-nitro-benzoesäure-ethylester wird unter Standardbedingungen zu 2,3-Diamino-6-fluor-benzoesäure-ethylester hydriert; HPLC-MS $[M+H]^+$ 199.2.

18.2 Man cyclisiert zum Benzimidazol nach Weg 1 Route A und erhält 2-[2-Amino-5-(1-methyl-1 H-pyrazol-4-yl)-pyridin-3-yl]-5-fluor-3H-benzimidazol-4-carbonsäure-ethylester ("A141", Vgl.-Bsp.); HPLC-MS $[M+H]^+$ 381.2;
$^1$H NMR (500 MHz, DMSO-$d_6$ + TFA-$d_1$) δ [ppm] 9.17 (d, *J* = 2.0, 1H), 8.36 (d, *J* = 2.0, 1 H), 8.18 (s, 1 H), 7.98 (s, 1 H), 7.94 (dd, *J* = 8.9, 4.3, 1 H), 7.24 (dd, *J* = 11.3, 8.9, 1H), 4.45 (q, *J* = 7.1, 2H), 3.90 (s, 3H), 1.37 (t, *J* = 7.1, 3H).

18.3 700 mg (1.840 mmol) "A141" wird in Dioxan gelöst und 7 ml (7 mmol) NaOH 1N zugegeben. Das Reaktionsgemisch wird 20 Stunden bei 60°C gerührt. Der entstehende Feststoff wird abgesaugt, mit Wasser und Aceton gewaschen und weiter mit RP-HPLC aufgereinigt. Man erhält 2-[2-Amino-5-(1-methyl-1H-pyrazol-4-yl)-pyridin-3-yl]-5-fluor-3H-benzimidazol-4-carbonsäure ("A142", Vgl.-Bsp.); HPLC-MS $[M+H]^+$ 353.2;
$^1$H NMR (500 MHz, DMSO-$d_6$ + TFA-$d_1$) δ [ppm] 9.26 (d, *J* = 2.0, 1H), 8.43 (d, *J* = 2.0, 1 H), 8.26 (s, 1 H), 8.04 (s, 1 H), 8.00 (dd, *J* = 8.8, 4.3, 1 H), 7.30 (dd, *J* = 11.3, 8.9, 1 H), 3.95 (s, 3H).

18.4 35 mg (0.099 mmol) "A142" werden in 3 ml DMF gelöst. 11.05 μl (0.109 mmol) 4-Picolylamin, 14.8 mg (0.109 mmol) HOBt, 20.9 mg (0.109 mmol) EDC und 12 μl (0.109 mmol) N-Methylmorpholin werden zugegeben. Das Reaktionsgemisch wird 6 Stunden bei Raumtemperatur gerührt und mit gesättigter $NaHCO_3$-Lösung versetzt. Der entstehende Niederschlag wird mit Wasser gewaschen und getrocknet. Man erhält: 2-[2-Amino-5-(1-methyl-1 H-pyrazol-4-yl)-pyridin-3-yl]-5-fluor-3H-benzimidazol-4-carbonsäure-(pyridin-4-ylmethyl)-amid; HPLC-MS $[M+H]^+$ 443.2;
$^1$H NMR (500 MHz, DMSO-$d_6$ + TFA-$d_1$) δ [ppm] 9.21 (d, *J* = 2.0, 1H), 9.01 - 8.94 (m, 2H), 8.46 (d, *J* = 2.1, 1 H), 8.24 (s, 1 H), 8.14 (d, *J* = 6.1, 2H), 8.03 (s, 1H), 7.94 (dd, *J* = 8.8, 4.5, 1H), 7.36 (dd, *J* = 11.0, 8.9, 1H), 4.95 (s, 2H), 3.95 (s, 3H).

Beispiel 19

Herstellung von 1-{2-[2-Amino-5-(1-methyl-1H-pyrazol-4-yl)-pyridin-3-yl]-1H-benzimidazol-5-yl}-1-phenyl-ethanol ("A143")

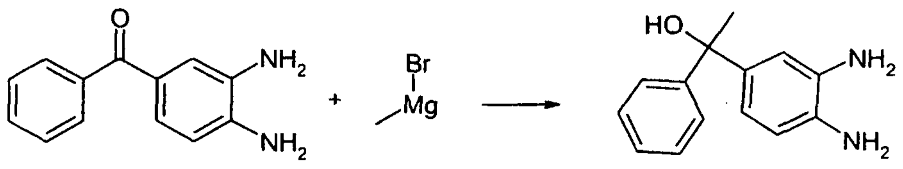

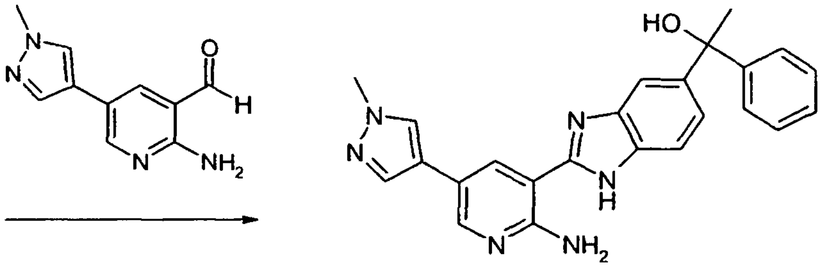

15 ml (45 mmol) Methylmagnesium-bromid-Lösung (3M in Diethylether) wird mit 13 ml Diethylether verdünnt. Eine Lösung von 2 g (9.423 mmol) 3,4-diaminophenon in 13 ml Diethylether wird zur Grignard-Lösung bei 0°C getropft. Das Reaktionsgemisch wird bei 0°C mit 15 ml gesättigter NaCl-Lösung gequencht und mit Diethylether versetzt. Die organische Phase wird abgetrennt und die wässrige Phase 2x mit Essigester extrahiert. Die vereinigten organischen Phasen werden über Natriumsulfat getrocknet, filtriert und das Lösungsmittel entfernt. Der Rückstand wird chromatographiert. Man erhält 1-(3,4-Diamino-phenyl)-1-phenyl-ethanol; HPLC-MS $[M+H]^+$ 229.2.
Man cyclisiert zum Benzimidazol nach Weg 1 Route A und erhält 1-{2-[2-Amino-5-(1-methyl-1H-pyrazol-4-yl)-pyridin-3-yl]-1H-benzimidazol-5-yl}-1-phenyl-ethanol; HPLC-MS $[M+H]^+$ 411.2;
$^1$H NMR (500 MHz, DMSO-$d_6$ + TFA-$d_1$) $\delta$ [ppm] 8.98 (d, $J$ = 2.1, 1H), 8.43 (d, $J$ = 2.1, 1H), 8.20 (s, 1 H), 7.98 (s, 1 H), 7.82 (d, $J$ = 1.2, 1 H), 7.67 (d, $J$ = 8.6, 1 H), 7.52 (dd, $J$ = 8.3, 1.1, 2H), 7.46 (dd, $J$ = 8.6, 1.5, 1 H), 7.32 (t, $J$ = 7.7, 2H), 7.21 (t, $J$ = 7.3, 1H), 3.95 (s, 3H), 1.97 (s, 3H).

Beispiel 20

Herstellung von 3-(6-Methoxy-1 H-benzimidazol-2-yl)-5-thiophen-3-yl-pyridin-2-ylamin ("A144", Vgl.-Bsp.)

Alternativ zu den Standardbedingungen der Suzuki-Kopplung in Weg 1 Route A, kann man die Suzuki-Kopplung auch mit Trifluoroborat-Edukten durchführen.

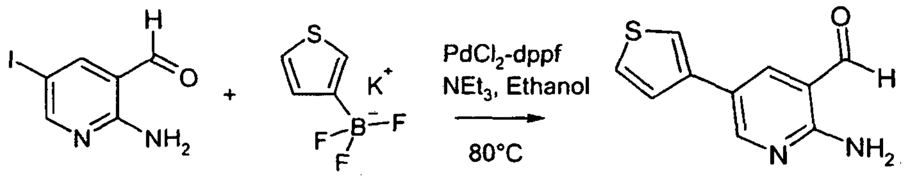


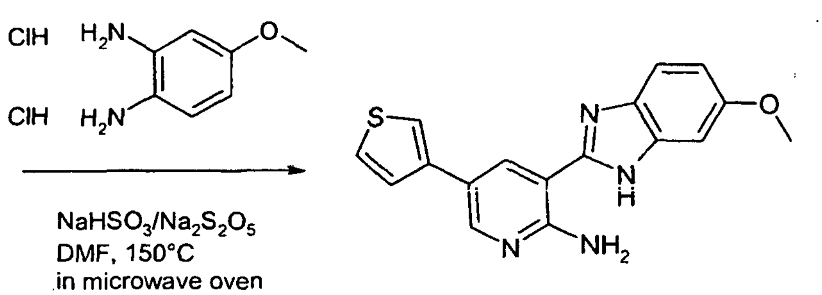


20.1 Suzuki Kopplung:

In einem mit Stickstoff befüllten Reaktionsgefäß werden 150 mg (0.605 mmol) 2-Amino-5-iod-pyridin-3-carbaldehyd und 115 mg (0.605 mmol) Kalium-3-thiophen-trifluoroborat in 4 ml Ethanol suspendiert und mit 251 µl (1.814 mmol) Triethylamin und 22.1 mg (0.030 mmol) [1,1'-Bis(diphenyl-phosphino)ferrocen] dichloropalladium(II) versetzt.

Das Reaktionsgemisch wird 14 h bei 80°C unter Stickstoffschutzatmosphäre gerührt. Das Reaktionsgemisch wird auf Raumtemperatur gekühlt und mit Wasser / Essigester verdünnt und abfiltriert. Die organische Phase wird getrennt und die wässrige Phase noch 2x mit Essigester extrahiert. Die vereinigten organischen Phasen werden über Natriumsulfat getrocknet, filtriert und das Lösungsmittel entfernt. Man erhält 2-Amino-5-thiophen-3-yl-pyridin-3-carbaldehyd; HPLC-MS $[M+H]^+$ 205.2.

20.2 Cyclisierung zum Benzimidazol:

Eine Lösung von 81.1 mg (0.250 mmol) 2-Amino-5-thiophen-3-yl-pyridin-3-carbaldehyd und 64.6 mg (0.300 mmol) 4-Methoxy-o-phenylendiamin Dihydrochlorid in 1.5 ml DMF wird mit 147.9 μl (0.750 mmol) Natriumhydrogensulfit-Lösung 38-40% versetzt und 30 min bei 150°C mit Mikrowellen im Biotage SmithSynthesizer bestrahlt. Das Reaktionsgemisch wird auf Raumtemperatur gekühlt, mit Wasser versetzt und 3x mit Essigester extrahiert. Die vereinigten organischen Phasen werden über Natriumsulfat getrocknet, filtriert und das Lösungsmittel entfernt. Der Rückstand wird mittels RP-HPLC aufgereinigt: 3-(6-Methoxy-1H-benzimidazol-2-yl)-5-thiophen-3-yl-pyridin-2-ylamin hydrochloride; HPLC-MS $[M+H]^+$ 323.2;
$^1H$ NMR (500 MHz, DMSO-$d_6$ + TFA-$d_1$) δ [ppm] 9.09 (d, $J$ = 2.0, 1 H), 8.54 (d, $J$ = 2.0, 1 H), 7.99 (d, $J$ = 1.6, 1 H), 7.73 (dd, $J$ = 5.0, 3.0, 1 H), 7.65 (dd, $J$ = 9.8, 4.8, 2H), 7.19 (d, $J$ = 2.2, 1 H), 7.00 (dd, $J$ = 8.8, 2.3, 1 H), 3.84 (s, 3H).

<u>Beispiel 21</u>

Herstellung von 5-Imidazol-1-yl-3-(5-methoxy-1 H-benzimidazol-2-yl)-pyridin-2-ylamin ("A145", Vgl.-Bsp.)

Alternativ zu den Standardbedingungen der Suzuki-Kopplung in Weg 1 Route A, kann man auch die Synthesesequenz mit einer Kupferkatalysierten Kopplung starten.

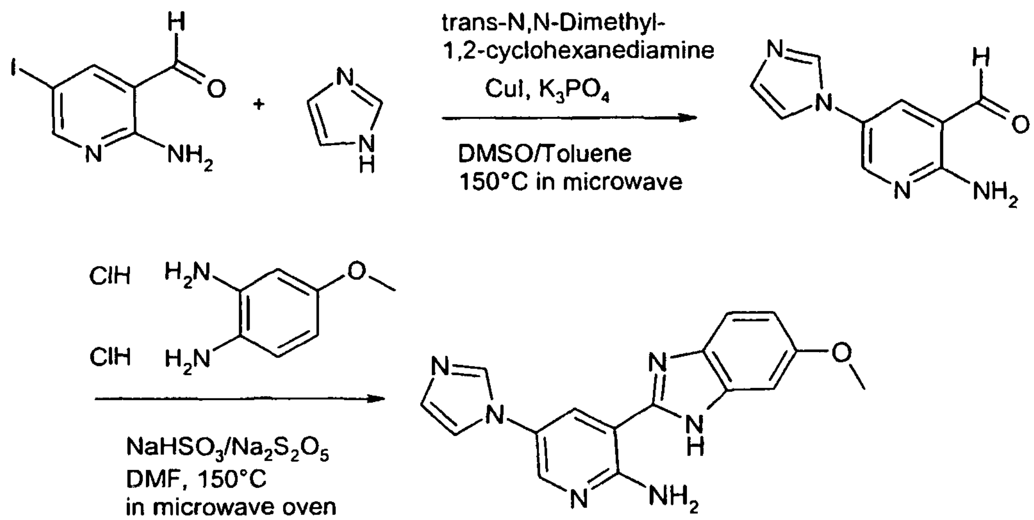


In einem mit Stickstoff befüllten Mikrowellengefäß werden 300 mg (1.210 mmol) 2-Amino-5-iod-pyridin-3-carbaldehyd und 24mg (3.629 mmol) Imidazol in ml DMSO gelöst. Dazu wird 51.6 mg (0.363 mmol) trans-N,N-Dimethyl-1,2-cyclohexanediamine, 69.1 mg (0.363 mmol) Kupferiodid, 523.8 mg (2.468 mmol) $K_3PO_4$ und 5 ml Toluol hinzugefügt. Das Reaktionsgemisch wird 2 Stunden bei 150°C mit Mikrowellen bestrahlt. Das Reaktionsgemisch wird auf Raumtemperatur gekühlt, abfiltriert und das Lösungsmittel entfernt. Der Rückstand wird mit Wasser/Essigester versetzt. Die organische Phase wird getrennt und die wässrige Phase noch 2x mit Essigester extrahiert. Die vereinigten organischen Phasen werden über Natriumsulfat getrocknet, filtriert und das Lösungsmittel entfernt. Man erhält 2-Amino-5-imidazol-1-yl-pyridin-3-carbaldehyd; HPLC-MS $[M+H]^+$ 189.2.
Die Cyclisierung zum Benzimidazol wird analog Beispiel 20 durchgeführt, man erhält 5-Imidazol-1-yl-3-(5-methoxy-1H-benzimidazol-2-yl)-pyridin-2-ylamin; HPLC-MS $[M+H]^+$ 307.2;
$^1H$ NMR (500 MHz, DMSO-$d_6$ + TFA-$d_1$) ö [ppm] 9.61 (t, $J$ = 1.4, 1 H), 8.66 (dd, $J$ = 7.9, 2.6, 2H), 8.15 (t, $J$ = 1.7, 1H), 7.98 - 7.93 (m, 1H), 7.68 (d, $J$ = 8.9, 1 H), 7.22 (d, $J$ = 2.3, 1 H), 7.06 (dd, $J$ = 8.9, 2.4, 1 H), 3.86 (s, 3H).

Beispiel 22

Herstellung von 4-{2-[2-Amino-5-(1-methyl-1H-pyrazol-4-yl)-pyridin-3-yl]-3H-benzimidazol-5-yl}-morpholin-3-on ("A146")

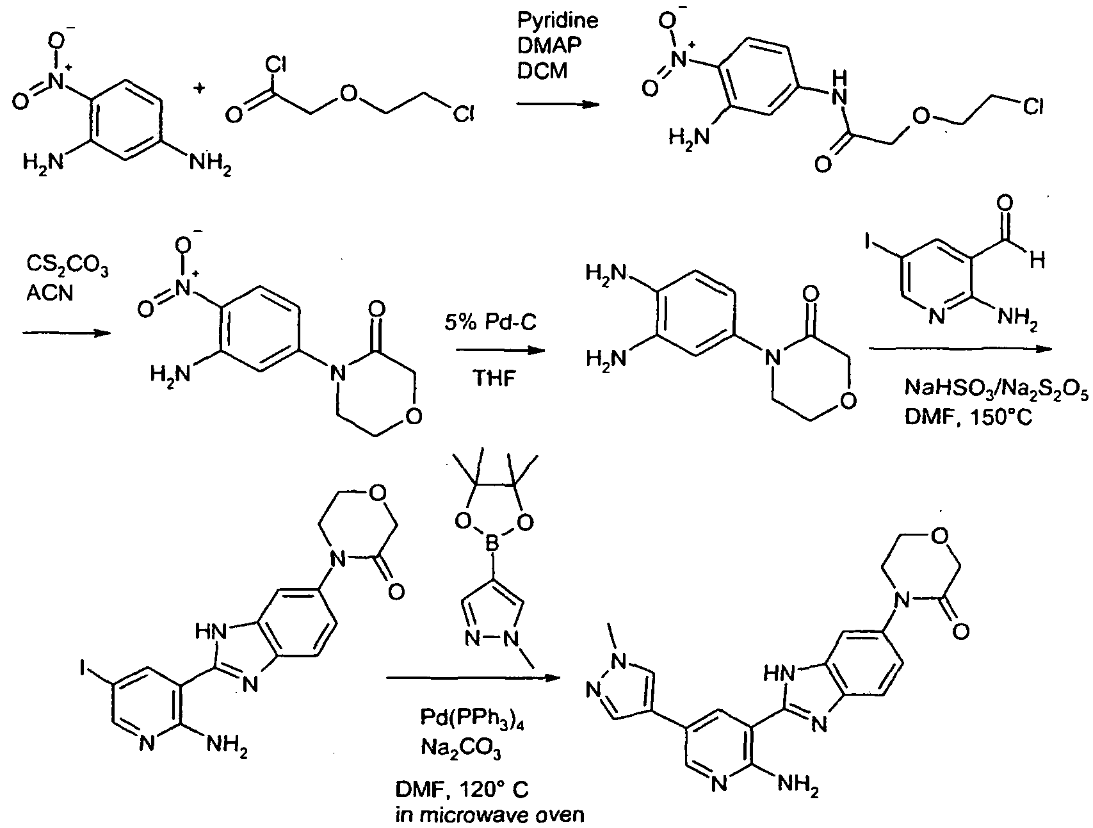


22.1 7.963 g (0.052 mol) 1,3-Diamino-4-nitrophenyl werden in 250 ml DCM suspendiert und bei Raumtemperatur unter Stickstoff werden 8.394 ml (0.104 mol) Pyridin und 1.271 g (0.01 mol) DMAP zugegeben. Anschließend werden 16.4 g (0.104 mol) (2-Chlor-ethoxy)-acetylchlorid langsam zu der gelben Suspension zugetropft. Da die Reaktion exotherm verläuft, wird durch Kühlen mit einem Eis-Wasserbad die Temperatur bei 20°C gehalten. Es wird 18h bei Raumtemperatur gerührt. Der Ansatz wird mit ca. 500 ml Dichlormethan verdünnt und anschließend mit 750 ml Wasser versetzt. Die organische Phase wird abgetrennt und die wässrige Phase wird noch 2x mit je 500 ml Essigester extrahiert. Die gesamten organischen Phasen werden mit wasserfreiem Natriumsulfat getrocknet, filtriert und das Lösungsmittel wird am Rotavapor bis auf ca. 200 ml abgezogen. Die beim Einrotieren entstehenden Kristalle werden abgetrennt und getrocknet. Man erhält N-(3-Amino-4-nitro-phenyl)-2-(2-chlor-ethoxy)-acetamid, HPLC-MS $[M+H]^+$ 274.1.

22.2 5.2 g (0.016 mol) N-(3-Amino-4-nitro-phenyl)-2-(2-chlor-ethoxy)-acetamid werden in 250 ml Acetonitril suspendiert und mit 6.6 g (0.02 mol) Cäsiumcarbonat versetzt. Es wird 18 h bei Raumtemperatur gerührt. Der Ansatz wird in ca. 250 ml Wasser gegossen und 3x mit je 200 ml Essigester extrahiert. Die gesamten organischen Phasen werden mit wasserfreiem Natriumsulfat getrocknet, filtriert und das Lösungsmittel wird am Rotavapor bis auf ca. 150 ml Restvolumen abgezogen. Die beim Einrotieren ausgefallenen Kristalle werden abgetrennt und getrocknet. Man erhält 4-(3-Amino-4-nitro-phenyl)-morpholin-3-on, HPLC-MS $[M+H]^+$ 238.1.

22.3 4-(3-Amino-4-nitro-phenyl)-morpholin-3-one wird unter Standardbedingungen hydriert. Man erhält 4-(3,4-Diamino-phenyl)-morpholin-3-on; HPLC-MS $[M+H]^+$ 208.1.

22.4 320 mg (1.544 mmol) 4-(3,4-Diamino-phenyl)-morpholin-3-on werden in 4 ml DMF gelöst und bei Raumtemperatur mit 382.99 mg (1.544 mmol) 2-Amino-5-iod-pyridin-3-carbaldehyd und 0.913 ml (4.633 mmol) Natriumhydrogensulfit-Lösung 38-40% versetzt. Es wird 18 Stunden bei 150°C gerührt. Der Ansatz wird auf Raumtemperatur abgekühlt, in 20 ml Wasser gegossen, der hierbei ausgefallene Niederschlag wird abgetrennt und gut mit ca. 10 ml Wasser gewaschen. Der Niederschlag wird mit ca. 25 ml Acetonitril verrieben und die nicht gelösten Kristalle werden abgetrennt und getrocknet. Man erhält 4-[2-(2-Amino-5-iod-pyridin-3-yl)-3H-benzimidazol-5-yl]-morpholin-3-on; HPLC-MS $[M+H]^+$ 436.0.

22.5 Die Suzuki-Kopplung wird analog zu Route1 Weg B durchgeführt; man erhält 4-{2-[2-Amino-5-(1-methyl-1H-pyrazol-4-yl)-pyridin-3-yl]-3H-benzimidazol-5-yl}-morpholin-3-on; HPLC-MS $[M+H]^+$ 390.2; $^1H$ NMR (500 MHz, DMSO-$d_6$ + TFA-$d_1$) $\delta$ [ppm] 9.05 (d, $J$ = 2.1, 1 H), 8.40 (d, $J$ = 2.1, 1H), 8.19 (s, 1 H), 7.97 (s, 1 H), 7.75 (dd, $J$ = 5.2, 3.2, 2H), 7.35 (dd, $J$ = 8.7, 1.9, 1 H), 4.24 (s, 2H), 4.04 - 3.99 (m, 2H), 3.92 (s, 3H), 3.84 - 3.78 (m, 2H).

Beispiel 23

Herstellung von 3-{2-[2-Amino-5-(1-methyl-1H-pyrazol-4-yl)-pyridin-3-yl]-1H-benzimidazol-5-yl}-[1,3]oxazinan-2-on ("A147")

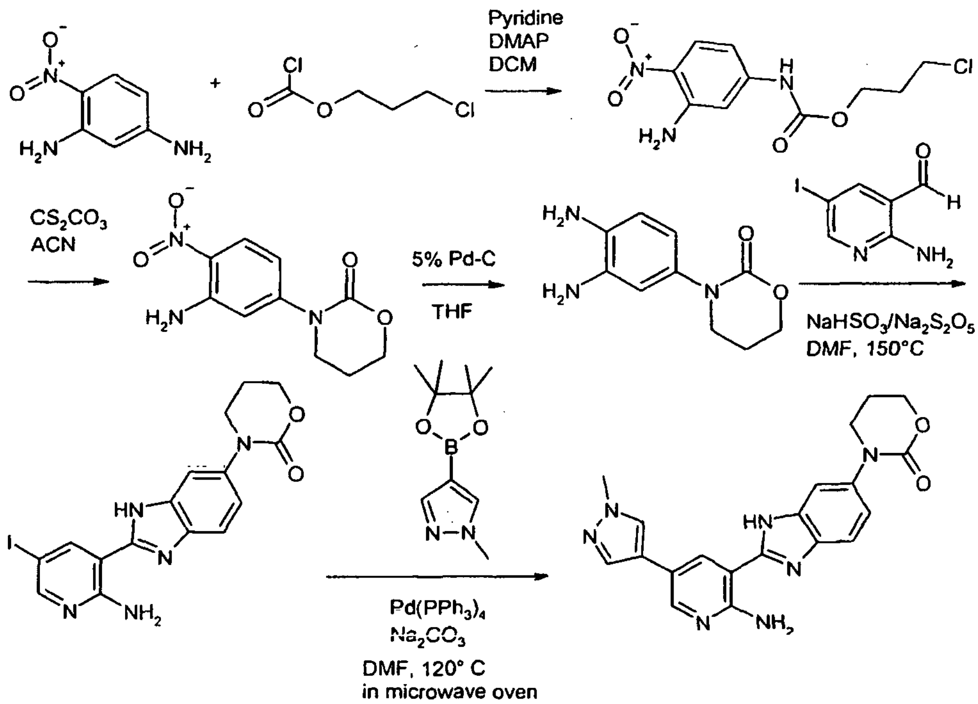


Die Synthesesequenz wird analog Beispiel 22 durchgeführt. Man erhält 3-{2-[2-Amino-5-(1-methyl-1H-pyrazol-4-yl)-pyridin-3-yl]-1H-benzimidazol-5-yl}-[1,3]oxazinan-2-on; HPLC-MS $[M+H]^+$ 390.2;
$^1H$ NMR (500 MHz, DMSO-$d_6$ + TFA-$d_1$) $\delta$ [ppm] 9.02 (d, $J$ = 2.0, 1 H), 8.39 (d, $J$ = 2.0, 1H), 8.17 (s, 1 H), 7.96 (s, 1 H), 7.72 (d, $J$ = 6.2, 1 H), 7.71 (s, 1 H), 7.33 (dd, $J$ = 8.8, 1.8, 1 H), 4.40 - 4.34 (m, 2H), 3.91 (s, 3H), 3.72 (t, $J$ = 6.0, 2H), 2.18 - 2.11 (m, 2H).

Beispiel 24

Analog Weg 1 Route A erhält man nachstehende Verbindungen

| Nr. | Name und/oder Struktur | Analytik |
|---|---|---|
| "A148" (Vgl.-Bsp.) | 3-(5-Fluor-6-methoxy-1H-benzimidazol-2-yl)-5-[1-(2-morpholin-4-yl-ethyl)-1H-pyrazol-4-yl]-pyridin-2-ylamin 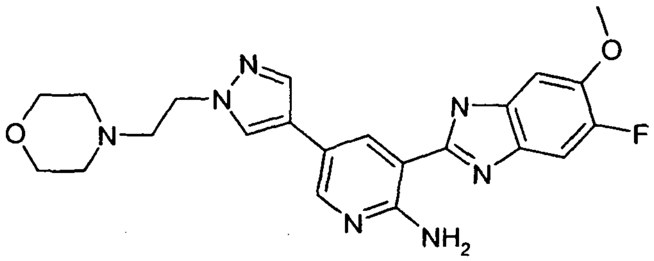 | MW 437.48 HPLC-MS $[M+H]^+$ 438.2 |
| $^1H$ NMR (500 MHz, DMSO-$d_6$ + TFA-$d_1$) δ 9.07 (d, J = 2.0, 1H), 8.43 (d, J = 2.0, 1H), 8.38 (s, 1H), 8.14 (s, 1H), 7.62 (d, J = 11.0, 1H), 7.41 (d, J = 7.7, 1H), 4.68 (t, J = 6.5, 2H), 4.08 - 3.98 (m, 2H), 3.96 (s, 3H), 3.74 (t, J = 6.5, 4H), 3.58 - 3.45 (m, 2H), 3.30 - 3.14 (m, 2H) | | |
| "A149" | 1-{2-[2-Amino-5-(1-piperidin-4-yl-1H-pyrazol-4-yl)-pyridin-3-yl]-1H-benzimidazol-5-yl}-1-phenyl-ethanol 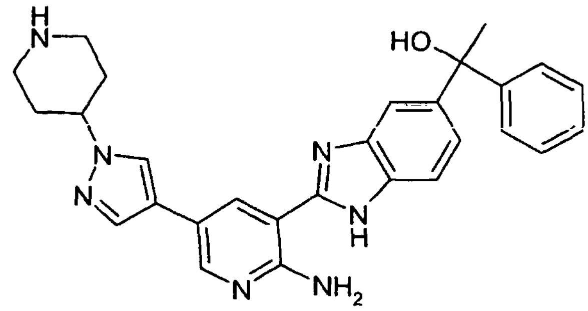 | |
| "A150" | 1-(2-{2-Amino-5-[1-(2-morpholin-4-yl-ethyl)-1H-pyrazol-4-yl]-pyridin-3-yl}-1H-benzimidazol-5-yl)-1-phenyl-ethanol, Formiat | MW 509,61 HPLC-MS $[M+H]^+$ 510.2 |
| | 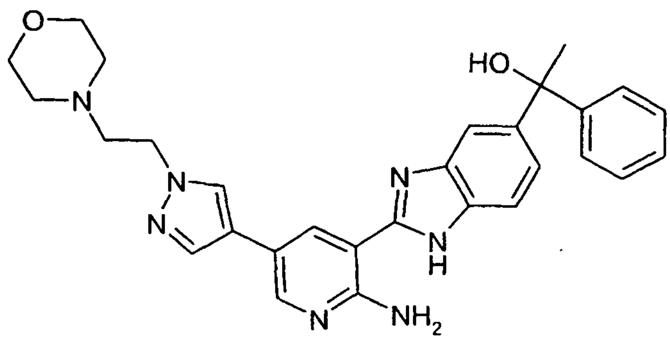 | |
| $^1H$ NMR (400 MHz, DMSO-$d_6$ + TFA-$d_1$) δ [ppm] 8.98 (d, J = 2.1, 1H), 8.43 (d, J = 2.1, 1H), 8.20 (s, 1H), 7.98 (s, 1H), 7.82 (d, J = 1.2, 1H), 7.67 (d, J = 8.6, 1H), 7.52 (dd, J = 8.3, 1.1, 2H), 7.46 (dd, J = 8.6, 1.5, 1H), 7.32 (t, J = 7.7, 2H), 7.21 (t, J = 7.3, 1H), 4.68 (t, J = 6.5, 2H), 4.08 - 3.98 (m, 2H), 3.74 (t, J = 6.5, 4H), 3.58 - 3.45 (m, 2H), 3.30 - 3.14 (m, 2H), 1.97 (s, 3H) | | |

Inhibierung von PDK1

$IC_{50}$ von erfindungsgemäßen Verbindungen

| Verbindung Nr. | $IC_{50}$ PDK1 (Enzym) | $IC_{50}$ PDK1 (Zelle) | $IC_{50}$ IRAK-1 (Enzym) | $IC_{50}$ IRAK-4 (Enzym) | $IC_{50}$ IRAK-4 (Zelle) |
|---|---|---|---|---|---|
| "A1" | A | B | B | C | |
| "A2" | A | B | | | |

(fortgesetzt)

| Verbindung Nr. | $IC_{50}$ PDK1 (Enzym) | $IC_{50}$ PDK1 (Zelle) | $IC_{50}$ IRAK-1 (Enzym) | $IC_{50}$ IRAK-4 (Enzym) | $IC_{50}$ IRAK-4 (Zelle) |
|---|---|---|---|---|---|
| "A3" | A | C | | | |
| "A4" | A | B | | | |
| "A8" | | | B | B | |
| "A9" | A | B | | | |
| "A11" | A | B | | | |
| "A12" | A | B | | | |
| "A13" | A | B | | | |
| "A15" | A | C | | | |
| "A16" | A | C | | | |
| "A17" | A | | | | |
| "A24" | A | | | | |
| "A25" | A | | | | |
| "A26" | A | | | | |
| "A29" | A | C | | | |
| "A30" | | | A | B | |
| "A32" | | | B | B | |
| "A34" | | | B | B | |
| "A35" | | | B | B | |
| "A42" | | | B | B | |
| "A43" | A | C | B | B | |
| "A44" | | | B | B | B |
| "A48" | | | B | B | |
| "A50" | | | B | A | |
| "A52" | A | B | B | B | |
| "A53" | A | B | | | |
| "A55" | A | | | | |
| "A62" | | | B | B | |
| "A64" | A | | | | |
| "A66" | | | B | B | |
| "A67" | A | B | | | |
| "A68" | A | C | | | |
| "A69" | A | | A | A | |
| "A70" | | | B | A | |
| "A72" | | | B | B | |
| "A74" | A | | B | B | |
| "A76" | | | B | C | |
| "A78" | A | | A | B | |

(fortgesetzt)

| Verbindung Nr. | $IC_{50}$ PDK1 (Enzym) | $IC_{50}$ PDK1 (Zelle) | $IC_{50}$ IRAK-1 (Enzym) | $IC_{50}$ IRAK-4 (Enzym) | $IC_{50}$ IRAK-4 (Zelle) |
|---|---|---|---|---|---|
| "A80" | A | C | | | |
| "A89" | A | | B | B | |
| "A92" | A | C | A | A | B |
| "A94" | | | B | B | |
| "A96" | | | B | B | |
| "A102" | A | B | | | |
| "A105" | | | B | B | |
| "A106" | A | C | | | |
| "A107" | | | A | A | B |
| "A113" | | | A | B | |
| "A114" | | | B | A | |
| "A115" | | | B | B | |
| "A120" | A | | | | |
| "A128" | A | C | B | B | |
| "A130" | | | A | A | |
| "A133" | A | C | A | A | |
| "A144" | A | C | B | B | |
| "A148" | B | | B | A | |
| "A150" | B | | | | |

$IC_{50}$: 1 nM - 0,1 μM = A
0,1 μM-10 μM=B
> 10 μM = C

Die nachfolgenden Beispiele betreffen Arzneimittel:

**Beispiel A: Injektionsgläser**

Eine Lösung von 100 g eines Wirkstoffes der Formel I und 5 g Dinatriumhydrogenphosphat wird in 3 l zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2 H_2O$, 28,48 g $Na_2HPO_4 \cdot 12 H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zur Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Patentansprüche**

**1.** Verbindungen ausgewählt aus der Gruppe

| Verbindung Nr. | Name und/oder Struktur |
|---|---|
| "A1" | 6-{2-[2-Amino-5-(1-methyl-1H-pyrazol-4-yl)-pyridin-3-yl]-3H-benzimidazol-5-yl}-5-methyl-4,5-dihydro-2H-pyridazin-3-on |
| "A2" | (R)-6-{2-[2-Amino-5-(1-methyl-1H-pyrazol-4-yl)-pyridin-3-yl]-3H-benzimidazol-5-yl}-5-methyl-4,5-dihydro-2H-pyridazin-3-on |
| "A3" | (S)-6-{2-[2-Amino-5-(1-methyl-1H-pyrazol-4-yl)-pyridin-3-yl]-3H-benzimidazol-5-yl}-5-methyl-4,5-dihydro-2H-pyridazin-3-on |
| "A4" | 6-{2-[2-Amino-5-(1-methyl-1H-pyrazol-4-yl)-pyridin-3-yl]-1H-benzimidazol-5-yl}-5-ethyl-4,5-dihydro-2H-pyridazin-3-on |
| "A5" | 6-{2-[2-Amino-5-(1-methyl-1H-pyrazol-4-yl)-pyridin-3-yl]-3H-benzimidazol-5-yl}-5,5-dimethyl-4,5-dihydro-2H-pyridazin-3-on |
| "A6" | 6-{2-[2-Amino-5-(1-methyl-1H-pyrazol-4-yl)-pyridin-3-yl]-3H-benzimidazol-5-yl}-2,5-dimethyl-4,5-dihydro-2H-pyridazin-3-on |
| "A7" | 6-{2-[2-Amino-5-(1-methyl-1H-pyrazol-4-yl)-pyridin-3-yl]-3H-benzimidazol-5-yl}-4,5-dihydro-2H-pyridazin-3-on |
| "A8" | 6-{2-[2-Amino-5-(1-methyl-1H-pyrazol-4-yl)-pyridin-3-yl]-3H-benzimidazol-5-yl}-2H-pyridazin-3-on |
| "A9" | 6-(2-{2-Amino-5-[1-(2-morpholin-4-yl-ethyl)-1H-pyrazol-4-yl]-pyridin-3-yl}-1H-benzimidazol-5-yl)-5-methyl-4,5-dihydro-2H-pyridazin-3-on |
| "A10" | 6-(2-{2-Amino-5-[1-(3-fluor-benzyl)-1H-pyrazol-4-yl]-pyridin-3-yl}-1H-benzimidazol-5-yl)-5-methyl-4,5-dihydro-2H-pyridazin-3-on |
| "A11" | 6-{2-[2-Amino-5-(1-isopropyl-1H-pyrazol-4-yl)-pyridin-3-yl]-1H-benzimidazol-5-yl}-5-methyl-4,5-dihydro-2H-pyridazin-3-on |

(fortgesetzt)

| Verbindung Nr. | Name und/oder Struktur |
|---|---|
| "A12" | 6-(2-{2-Amino-5-[1-(2-methoxy-ethyl)-1H-pyrazol-4-yl]-pyridin-3-yl}-1H-benzimidazol-5-yl)-5-methyl-4,5-dihydro-2H-pyridazin-3-on |
| "A13" | 6-(2-{2-Amino-5-[1-(3-methoxy-propyl)-1H-pyrazol-4-yl]-pyridin-3-yl}-1H-benzimidazol-5-yl)-5-methyl-4,5-dihydro-2H-pyridazin-3-on |
| "A14" | 6-(2-{2-Amino-5-[1-(2-pyrrolidin-1-yl-ethyl)-1H-pyrazol-4-yl]-pyridin-3-yl}-1H-benzimidazol-5-yl)-5-methyl-4,5-dihydro-2H-pyridazin-3-on |
| "A15" | 6-(2-{2-Amino-5-[1-(2-dimethylamino-ethyl)-1H-pyrazol-4-yl]-pyridin-3-yl}-1H-benzimidazol-5-yl)-5-methyl-4,5-dihydro-2H-pyridazin-3-on |
| "A16" | 6-(2-{2-Amino-5-[1-(1-methyl-piperidin-4-yl)-1H-pyrazol-4-yl]-pyridin-3-yl}-1H-benzimidazol-5-yl)-5-methyl-4,5-dihydro-2H-pyridazin-3-on |
| "A17" | 6-{2-[2-Amino-5-(1-piperidin-4-yl-1H-pyrazol-4-yl)-pyridin-3-yl]-1H-benzimidazol-5-yl}-5-methyl-4,5-dihydro-2H-pyridazin-3-on |
| "A18" | 6-(2-{2-Amino-5-[1-(tetrahydro-furan-2-ylmethyl)-1H-pyrazol-4-yl]-pyridin-3-yl}-1H-benzimidazol-5-yl)-5-methyl-4,5-dihydro-2H-pyridazin-3-on |
| "A19" | 6-[2-(6-Amino-6'-piperazin-1-yl-[3,3']bipyridinyl-5-yl)-1H-benzimidazol-5-yl]-5-methyl-4,5-dihydro-2H-pyridazin-3-on |
| "A20" | 6-{2-[2-Amino-5-(2-dimethylamino-thiazol-4-yl)-pyridin-3-yl]-1H-benzimidazol-5-yl}-5-methyl-4,5-dihydro-2H-pyridazin-3-on |
| "A21" | 6-[2-(6-Amino-6'-methoxy-[3,3']bipyridinyl-5-yl)-1H-benzimidazol-5-yl]-5-methyl-4,5-dihydro-2H-pyridazin-3-on |
| "A22" | 6-[2-(6,6'-Diamino-[3,3']bipyridinyl-5-yl)-1H-benzimidazol-5-yl]-5-methyl-4,5-dihydro-2H-pyridazin-3-on |
| "A23" | 6-{2-[2-Amino-5-(4-fluor-2H-pyrazol-3-yl)-pyridin-3-yl]-1H-benzimidazol-5-yl}-5-methyl-4,5-dihydro-2H-pyridazin-3-on |
| "A24" | (4-{6-Amino-5-[5-(4-methyl-6-oxo-1,4,5,6-tetrahydro-pyridazin-3-yl)-1H-benzimidazol-2-yl]-pyridin-3-yl}-pyrazol-1-yl)-essigsäure |
| "A25" | 2-(4-{6-Amino-5-[5-(4-methyl-6-oxo-1,4,5,6-tetrahydro-pyridazin-3-yl)-1H-benzimidazol-2-yl]-pyridin-3-yl}-pyrazol-1-yl)-N,N-dimethyl-acetamid |
| "A26" | 6-[2-(2-Amino-5-{1-[2-(4-methyl-piperazin-1-yl)-2-oxo-ethyl]-1H-pyrazol-4-yl}-pyridin-3-yl)-1H-benzimidazol-5-yl]-5-methyl-4,5-dihydro-2H-pyridazin-3-on |
| "A27" | 2-{4-[6-Amino-5-(6-methoxy-1H-benzimidazol-2-yl)-pyridin-3-yl]-pyrazol-1-yl}-N-(3,4-difluor-benzyl)-acetamid |
| "A28" | 6-(2-{2-Amino-5-[1-(2-oxo-2-piperidin-1-yl-ethyl)-1H-pyrazol-4-yl]-pyridin-3-yl}-3H-benzimidazol-5-yl)-5-methyl-4,5-dihydro-2H-pyridazin-3-on |
| "A29" | 6-(2-{2-Amino-5-[1-(2-hydroxy-ethyl)-1H-pyrazol-4-yl]-pyridin-3-yl}-3H-benzimidazol-5-yl)-5-methyl-4,5-dihydro-2H-pyridazin-3-on |
| "A30" | 2-{4-[6-Amino-5-(5-fluor-6-methoxy-1H-benzimidazol-2-yl)-pyridin-3-yl]-pyrazol-1-yl}-1-piperidin-1-yl-ethanon |
| "A49" | 3-(5H-[1,3]Dioxolo[4',5':4,5]benzo[1,2-d]imidazol-6-yl)-5-(1-piperidin-4-yl-1 H-pyrazol-4-yl)-pyridin-2-ylamine |
| "A54" | 3-(2,2-Difluor-5H-[1,3]dioxolo[4',5':4,5]benzo[1,2-d]imidazol-6-yl)-5-(1-piperidin-4-yl-1H-pyrazol-4-yl)-pyridin-2-ylamin |
| "A55" | 6-[2-Amino-5-(1-piperidin-4-yl-1H-pyrazol-4-yl)-pyridin-3-yl]-3,5-dihydro-1H-benzo[1,2-d;4,5-d']diimidazol-2-on |

(fortgesetzt)

| Verbindung Nr. | Name und/oder Struktur |
|---|---|
| "A61" | 3-(6,7-Dihydro-1H-5,8-dioxa-1,3-diaza-cyclopenta[b]naphthalin-2-yl)-5-(1-piperidin-4-yl-1H-pyrazol-4-yl)-pyridin-2-ylamin |
| "A71" | 3-[6-Fluor-5-(4-methyl-piperazin-1-yl)-1H-benzimidazol-2-yl]-5-(1-piperidin-4-yl-1H-pyrazol-4-yl)-pyridin-2-ylamin |
| "A73" | 3-(6-Fluor-5-morpholin-4-yl-1H-benzimidazol-2-yl)-5-(1-piperidin-4-yl-1H-pyrazol-4-yl)-pyridin-2-ylamin |
| "A90" | 3-[5-(4-Methyl-piperazin-1-yl)-1H-benzimidazol-2-yl]-5-(1-methyl-1 H-pyrazol-4-yl)-pyridin-2-ylamin |
| "A115" | 3-(1,7-Dihydro-imidazo[4,5-f]indazol-6-yl)-5-(1-methyl-1H-pyrazol-4-yl)-pyridin-2-ylamin |
| "A119" | 2-{2-[2-Amino-5-(1-methyl-1H-pyrazol-4-yl)-pyridin-3-yl]-1H-benzimidazol-5-yl}-N-(3,4-difluor-benzyl)-acetamid |
| "A120" | 2-{2-[2-Amino-5-(1-methyl-1H-pyrazol-4-yl)-pyridin-3-yl]-1H-benzimidazol-5-yl}-acetamid |
| "A121" | 2-{2-[2-Amino-5-(1-methyl-1H-pyrazol-4-yl)-pyridin-3-yl]-3H-benzimidazol-5-yl}-N-methyl-acetamid |
| "A122" | 2-{2-[2-Amino-5-(1-methyl-1H-pyrazol-4-yl)-pyridin-3-yl]-3H-benzimidazol-5-yl}-N-Ethylacetamid |
| "A123" | 2-[2-Amino-5-(1-methyl-1H-pyrazol-4-yl)-pyridin-3-yl]-3H-benzimidazol-5-carbonsäure-isopropylamid |
| "A124" | 2-[2-Amino-5-(1-methyl-1H-pyrazol-4-yl)-pyridin-3-yl]-3H-benzimidazole-5-carbonsäure-dimethylamid |
| "A125" | 4-({2-[2-Amino-5-(1-methyl-1H-pyrazol-4-yl)-pyridin-3-yl]-3H-benzimidazole-5-carbonyl}-amino)-piperidin-1-carbonsäure-tert.-butylester |
| "A126" | 2-[2-Amino-5-(1-methyl-1H-pyrazol-4-yl)-pyridin-3-yl]-3H-benzimidazol-5-carbonsäure-piperidin-4-ylamid |
| "A127" | 2-[2-Amino-5-(1-methyl-1H-pyrazol-4-yl)-pyridin-3-yl]-1H-benzimidazol-4-carbonsäure-(2-methoxy-ethyl)-amid |
| "A128" | 2-[2-Amino-5-(1-methyl-1H-pyrazol-4-yl)-pyridin-3-yl]-3H-benzimidazol-5-carbonsäure-[2-(3,4-difluor-phenyl)-ethyl]-amid |
| "A129" | 2-[2-Amino-5-(1-methyl-1H-pyrazol-4-yl)-pyridin-3-yl]-1H-benzimidazol-4-carbonsäure-piperidin-4-ylamid |
| "A130" | 5-[6-Amino-5-(6-methoxy-1H-benzimidazol-2-yl)-pyridin-3-yl]-thiophen-2-carbonsäure-(2-methoxy-ethyl)-amid |
| "A131" | 5-[6-Amino-5-(6-methoxy-1H-benzimidazol-2-yl)-pyridin-3-yl]-thiophen-2-carbonsäure-(2-dimethylamino-ethyl)-amid |
| "A132" | 5-[6-Amino-5-(6-methoxy-1H-benzimidazol-2-yl)-pyridin-3-yl]-thiophen-2-carbonsäure-piperidin-4-ylamid |
| "A133" | 5-[6-Amino-5-(6-methoxy-1H-benzimidazol-2-yl)-pyridin-3-yl]-thiophen-2-carbonsäure-(1-methyl-piperidin-4-yl)-amid |
| "A134" | 5-[6-Amino-5-(6-methoxy-1H-benzimidazol-2-yl)-pyridin-3-yl]-thiophen-2-carbonsäure-(pyridin-4-ylmethyl)-amid |
| "A135" | N-(2-{2-[2-Amino-5-(1-methyl-1H-pyrazol-4-yl)-pyridin-3-yl]-3H-benzimidazol-5-yl}-ethyl)-3,4-difluor-benzamid |
| "A137" | 3-[5-(4-Methyl-piperazin-1-sulfonyl)-1H-benzimidazol-2-yl]-5-(1-methyl-1H-pyrazol-4-yl)-pyridin-2-ylamin |

(fortgesetzt)

| Verbindung Nr. | Name und/oder Struktur |
|---|---|
| "A140" | 2-[2-Amino-5-(1-methyl-1H-pyrazol-4-yl)-pyridin-3-yl]-5-fluor-3H-benzimidazol-4-carbonsäure-(pyridin-4-ylmethyl)-amid |
| "A143" | 1-{2-[2-Amino-5-(1-methyl-1H-pyrazol-4-yl)-pyridin-3-yl]-1H-benzimidazol-5-yl}-1-phenyl-ethanol |
| "A146" | 4-{2-[2-Amino-5-(1-methyl-1H-pyrazol-4-yl)-pyridin-3-yl]-3H-benzimidazol-5-yl}-morpholin-3-on |
| "A147" | 3-{2-[2-Amino-5-(1-methyl-1H-pyrazol-4-yl)-pyridin-3-yl]-1H-benzimidazol-5-yl}-[1,3]oxazinan-2-on |
| "A149" | 1-{2-[2-Amino-5-(1-piperidin-4-yl-1H-pyrazol-4-yl)-pyridin-3-yl]-1H-benzimidazol-5-yl}-1-phenyl-ethanol |
| "A150" | 1-(2-{2-Amino-5-[1-(2-morpholin-4-yl-ethyl)-1H-pyrazol-4-yl]-pyridin-3-yl}-1H-benzimidazol-5-yl)-1-phenyl-ethanol |

sowie ihre pharmazeutisch verwendbaren Salze, Tautomere und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

3. Verwendung von Verbindungen nach Anspruch 1 sowie ihrer pharmazeutisch verwendbaren Salze, Tautomeren und Stereoisomeren, einschließlich deren Mischungen in allen Verhältnissen, zur Herstellung eines Arzneimittels zur Behandlung von Tumoren, Tumorwachstum, Tumormetastasen und/oder AIDS.

4. Verwendung von Verbindungen nach Anspruch 1 sowie ihrer pharmazeutisch verwendbaren Salze, Tautomeren und Stereoisomeren, einschließlich deren Mischungen in allen Verhältnissen, zur Herstellung eines Arzneimittels zur Behandlung von entzündlichen oder allergischen Erkrankungen, entzündlichen Darmerkrankungen, Vaginitis, Psoriasis, entzündlichen Dermatosen, Vasculitis, Spondyloarthropathien, Sklerodermie, Asthma, respiratorischen allergischen Erkrankungen, Autoimmunerkrankungen, Transplantatabstoßung, Atherosklerose, Myositis, neurologische Störungen, ischämische Reperfusionsverletzungen, offene Schädelhirntraumata, gedeckte Schädelhirntraumata, neurodegenerative Erkrankungen, Multiple Sklerose, Morbus Alzheimer, Enzephalitis, Meningitis, Osteoporose, Gicht, Hepatitis, Nephritis, Gallenblasenerkrankungen, Sepsis, Sarkoidose, Konjunktivitis, Otitis, chronisch obstruktive Lungenerkrankung, Sinusitis und Behcet-Syndrom, zellproliferative oder neoplastische Erkrankungen, Fettsucht, Diabetes Typ II, metabolisches Syndrom, Insulinresistenz, Hyperglykämie, Hyperurikämie, Hyperinsulinämie, Kachexie, Hyper-cholesterolämie, Hyperlipidämie, Dyslipidämie, gemischte Dyslipidämie und Hypertriglyceridämie, Essstörungen, infektiöse Erkrankungen, akutes Herzversagen, Hypotonie, Hypertonie, Angina Pectoris, Myokardinfarkt, Kardiomyopathie, kongestive Herzinsuffizienz, Atherosklerose, Erkrankungen der Koronararterien, Restenose und vaskuläre Stenose.

## Claims

1. Compounds selected from the group

| Compound No. | Name and/or structure |
|---|---|
| "A1" | 6-{2-[2-Amino-5-(1-methyl-1H-pyrazol-4-yl)pyridin-3-yl]-3H-benzimidazol-5-yl}-5-methyl-4,5-dihydro-2H-pyridazin-3-one |
| "A2" | (R)-6-{2-[2-Amino-5-(1-methyl-1H-pyrazol-4-yl)pyridin-3-yl]-3H-benzimidazol-5-yl}-5-methyl-4,5-dihydro-2H-pyridazin-3-one |
| "A3" | (S)-6-{2-[2-Amino-5-(1-methyl-1H-pyrazol-4-yl)pyridin-3-yl]-3H-benzimidazol-5-yl}-5-methyl-4,5-dihydro-2H-pyridazin-3-one |

(continued)

| Compound No. | Name and/or structure |
|---|---|
| "A4" | 6-{2-[2-Amino-5-(1-methyl-1H-pyrazol-4-yl)pyridin-3-yl]-1H-benzimidazol-5-yl}-5-ethyl-4,5-dihydro-2H-pyridazin-3-one |
| "A5" | 6-{2-[2-Amino-5-(1-methyl-1H-pyrazol-4-yl)pyridin-3-yl]-3H-benzimidazol-5-yl}-5,5-dimethyl-4,5-dihydro-2H-pyridazin-3-one |
| "A6" | 6-{2-[2-Amino-5-(1-methyl-1H-pyrazol-4-yl)pyridin-3-yl]-3H-benzimidazol-5-yl}-2,5-dimethyl-4,5-dihydro-2H-pyridazin-3-one |
| "A7" | 6-{2-[2-Amino-5-(1-methyl-1H-pyrazol-4-yl)pyridin-3-yl]-3H-benzimidazol-5-yl}-4,5-dihydro-2H-pyridazin-3-one |
| "A8" | 6-{2-[2-Amino-5-(1-methyl-1H-pyrazol-4-yl)pyridin-3-yl]-3H-benzimidazol-5-yl}-2H-pyridazin-3-one |
| "A9" | 6-(2-{2-Amino-5-[1-(2-morpholin-4-ylethyl)-1H-pyrazol-4-yl]pyridin-3-yl}-1H-benzimidazol-5-yl)-5-methyl-4,5-dihydro-2H-pyridazin-3-one |
| "A10" | 6-(2-{2-Amino-5-[1-(3-fluorobenzyl)-1H-pyrazol-4-yl]-pyridin-3-yl}-1H-benzimidazol-5-yl)-5-methyl-4,5-dihydro-2H-pyridazin-3-one |
| "A11" | 6-{2-[2-Amino-5-(1-isopropyl-1H-pyrazol-4-yl)pyridin-3-yl]-1H-benzimidazol-5-yl}-5-methyl-4,5-dihydro-2H-pyridazin-3-one |
| "A12" | 6-(2-{2-Amino-5-[1-(2-methoxyethyl)-1H-pyrazol-4-yl]-pyridin-3-yl}-1H-benzimidazol-5-yl)-5-methyl-4,5-dihydro-2H-pyridazin-3-one |
| "A13" | 6-(2-{2-Amino-5-[1-(3-methoxypropyl)-1H-pyrazol-4-yl]-pyridin-3-yl}-1H-benzimidazol-5-yl)-5-methyl-4,5-dihydro-2H-pyridazin-3-one |
| "A14" | 6-(2-{2-Amino-5-[1-(2-pyrrolidin-1-ylethyl)-1H-pyrazol-4-yl]-pyridin-3-yl}-1H-benzimidazol-5-yl)-5-methyl-4,5-dihydro-2H-pyridazin-3-one |
| "A15" | 6-(2-{2-Amino-5-[1-(2-dimethylaminoethyl)-1H-pyrazol-4-yl]pyridin-3-yl}-1H-benzimidazol-5-yl)-5-methyl-4,5-dihydro-2H-pyridazin-3-one |
| "A16" | 6-(2-{2-Amino-5-[1-(1-methylpiperidin-4-yl)-1H-pyrazol-4-yl]pyridin-3-yl}-1H-benzimidazol-5-yl)-5-methyl-4,5-dihydro-2H-pyridazin-3-one |
| "A17" | 6-{2-[2-Amino-5-(1-piperidin-4-yl-1H-pyrazol-4-yl)pyridin-3-yl]-1H-benzimidazol-5-yl}-5-methyl-4,5-dihydro-2H-pyridazin-3-one |
| "A18" | 6-(2-{2-Amino-5-[1-(tetrahydrofuran-2-ylmethyl)-1H-pyrazol-4-yl]pyridin-3-yl}-1H-benzimidazol-5-yl)-5-methyl-4,5-dihydro-2H-pyridazin-3-one |
| "A19" | 6-[2-(6-Amino-6’-piperazin-1-yl-[3,3’]bipyridinyl-5-yl)-1H-benzimidazol-5-yl]-5-methyl-4,5-dihydro-2H-pyridazin-3-one |
| "A20" | 6-{2-[2-Amino-5-(2-dimethylaminothiazol-4-yl)pyridin-3-yl]-1H-benzimidazol-5-yl}-5-methyl-4,5-dihydro-2H-pyridazin-3-one |
| "A21" | 6-[2-(6-Amino-6’-methoxy-[3,3’]bipyridinyl-5-yl)-1H-benzimidazol-5-yl]-5-methyl-4,5-dihydro-2H-pyridazin-3-one |
| "A22" | 6-[2-(6,6’-Diamino-[3,3’]bipyridinyl-5-yl)-1H-benzimidazol-5-yl]-5-methyl-4,5-dihydro-2H-pyridazin-3-one |
| "A23" | 6-{2-[2-Amino-5-(4-fluoro-2H-pyrazol-3-yl)pyridin-3-yl]-1H-benzimidazol-5-yl}-5-methyl-4,5-dihydro-2H-pyridazin-3-one |
| "A24" | (4-{6-Amino-5-[5-(4-methyl-6-oxo-1,4,5,6-tetrahydro-pyridazin-3-yl)-1H-benzimidazol-2-yl]pyridin-3-yl}pyrazol-1-yl)acetic acid |
| "A25" | 2-(4-{6-Amino-5-[5-(4-methyl-6-oxo-1,4,5,6-tetrahydro-pyridazin-3-yl)-1H-benzimidazol-2-yl]pyridin-3-yl}pyrazol-1-yl)-N,N-dimethylacetamide |

(continued)

| Compound No. | Name and/or structure |
|---|---|
| "A26" | 6-[2-(2-Amino-5-{1-[2-(4-methylpiperazin-1-yl)-2-oxoethyl]-1H-pyrazol-4-yl}pyridin-3-yl)-1H-benzimidazol-5-yl]-5-methyl-4,5-dihydro-2H-pyridazin-3-one |
| "A27" | 2-{4-[6-Amino-5-(6-methoxy-1H-benzimidazol-2-yl)pyridin-3-yl]pyrazol-1-yl}-N-(3,4-difluorobenzyl)acetamide |
| "A28" | 6-(2-{2-Amino-5-[1-(2-oxo-2-piperidin-1-ylethyl)-1H-pyrazol-4-yl]pyridin-3-yl}-3H-benzimidazol-5-yl)-5-methyl-4,5-dihydro-2H-pyridazin-3-one |
| "A29" | 6-(2-{2-Amino-5-[1-(2-hydroxyethyl)-1H-pyrazol-4-yl]-pyridin-3-yl}-3H-benzimidazol-5-yl)-5-methyl-4,5-dihydro-2H-pyridazin-3-one |
| "A30" | 2-{4-[6-Amino-5-(5-fluoro-6-methoxy-1H-benzimidazol-2-yl)pyridin-3-yl]pyrazol-1-yl}-1-piperidin-1-ylethanone |
| "A49" | 3-(5H-[1,3]Dioxolo[4',5':4,5]benzo[1,2-d]imidazol-6-yl)-5-(1-piperidin-4-yl-1H-pyrazol-4-yl)pyridin-2-ylamine |
| "A54" | 3-(2,2-Difluoro-5H-[1,3]dioxolo[4',5':4,5]benzo[1,2-d]-imidazol-6-yl)-5-(1-piperidin-4-yl-1H-pyrazol-4-yl)pyridin-2-ylamine |
| "A55" | 6-[2-Amino-5-(1-piperidin-4-yl-1H-pyrazol-4-yl)pyridin-3-yl]-3,5-dihydro-1H-benzo[1,2-d;4,5-d']diimidazol-2-one |
| "A61" | 3-(6,7-Dihydro-1H-5,8-dioxa-1,3-diaza-cyclopenta[b]naphthalen-2-yl)-5-(1-piperidin-4-yl-1H-pyrazol-4-yl)pyridin-2-ylamine |
| "A71" | 3-[6-Fluoro-5-(4-methylpiperazin-1-yl)-1H-benzimidazol-2-yl]-5-(1-piperidin-4-yl-1H-pyrazol-4-yl)pyridin-2-ylamine |
| "A73" | 3-(6-Fluoro-5-morpholin-4-yl-1H-benzimidazol-2-yl)-5-(1-piperidin-4-yl-1H-pyrazol-4-yl)pyridin-2-ylamine |
| "A90" | 3-[5-(4-Methylpiperazin-1-yl)-1H-benzimidazol-2-yl]-5-(1-methyl-1H-pyrazol-4-yl)pyridin-2-ylamine |
| "A115" | 3-(1,7-Dihydroimidazo[4,5-f]indazol-6-yl)-5-(1-methyl-1H-pyrazol-4-yl)pyridin-2-ylamine |
| "A119" | 2-{2-[2-Amino-5-(1-methyl-1H-pyrazol-4-yl)pyridin-3-yl]-1H-benzimidazol-5-yl}-N-(3,4-difluorobenzyl)acetamide |
| "A120" | 2-{2-[2-Amino-5-(1-methyl-1H-pyrazol-4-yl)pyridin-3-yl]-1H-benzimidazol-5-yl}-acetamide |
| "A121" | 2-{2-[2-Amino-5-(1-methyl-1H-pyrazol-4-yl)pyridin-3-yl]-3H-benzimidazol-5-yl}-N-methylacetamide |
| "A122" | 2-{2-[2-Amino-5-(1-methyl-1H-pyrazol-4-yl)pyridin-3-yl]-3H-benzimidazol-5-yl}-N-ethylacetamide |
| "A123" | N-Isopropyl-2-[2-amino-5-(1-methyl-1H-pyrazol-4-yl)-pyridin-3-yl]-3H-benzimidazole-5-carboxamide |
| "A124" | N,N-Dimethyl-2-[2-amino-5-(1-methyl-1H-pyrazol-4-yl)-pyridin-3-yl]-3H-benzimidazole-5-carboxamide |
| "A125" | tert-Butyl 4-({2-[2-amino-5-(1-methyl-1H-pyrazol-4-yl)-pyridin-3-yl]-3H-benzimidazole-5-carbonyl}amino)-piperidine-1-carboxylate |
| "A126" | N-Piperidin-4-yl-2-[2-amino-5-(1-methyl-1H-pyrazol-4-yl)-pyridin-3-yl]-3H-benzimidazole-5-carboxamide |
| "A127" | N-(2-Methoxyethyl)-2-[2-amino-5-(1-methyl-1H-pyrazol-4-yl)pyridin-3-yl]-1H-benzimidazole-4-carboxamide |
| "A128" | N-[2-(3,4-Difluorophenyl)ethyl]-2-[2-amino-5-(1-methyl-1H-pyrazol-4-yl)pyridin-3-yl]-3H-benzimidazole-5-carboxamide |

(continued)

| Compound No. | Name and/or structure |
|---|---|
| "A129" | N-Piperidin-4-yl-2-[2-amino-5-(1-methyl-1H-pyrazol-4-yl)-pyridin-3-yl]-1H-benzimidazole-4-carboxamide |
| "A130" | N-(2-Methoxyethyl)-5-[6-amino-5-(6-methoxy-1H-benzimidazol-2-yl)pyridin-3-yl]thiophene-2-carboxamide |
| "A131" | N-(2-Dimethylaminoethyl)-5-[6-amino-5-(6-methoxy-1H-benzimidazol-2-yl)pyridin-3-yl]thiophene-2-carboxamide |
| "A132" | N-Piperidin-4-yl-5-[6-amino-5-(6-methoxy-1H-benzimidazol-2-yl)pyridin-3-yl]thiophene-2-carboxamide |
| "A133" | N-(1-Methylpiperidin-4-yl)-5-[6-amino-5-(6-methoxy-1H-benzimidazol-2-yl)pyridin-3-yl]thiophene-2-carboxamide |
| "A134" | N-(Pyridin-4-ylmethyl)-5-[6-amino-5-(6-methoxy-1H-benzimidazol-2-yl)pyridin-3-yl]thiophene-2-carboxamide |
| "A135" | N-(2-{2-[2-Amino-5-(1-methyl-1H-pyrazol-4-yl)pyridin-3-yl]-3H-benzimidazol-5-yl}ethyl)-3,4-difluorobenzamide |
| "A137" | 3-[5-(4-Methylpiperazine-1-sulfonyl)-1H-benzimidazol-2-yl]-5-(1-methyl-1H-pyrazol-4-yl)pyridin-2-ylamine |
| "A140" | N-(Pyridin-4-ylmethyl)-2-[2-amino-5-(1-methyl-1H-pyrazol-4-yl)pyridin-3-yl]-5-fluoro-3H-benzimidazole-4-carboxamide |
| "A143" | 1-{2-[2-Amino-5-(1-methyl-1H-pyrazol-4-yl)pyridin-3-yl]-1H-benzimidazol-5-yl}-1-phenylethanol |
| "A146" | 4-{2-[2-Amino-5-(1-methyl-1H-pyrazol-4-yl)pyridin-3-yl]-3H-benzimidazol-5-yl}morpholin-3-one |
| "A147" | 3-{2-[2-Amino-5-(1-methyl-1H-pyrazol-4-yl)pyridin-3-yl]-1H-benzimidazol-5-yl}-1,3-oxazinan-2-one |
| "A149" | 1-{2-[2-Amino-5-(1-piperidin-4-yl-1H-pyrazol-4-yl)pyridin-3-yl]-1H-benzimidazol-5-yl}-1-phenylethanol |
| "A150" | 1-(2-{2-Amino-5-[1-(2-morpholin-4-ylethyl)-1H-pyrazol-4-yl]pyridin-3-yl}-1H-benzimidazol-5-yl)-1-phenylethanol |

and pharmaceutically usable salts, tautomers and stereoisomers thereof, including mixtures thereof in all ratios.

2. Medicaments comprising at least one compound according to Claim 1 and/or pharmaceutically usable salts, tautomers and stereoisomers thereof, including mixtures thereof in all ratios, and optionally excipients and/or adjuvants.

3. Use of compounds according to Claim 1
and pharmaceutically usable salts, tautomers and stereoisomers thereof, including mixtures thereof in all ratios, for the preparation of a medicament for the treatment of tumours, tumour growth, tumour metastases and/or AIDS.

4. Use of compounds according to Claim 1
and pharmaceutically usable salts, tautomers and stereoisomers thereof, including mixtures thereof in all ratios, for the preparation of a medicament for the treatment of
inflammatory or allergic diseases, inflammatory bowel diseases, vaginitis, psoriasis, inflammatory dermatoses, vasculitis, spondyloarthropathies, scleroderma, asthma, respiratory allergic diseases, autoimmune diseases, transplant rejection, atherosclerosis, myositis, neurological disorders, ischaemic reperfusion injuries, open traumatic brain injuries, closed traumatic brain injuries, neurodegenerative diseases, multiple sclerosis, Alzheimer's disease, encephalitis, meningitis, osteoporosis, gout, hepatitis, nephritis, gall bladder diseases, sepsis, sarcoidosis, conjunctivitis, otitis, chronic obstructive pulmonary disease, sinusitis and Behcet's syndrome, cell-proliferative or neoplastic diseases,
obesity, type II diabetes, metabolic syndrome, insulin resistance, hyper-glycaemia, hyperuricaemia, hyperinsulinaemia, cachexia, hypercholesterolaemia, hyperlipidaemia, dyslipidaemia, mixed dyslipidaemia and hypertriglyceridaemia, eating disorders, infectious diseases, acute heart failure, hypotension, hypertension, angina pectoris, myocardial infarction, cardiomyopathy, congestive heart failure, atherosclerosis, coronary artery diseases, restenosis and vascular stenosis.

## Revendications

**1.** Composés choisis dans le groupe constitué par

| Composé n° | Nom et/ou structure |
|---|---|
| "A1" | 6-{2-[2-Amino-5-(1-méthyl-1H-pyrazol-4-yl)pyridin-3-yl]-3H-benzimidazol-5-yl}-5-méthyl-4,5-dihydro-2H-pyridazin-3-one |
| "A2" | (R)-6-{2-[2-Amino-5-(1-méthyl-1H-pyrazol-4-yl)pyridin-3-yl]-3H-benzimidazol-5-yl}-5-méthyl-4,5-dihydro-2H-pyridazin-3-one |
| "A3" | (S)-6-{2-[2-Amino-5-(1-méthyl-1H-pyrazol-4-yl)pyridin-3-yl]-3H-benzimidazol-5-yl}-5-méthyl-4,5-dihydro-2H-pyridazin-3-one |
| "A4" | 6-{2-[2-Amino-5-(1-méthyl-1H-pyrazol-4-yl)pyridin-3-yl]-1H-benzimidazol-5-yl}-5-éthyl-4,5-dihydro-2H-pyridazin-3-one |
| "A5" | 6-{2-[2-Amino-5-(1-méthyl-1H-pyrazol-4-yl)pyridin-3-yl]-3H-benzimidazol-5-yl}-5,5-diméthyl-4,5-dihydro-2H-pyridazin-3-one |
| "A6" | 6-{2-[2-Amino-5-(1-méthyl-1H-pyrazol-4-yl)pyridin-3-yl]-3H-benzimidazol-5-yl}-2,5-diméthyl-4,5-dihydro-2H-pyridazin-3-one |
| "A7" | 6-{2-[2-Amino-5-(1-méthyl-1H-pyrazol-4-yl)pyridin-3-yl]-3H-benzimidazol-5-yl}-4,5-dihydro-2H-pyridazin-3-one |
| "A8" | 6-{2-[2-Amino-5-(1-méthyl-1H-pyrazol-4-yl)pyridin-3-yl]-3H-benzimidazol-5-yl}-2H-pyridazin-3-one |
| "A9" | 6-(2-{2-Amino-5-[1-(2-morpholin-4-yléthyl)-1H-pyrazol-4-yl]pyridin-3-yl}-1H-benzimidazol-5-yl)-5-méthyl-4,5-dihydro-2H-pyridazin-3-one |
| "A10" | 6-(2-{2-Amino-5-[1-(3-fluorobenzyl)-1H-pyrazol-4-yl]-pyridin-3-yl}-1H-benzimidazol-5-yl)-5-méthyl-4,5-dihydro-2H-pyridazin-3-one |
| "A11" | 6-{2-[2-Amino-5-(1-isopropyl-1H-pyrazol-4-yl)pyridin-3-yl]-1H-benzimidazol-5-yl}-5-méthyl-4,5-dihydro-2H-pyridazin-3-one |
| "A12" | 6-(2-{2-Amino-5-[1-(2-méthoxyéthyl)-1H-pyrazol-4-yl]-pyridin-3-yl}-1H-benzimidazol-5-yl)-5-méthyl-4,5-dihydro-2H-pyridazin-3-one |
| "A13" | 6-(2-{2-Amino-5-[1-(3-méthoxypropyl)-1H-pyrazol-4-yl]-pyridin-3-yl}-1H-benzimidazol-5-yl)-5-méthyl-4,5-dihydro-2H-pyridazin-3-one |
| "A14" | 6-(2-{2-Amino-5-[1-(2-pyrrolidin-1-yléthyl)-1H-pyrazol-4-yl]-pyridin-3-yl}-1H-benzimidazol-5-yl)-5-méthyl-4,5-dihydro-2H-pyridazin-3-one |
| "A15" | 6-(2-{2-Amino-5-[1-(2-diméthylaminoéthyl)-1H-pyrazol-4-yl]pyridin-3-yl}-1H-benzimidazol-5-yl)-5-méthyl-4,5-dihydro-2H-pyridazin-3-one |
| "A16" | 6-(2-{2-Amino-5-[1-(1-méthylpipéridin-4-yl)-1H-pyrazol-4-yl]pyridin-3-yl}-1H-benzimidazol-5-yl)-5-méthyl-4,5-dihydro-2H-pyridazin-3-one |
| "A17" | 6-{2-[2-Amino-5-(1-pipéridin-4-yl-1H-pyrazol-4-yl)pyridin-3-yl]-1H-benzimidazol-5-yl}-5-méthyl-4,5-dihydro-2H-pyridazin-3-one |
| "A18" | 6-(2-{2-Amino-5-[1-(tétrahydrofuran-2-ylméthyl)-1H-pyrazol-4-yl]pyridin-3-yl}-1H-benzimidazol-5-yl)-5-méthyl-4,5-dihydro-2H-pyridazin-3-one |
| "A19" | 6-[2-(6-Amino-6'-pipérazin-1-yl-[3,3']bipyridinyl-5-yl)-1H-benzimidazol-5-yl]-5-méthyl-4,5-dihydro-2H-pyridazin-3-one |

(suite)

| Composé n° | Nom et/ou structure |
|---|---|
| "A20" | 6-{2-[2-Amino-5-(2-diméthylaminothiazol-4-yl)pyridin-3-yl]-1H-benzimidazol-5-yl}-5-méthyl-4,5-dihydro-2H-pyridazin-3-one |
| "A21" | 6-[2-(6-Amino-6'-méthoxy-[3,3']bipyridinyl-5-yl)-1H-benzimidazol-5-yl]-5-méthyl-4,5-dihydro-2H-pyridazin-3-one |
| "A22" | 6-[2-(6,6'-Diamino-[3,3']bipyridinyl-5-yl)-1H-benzimidazol-5-yl]-5-méthyl-4,5-dihydro-2H-pyridazin-3-one |
| "A23" | 6-{2-[2-Amino-5-(4-fluoro-2H-pyrazol-3-yl)pyridin-3-yl]-1H-benzimidazol-5-yl}-5-méthyl-4,5-dihydro-2H-pyridazin-3-one |
| "A24" | Acide (4-{6-amino-5-[5-(4-méthyl-6-oxo-1,4,5,6-tétrahydro-pyridazin-3-yl)-1H-benzimidazol-2-yl]pyridin-3-yl}pyrazol-1-yl)acétique |
| "A25" | 2-(4-{6-Amino-5-[5-(4-méthyl-6-oxo-1,4,5,6-tétrahydro-pyridazin-3-yl)-1H-benzimidazol-2-yl]pyridin-3-yl}pyrazol-1-yl)-N,N-diméthylacétamide |
| "A26" | 6-[2-(2-Amino-5-{1-[2-(4-méthylpipérazin-1-yl)-2-oxoéthyl]-1H-pyrazol-4-yl}pyridin-3-yl)-1H-benzimidazol-5-yl]-5-méthyl-4,5-dihydro-2H-pyridazin-3-one |
| "A27" | 2-{4-[6-Amino-5-(6-méthoxy-1H-benzimidazol-2-yl)pyridin-3-yl]pyrazol-1-yl}-N-(3,4-difluorobenzyl)acétamide |
| "A28" | 6-(2-{2-Amino-5-[1-(2-oxo-2-pipéridin-1-yléthyl)-1H-pyrazol-4-yl]pyridin-3-yl}-3H-benzimidazol-5-yl)-5-méthyl-4,5-dihydro-2H-pyridazin-3-one |
| "A29" | 6-(2-{2-Amino-5-[1-(2-hydroxyéthyl)-1H-pyrazol-4-yl]-pyridin-3-yl}-3H-benzimidazol-5-yl)-5-méthyl-4,5-dihydro-2H-pyridazin-3-one |
| "A30" | 2-{4-[6-Amino-5-(5-fluoro-6-méthoxy-1H-benzimidazol-2-yl)pyridin-3-yl]pyrazol-1-yl}-1-pipéridin-1-yléthanone |
| "A49" | 3-(5H-[1,3]Dioxolo[4',5':4,5]benzo[1,2-d]imidazol-6-yl)-5-(1-pipéridin-4-yl-1H-pyrazol-4-yl)pyridin-2-ylamine |
| "A54" | 3-(2,2-Difluoro-5H-[1,3]dioxolo[4',5':4,5]benzo[1,2-d]-imidazol-6-yl)-5-(1-pipéridin-4-yl-1H-pyrazol-4-yl)pyridin-2-ylamine |
| "A55" | 6-[2-Amino-5-(1-pipéridin-4-yl-1H-pyrazol-4-yl)pyridin-3-yl]-3,5-dihydro-1H-benzo[1,2-d;4,5-d']diimidazol-2-one |
| "A61" | 3-(6,7-Dihydro-1H-5,8-dioxa-1,3-diaza-cyclopenta[b]naphtalén-2-yl)-5-(1-pipéridin-4-yl-1H-pyrazol-4-yl)pyridin-2-ylamine |
| "A71" | 3-[6-Fluoro-5-(4-méthylpipérazin-1-yl)-1H-benzimidazol-2-yl]-5-(1-pipéridin-4-yl-1H-pyrazol-4-yl)pyridin-2-ylamine |
| "A73" | 3-(6-Fluoro-5-morpholin-4-yl-1H-benzimidazol-2-yl)-5-(1-pipéridin-4-yl-1H-pyrazol-4-yl)pyridin-2-ylamine |
| "A90" | 3-[5-(4-Méthylpipérazin-1-yl)-1H-benzimidazol-2-yl]-5-(1-méthyl-1H-pyrazol-4-yl)pyridin-2-ylamine |
| "A115" | 3-(1,7-Dihydroimidazo[4,5-f]indazol-6-yl)-5-(1-méthyl-1H-pyrazol-4-yl)pyridin-2-ylamine |
| "A119" | 2-{2-[2-Amino-5-(1-méthyl-1H-pyrazol-4-yl)pyridin-3-yl]-1H-benzimidazol-5-yl}-N-(3,4-difluorobenzyl)acétamide |
| "A120" | 2-{2-[2-Amino-5-(1-méthyl-1H-pyrazol-4-yl)pyridin-3-yl]-1H-benzimidazol-5-yl}-acétamide |
| "A121" | 2-{2-[2-Amino-5-(1-méthyl-1H-pyrazol-4-yl)pyridin-3-yl]-3H-benzimidazol-5-yl}-N-méthylacétamide |
| "A122" | 2-{2-[2-Amino-5-(1-méthyl-1H-pyrazol-4-yl)pyridin-3-yl]-3H-benzimidazol-5-yl}-N-éthylacétamide |
| "A123" | N-Isopropyl-2-[2-amino-5-(1-méthyl-1H-pyrazol-4-yl)-pyridin-3-yl]-3H-benzimidazole-5-carboxamide |

(suite)

| Composé n° | Nom et/ou structure |
|---|---|
| "A124" | N,N-Diméthyl-2-[2-amino-5-(1-méthyl-1H-pyrazol-4-yl)-pyridin-3-yl]-3H-benzimidazole-5-carboxamide |
| "A125" | 4-({2-[2-Amino-5-(1-méthyl-1H-pyrazol-4-yl)pyridin-3-yl]-3H-benzimidazole-5-carbonyl}amino)-pipéridine-1-carboxylatedetertio-butyle |
| "A126" | N-Pipéridin-4-yl-2-[2-amino-5-(1-méthyl-1H-pyrazol-4-yl)-pyridin-3-yl]-3H-benzimidazole-5-carboxamide |
| "A127" | N-(2-Méthoxyéthyl)-2-[2-amino-5-(1-méthyl-1H-pyrazol-4-yl)pyridin-3-yl]-1H-benzimidazole-4-carboxamide |
| "A128" | N-[2-(3,4-Difluorophényl)éthyl]-2-[2-amino-5-(1-méthyl-1H-pyrazol-4-yl)pyridin-3-yl]-3H-benzimidazole-5-carboxamide |
| "A129" | N-Pipéridin-4-yl-2-[2-amino-5-(1-méthyl-1H-pyrazol-4-yl)-pyridin-3-yl]-1H-benzimidazole-4-carboxamide |
| "A130" | N-(2-Méthoxyéthyl)-5-[6-amino-5-(6-méthoxy-1H-benzimidazol-2-yl)pyridin-3-yl]thiophène-2-carboxamide |
| "A131" | N-(2-Diméthylaminoéthyl)-5-[6-amino-5-(6-méthoxy-1H-benzimidazol-2-yl)pyridin-3-yl]thiophène-2-carboxamide |
| "A132" | N-Pipéridin-4-yl-5-[6-amino-5-(6-méthoxy-1H-benzimidazol-2-yl)pyridin-3-yl]thiophène-2-carboxamide |
| "A133" | N-(1-Méthylpipéridin-4-yl)-5-[6-amino-5-(6-méthoxy-1H-benzimidazol-2-yl)pyridin-3-yl]thiophène-2-carboxamide |
| "A134" | N-(Pyridin-4-ylméthyl)-5-[6-amino-5-(6-méthoxy-1H-benzimidazol-2-yl)pyridin-3-yl]thiophène-2-carboxamide |
| "A135" | N-(2-{2-[2-Amino-5-(1-méthyl-1H-pyrazol-4-yl)pyridin-3-yl]-3H-benzimidazol-5-yl}éthyl)-3,4-difluorobenzamide |
| "A137" | 3-[5-(4-Méthylpipérazine-1-sulfonyl)-1H-benzimidazol-2-yl]-5-(1-méthyl-1H-pyrazol-4-yl)pyridin-2-ylamine |
| "A140" | N-(Pyridin-4-ylméthyl)-2-[2-amino-5-(1-méthyl-1H-pyrazol-4-yl)pyridin-3-yl]-5-fluoro-3H-benzimidazole-4-carboxamide |
| "A143" | 1-{2-[2-Amino-5-(1-méthyl-1H-pyrazol-4-yl)pyridin-3-yl]-1H-benzimidazol-5-yl}-1-phényléthanol |
| "A146" | 4-{2-[2-Amino-5-(1-méthyl-1H-pyrazol-4-yl)pyridin-3-yl]-3H-benzimidazol-5-yl}morpholin-3-one |
| "A147" | 3-{2-[2-Amino-5-(1-méthyl-1H-pyrazol-4-yl)pyridin-3-yl]-1H-benzimidazol-5-yl}-1,3-oxazinan-2-one |
| "A149" | 1-{2-[2-Amino-5-(1-pipéridin-4-yl-1H-pyrazol-4-yl)pyridin-3-yl]-1H-benzimidazol-5-yl}-1-phényléthanol |
| "A150" | 1-(2-{2-Amino-5-[1-(2-morpholin-4-yléthyl)-1H-pyrazol-4-yl]pyridin-3-yl}-1H-benzimidazol-5-yl)-1-phényléthanol |

et les sels, tautomères et stéréoisomères pharmaceutiquement utilisables de ceux-ci, y compris des mélanges de ceux-ci en toutes proportions.

**2.** Médicaments comprenant au moins un composé selon la revendication 1 et/ou des sels, tautomères et stéréoisomères pharmaceutiquement utilisables de celui-ci, y compris des mélanges de ceux-ci en toutes proportions, et éventuellement des excipients et/ou des adjuvants.

**3.** Utilisation de composés selon la revendication 1
et de sels, tautomères et stéréoisomères pharmaceutiquement utilisables de ceux-ci, y compris des mélanges de ceux-ci en toutes proportions, pour la préparation d'un médicament destiné au traitement de tumeurs, d'une croissance tumorale, de métastases tumorales et/ou du SIDA.

4. Utilisation de composés selon la revendication 1
et de sels, tautomères et stéréoisomères pharmaceutiquement utilisables de ceux-ci, y compris des mélanges de ceux-ci en toutes proportions, pour la préparation d'un médicament destiné au traitement des maladies inflammatoires ou allergiques, d'affections abdominales inflammatoires, de la vaginite, du psoriasis, des dermatoses inflammatoires, de l'angéite, des spondyloarthropathies, de la sclérodermie, de l'asthme, des maladies respiratoires allergiques, des maladies auto-immunes, du rejet de greffes, de l'athérosclérose, de la myosite, des troubles neurologiques, des lésions d'ischémie-reperfusion, des lésions du cerveau traumatiques ouvertes, des lésions du cerveau traumatiques fermées, des maladies neurodégénératives, de la sclérose en plaques, de la maladie d'Alzheimer, de l'encéphalite, de la méningite, de l'ostéoporose, de la goutte, de l'hépatite, de la néphrite, des maladies de la vésicule biliaire, de la sepsie, de la sarcoïdose, de la conjonctivite, de l'otite, de la broncho-pneumopathie chronique obstructive, de la sinusite et du syndrome de Behçet, des maladies de prolifération cellulaire ou néoplasiques,
de l'obésité, du diabète de type II, du syndrome métabolique, de l'in-sulinorésistance, de l'hyperglycémie, de l'hyperuricémie, de l'hyper-insulinémie, de la cachexie, de l'hypercholestérolémie, de l'hyperlipidémie, de la dyslipidémie, de la dyslipidémie et de l'hypertriglycéridémie mixtes, des troubles de l'alimentation, des maladies infectieuses, de l'insuffisance cardiaque aiguë, de l'hypotension, de l'hypertension, de l'angine de poitrine, de l'infarctus du myocarde, de la cardiomyopathie, de l'insuffisance cardiaque congestive, de l'athérosclérose, des maladies coronariennes, de la resténose et de la sténose vasculaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006021886 A
- WO 2007111904 A
- WO 2004069160 A
- WO 2009053737 A
- WO 2009126003 A
- US 20090197862 A
- WO 01051641 A
- US 7132438 B2
- US 7199119 B2
- WO 0050032 A
- US 6069134 A


### In der Beschreibung aufgeführte Nicht-Patentliteratur


- **ÜBERBLICK, Z. B. DINARELLO.** *Cytokine Growth Factor Rev.,* 1997, vol. 8, 253-265
- **WESCHE et al.** *J. Biol. Chem.,* 1999, vol. 274, 19403-19410
- **SIEHE, O'NEILL et al.** *J. Leukoc. Biol.,* 1998, vol. 63 (6), 650-657
- **AURON.** *Cytokine Growth Factor Rev.,* 1998, vol. 9 (3-4), 221-237
- **O'NE.** *Biochem. Soc. Trans.,* 2000, vol. 28 (5), 557-563
- **CAO et al.** *Nature,* 1996, vol. 383, 443-446
- **CAO et al.** *Science,* 1996, vol. 271, 1128-1131
- **MUZIO et al.** *Science,* 1997, vol. 278, 1612-1615
- **WESCHE et al.** *J. Biol. Chem.,* 1999, vol. 274, 19403-10
- **KANAKARAJ et al.** *J. Exp. Med.,* 1999, vol. 189 (7), 1129-1138
- **YANG et al.** *J. Immunol.,* 1999, vol. 163, 639-643